# FASCICULE DE BREVET EUROPEEN

(11) **EP 3 373 953 B1**
(45) Date de publication et mention de la délivrance du brevet: **26.08.2020**
(21) Numéro de dépôt: 16797554.9
(22) Date de dépôt: 16.11.2016
(51) Int. Cl.: A61K 38/28, A61K 47/12, A61K 9/08

(54) **COMPOSITION A ACTION RAPIDE D'INSULINE COMPRENANT UN CITRATE SUBSTITUE**
SCHNELL WIRKENDE INSULINZUSAMMENSETZUNG MIT EINEM SUBSTITUIERTEN CITRAT
FAST-ACTING INSULIN COMPOSITION COMPRISING A SUBSTITUTED CITRATE

(30) Priorité: 16.11.2015 FR 1561017
(43) Date de publication de la demande: 19.09.2018
(73) Titulaire: Adocia, 69003 Lyon (FR)
(72) Inventeur: SOULA, Olivier, 69330 Meyzieu (FR); CHARVET, Richard, 69140 Rillieux La Pape (FR); ALLUIS, Bertrand, 69740 Genas (FR)
(74) Mandataire: Tripoz, Inès
(86) Numéro de dépôt international: PCT/EP2016/077912
(87) Numéro de publication internationale: WO 2017/085151

(56) Documents cités:
- EP-A2- 0 093 551
- WO-A1-2013/064787
- WO-A2-2015/173427
- KALRA SANJAY ET AL: "Ultra-fast acting insulin analogues", RECENT PATENTS ON ENDOCRINE, METABOLIC AND IMMUNE DRUG DISCOVERY,, vol. 8, no. 2, 1 janvier 2014 (2014-01-01) , pages 117-123, XP009183310, ISSN: 1872-2148

## Description

La présente invention concerne une composition à action rapide d'insuline comprenant un citrate substitué. L'invention concerne également la composition à action rapide d'insuline comprenant un citrate substitué comprenant en outre un composé polyanionique.

Depuis la production d'insuline par génie génétique, au début des années 80, les patients diabétiques bénéficient d'insuline humaine pour se traiter. Ce produit a grandement amélioré cette thérapie puisque les risques immunologiques liés à l'utilisation d'insuline non humaine, en particulier de porc, se trouvent éliminés. Cependant, l'insuline humaine injectée par voie sous-cutanée a un effet hypoglycémiant seulement après 60 minutes, ce qui implique que les patients diabétiques traités avec l'insuline humaine doivent procéder à l'injection 30 minutes avant le repas.

Un des problèmes à résoudre pour améliorer la santé et le confort des patients diabétiques est de mettre à leur disposition des formulations d'insuline qui permettent d'apporter une réponse hypoglycémiante plus rapide que celle de l'insuline humaine et si possible s'approchant de la réponse physiologique de la personne saine. La sécrétion d'insuline endogène chez l'individu sain est immédiatement déclenchée par l'augmentation de la glycémie. L'objectif est de réduire le plus possible le délai entre l'injection d'insuline et le début du repas.

Aujourd'hui, il est admis que la mise à disposition de telles formulations est utile pour que la prise en charge de la maladie soit la meilleure possible.

Le génie génétique a permis d'apporter une réponse avec le développement d'insulines analogues rapides. Ces insulines sont modifiées sur un ou deux acides aminés pour être plus rapidement absorbées dans le compartiment sanguin après une injection sous-cutanée. Ces insulines lispro (Humalog®, Lilly), aspart (Novolog®, Novo Nordisk) et glulisine (Apidra®, Sanofi Aventis) sont des solutions stables d'insuline avec une réponse hypoglycémiante plus rapide que celle de l'insuline humaine. Dès lors, les patients traités avec ces insulines analogues rapides peuvent procéder à l'injection d'insuline seulement 15 minutes avant le repas.

Le principe des insulines analogues rapides est de former des hexamères à la concentration de 100 UI/mL pour assurer la stabilité de l'insuline dans le produit commercial tout en favorisant la dissociation très rapide de ces hexamères en monomères après injection sous-cutanée afin d'obtenir une action rapide.

L'insuline humaine, telle que formulée sous sa forme commerciale, ne permet pas d'obtenir une réponse hypoglycémiante proche en terme de cinétique de la réponse physiologique générée par le début d'un repas (hausse de la glycémie), car à la concentration d'usage (100 UI/mL), en présence de zinc et d'autres excipients tels que le phénol ou le *m*-crésol, elle s'assemble sous forme d'hexamère alors qu'elle est active sous forme de monomère et de dimère. L'insuline humaine est préparée sous forme d'hexamères pour être stable près de 2 ans à 4°C car sous forme de monomères, elle a une très forte propension à s'agréger puis à fibriller, ce qui lui fait perdre son activité. De plus sous cette forme agrégée, elle présente un risque immunologique pour le patient.

Les dissociations des hexamères en dimères et des dimères en monomères retardent son action de près de 20 minutes comparativement à une insuline analogue rapide (Brange J., et al, Advanced Drug Delivery Review, 35, 1999, 307-335).

En outre, la cinétique de passage des insulines analogues dans le sang, ainsi que leur cinétique de réduction de la glycémie, ne sont pas optimales et il existe un réel besoin d'une formulation ayant un temps d'action encore plus court afin de s'approcher des cinétiques de sécrétion d'insuline endogène chez les personnes saines.

La société Biodel a proposé une solution à ce problème avec une formulation d'insuline humaine comprenant de l'EDTA et de l'acide citrique telle que décrite dans la demande de brevet US200839365. L'EDTA par sa capacité à complexer les atomes de zinc et l'acide citrique par ses interactions avec les zones cationiques présentes à la surface de l'insuline sont décrits comme déstabilisant la forme hexamérique de l'insuline, réduisant ainsi son temps d'action.

Cependant, une telle formulation présente notamment comme inconvénient de dissocier la forme hexamérique de l'insuline qui est la seule forme stable susceptible de répondre aux exigences de stabilité de la réglementation pharmaceutique.

On connait également au nom de la demanderesse, les demandes PCT WO2010/122385 et WO2013/064787 qui décrivent des formulations d'insuline humaine ou analogue et d'un polysaccharide ou d'un oligosaccharide substitué comprenant des groupes carboxyles.

Cependant, les exigences entraînées par l'usage chronique et intensif voire l'usage pédiatrique de telles formulations conduisent l'homme de l'art à rechercher à utiliser des excipients dont la masse molaire et la taille soient les plus réduites possibles pour en faciliter l'élimination.

Les polysaccharides décrits dans les demandes WO 2010/122385A1 et US 2012/094902A1 comme excipients sont des composés constitués de chaînes dont les longueurs sont statistiquement variables et qui présentent une grande richesse de sites d'interaction possibles avec des principes actifs protéiques. Cette richesse pourrait induire un manque de spécificité en termes d'interaction et une molécule plus petite et mieux définie pourrait permettre d'être davantage spécifique à ce sujet.

En outre, une molécule avec un squelette bien défini est en général plus facilement traçable (MS/MS par exemple) dans les milieux biologiques lors d'expériences de pharmacocinétique ou d'ADME (administration, distribution, métabolisme, élimination) par rapport à un polymère qui donne généralement un signal très diffus et bruité en spectrométrie de masse.

A contrario, il n'est pas exclu qu'une molécule bien définie et plus courte puisse présenter un déficit de sites d'interaction possibles avec des principes actifs protéiques. En effet, en raison de leur taille réduite, les petites molécules ne présentent pas les mêmes propriétés que les polymères de type polysaccharide car il peut y avoir perte de l'effet polymère.

Les citrates substitués des compositions selon l'invention présentent un taux de substitution généralement supérieur à celui observé dans les polymères/oligomères de l'art antérieur.

La demande EP 0 093 551 divulgue des molécules dérivées de phénylalanine capables d'améliorer l'absorption d'une substance médicinale (comme l'insuline) dans le cadre d'une administration orale ou rectale. Ces modes d'administration admettent des formes galéniques qui ne sont pas des solutions aqueuses. Par exemple cette demande décrit des suspensions, des comprimés, des gélules, ou encore des suppositoires. Elle décrit également qu'un support liquide de type huile grasse peut être envisagé.

La demanderesse a cependant réussi à mettre au point des formulations susceptibles d'accélérer l'insuline en utilisant un citrate substitué.

Dans un mode de réalisation, la formulation peut comprendre en outre un composé polyanionique.

De plus, comme dans le cas de l'utilisation de polysaccharides, la nature hexamérique de l'insuline n'est pas affectée, donc la stabilité des formulations n'est pas affectée, comme cela est d'ailleurs confirmé par les exemples d'état d'association d'insuline analogue lispro et d'insuline humaine en dichroïsme circulaire en présence de citrates substitués selon l'invention, et éventuellement de composé polyanionique.

La stabilité, notamment physique, des formulations selon l'invention est également un critère de développement important. En particulier les formulations doivent répondre aux exigences des pharmacopées en terme de stabilité physique et/ou chimique à long terme.

La présente invention permet de résoudre les différents problèmes ci-dessus exposés, en tout ou partie, puisqu'elle permet notamment de réaliser une formulation d'insuline, humaine ou analogue, capable après administration, d'accélérer le passage de l'insuline humaine ou de ses analogues dans le sang et de réduire plus rapidement la glycémie en comparaison des produits commerciaux d'insuline correspondants. Les compositions selon l'invention sont des solutions aqueuses injectables selon la pharmacopée, notamment Européenne ou Américaine.

L'invention consiste en une composition, sous forme de solution aqueuse, comprenant une insuline sous forme hexamérique et au moins un citrate substitué selon la revendication 1.

Dans un mode réalisation, la composition comprend en outre, au moins un composé polyanionique différent dudit citrate substitué.

Dans un mode de réalisation, le pH de la composition est compris entre 6 et 8.

Par « solution aqueuse » on entend une solution au sens de la Pharmacopée Européenne.

La solution selon l'invention peut ainsi répondre à la Pharmacopée Européenne 8.0 qui définit qu'une préparation injectable d'insuline soluble présente les caractères de liquide incolore, non opalescent, exempt de substances étrangères ; des traces de sédiments très fins pouvant se déposer durant la conservation (01/2008 :0834).

La solution selon l'invention peut être un liquide non opalescent, voire limpide.

Selon la Pharmacopée Européenne 8.0 au point 2.2.1. un liquide est considéré comme limpide lorsqu'il présente une opalescence qui n'est pas plus prononcée que celle de la suspension témoin I, qui présente une valeur d'opalescence de 3 UTN. L'opalescence de la solution peut être déterminée par la méthode visuelle et/ou par la méthode instrumentale, dite turbidimétrie. Lesdites méthodes étant définies dans la Pharmacopée Européenne 8.0 au point 2.2.1.

Tout particulièrement, la solution selon l'invention présente une turbidité inférieure ou égale à 3 UTN selon les différentes méthodes décrites dans la Pharmacopée Européenne 8.0 au point 2.2.1.

Dans un mode de réalisation les compositions selon l'invention sont stérilisées par filtration sur membrane 0,22 µm par exemple par filtration sur une membrane SLGV033RS, Millex-GV de Millipore, membrane de 0,22 µm en PVDF.

Dans un mode de réalisation le pH de la composition est compris entre 6 et 8.

l'invention concerne une composition, sous forme de solution aqueuse, comprenant une insuline sous forme hexamèrique, et au moins un citrate substitué de Formule I: 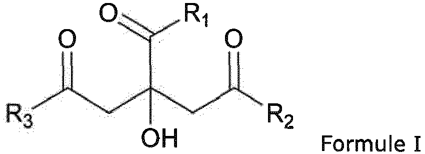 dans laquelle :
- R₁, R₂, R₃ identiques ou différents, représentent OH ou AA,
- au moins un des R₁, R₂, R₃ est un radical AA,
- AA est un radical issu d'un acide aminé aromatique naturel ou synthétique comprenant au moins un groupement phényle ou un groupement indole, substitué ou non substitué, ledit radical AA présentant au moins une fonction acide carboxylique libre,
- les fonctions acide carboxylique sont sous forme de sel d'un métal alcalin choisi parmi Na⁺ et K⁺.

Dans un mode de réalisation, la composition est caractérisée en ce qu'elle comprend en outre au moins un composé polyanionique différent dudit citrate substitué.

Par « acide aminé aromatique naturel ou synthétique comprenant au moins un groupement phényle ou un groupement indole, substitué ou non substitué », on entend un composé comprenant de 7 à 20 atomes de carbone, un phényle ou un indole, substitué ou non substitué, une fonction amine et une fonction acide carboxylique.

Dans un mode de réalisation, le citrate substitué forme avec le radical issu d'un acide aminé -AA une liaison amide. Ainsi, les groupe R₁, R₂ ou R₃ sont des résidus d'acide aminé, liés via leur fonction amine à une fonction acide du citrate, formant ainsi une liaison amide.

Comme il a été précisé ci-dessus, les taux de substitution en radical AA par gramme de composés sont élevés en regard de ceux des polymères/oligomères de l'art antérieur.

Par ailleurs, le nombre de fonctions carboxylates du citrate substitué, provenant du citrate ou des radicaux AA, est supérieur au nombre de fonctions carboxylates portés par unité monomérique des polymères/oligomères de l'art antérieur.

Enfin, le ratio fonctions carboxylates par rapport aux fonctions hydroxyles est beaucoup plus élevé dans le cas du citrate substitué que dans le cas des polymères/oligomères de l'art antérieur.

Selon un mode de réalisation, la composition est caractérisée en ce que le citrate substitué est choisi parmi les composés de formule I dans laquelle le radical AA est issu d'un acide aminé aromatique naturel ou synthétique comprenant au moins un groupement phényle ou un groupement indole, substitué ou non substitué, choisi parmi les acides alpha- ou béta- aminés. Les acides aminés aromatiques comportant un phényle ou un indole, substitué ou non substitué, peuvent être choisis dans le groupe constitué de la phénylalanine, l'alpha-méthyl phénylalanine, la 3,4-dihydroxyphénylalanine, l'alpha phénylglycine, la 4-hydroxyphénylglycine, la 3,5-dihydroxyphénylglycine, la tyrosine, l'alpha-méthyl tyrosine, la O-méthyl tyrosine et le tryptophane.

Dans un mode de réalisation, la composition est caractérisée en ce que le citrate substitué est choisi parmi les composés de formule I dans laquelle le radical AA est issu d'un acide aminé aromatique naturel.

Selon un mode de réalisation, l'acide aminé aromatique naturel est choisi dans le groupe constitué de la phénylalanine, la tyrosine et le tryptophane.

Dans un mode de réalisation préféré, l'acide aminé aromatique naturel est la phénylalanine.

Les acides aminés aromatiques peuvent, le cas échéant, être sous forme lévogyre, dextrogyre ou sous forme racémique. En particulier, ils sont sous forme lévogyre.

Dans un mode de réalisation préféré, l'acide aminé aromatique est la L-phénylalanine.

Dans un mode de réalisation préféré, l'acide aminé aromatique est le L-tryptophane.

Dans un mode de réalisation préféré, l'acide aminé aromatique est la L-tyrosine.

Dans un mode de réalisation, la composition est caractérisée en ce que le citrate substitué est choisi parmi les composés de formule I dans laquelle le radical AA est issu d'un acide aminé aromatique synthétique.

Selon un mode de réalisation, l'acide aminé aromatique synthétique est l'alpha phénylglycine.

Selon un mode de réalisation, la composition est caractérisée en ce que le citrate substitué comprend de 1 à 3 radicaux AA.

Dans un mode de réalisation, la composition est caractérisée en ce que le citrate substitué est choisi parmi les composés de formule I dans laquelle R₁ est un radical AA.

Dans un mode de réalisation, la composition est caractérisée en ce que le citrate substitué est choisi parmi les composés de formule I dans laquelle R₂ est un radical AA.

Dans un mode de réalisation, la composition est caractérisée en ce que le citrate substitué est choisi parmi les composés de formule I dans laquelle au moins R₁ est un radical AA.

Dans un mode de réalisation, la composition est caractérisée en ce que le citrate substitué est choisi parmi les composés de formule I dans laquelle au moins R₂ est un radical AA.

Dans un mode de réalisation, la composition est caractérisée en ce que le citrate substitué est choisi parmi les composés de formule I dans laquelle au moins deux des R₁, R₂, R₃ sont un radical AA.

Dans un mode de réalisation, la composition est caractérisée en ce que le citrate substitué est choisi parmi les composés de formule I dans laquelle au moins R₁ et R₂ sont un radical AA.

Dans un mode de réalisation, la composition est caractérisée en ce que le citrate substitué est choisi parmi les composés de formule I dans laquelle au moins R₂ et R₃ sont un radical AA.

Dans un mode de réalisation, la composition est caractérisée en ce que le citrate substitué est choisi parmi les composés de formule I dans laquelle R₁, R₂ et R₃ sont un radical AA.

Dans un mode de réalisation, la composition est caractérisée en ce que le citrate substitué est choisi parmi les composés de formule I dans laquelle R₁ est un radical AA et R₂ et R₃ sont OH.

Dans un mode de réalisation, la composition est caractérisée en ce que le citrate substitué est choisi parmi les composés de formule I dans laquelle R₂ est un radical AA et R₁ et R₃ sont OH.

Dans un mode de réalisation, la composition est caractérisée en ce que le citrate substitué est choisi parmi les composés de formule I dans laquelle R₁ est OH et R₂ et R₃ sont un radical AA.

Dans un mode de réalisation, lorsque R₁ et R₂, R₁ et R₃, R₂ et R₃ ou R₁, R₂ et R₃ sont un radical AA, tous les radicaux AA sont identiques.

Dans un mode de réalisation, lorsque R₁ et R₂, R₁ et R₃, R₂ et R₃ ou R₁, R₂ et R₃ sont un radical AA, tous les radicaux AA sont issus de la phénylalanine, notamment de la L-phénylalanine.

Dans un mode de réalisation, la composition est caractérisée en ce que le citrate substitué est choisi parmi les composés de formule II suivante : 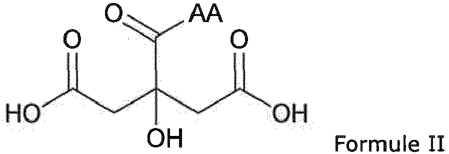 dans laquelle AA est défini tel que ci-dessus,
les fonctions acide carboxylique sont sous forme de sel d'un métal alcalin choisi parmi Na⁺ et K⁺.

Dans un mode de réalisation, la composition est caractérisée en ce que le citrate substitué est choisi parmi les composés de formule II dans laquelle AA est issu d'un acide aminé aromatique choisi dans le groupe constitué de la phénylalanine, de la tyrosine et du tryptophane.

Dans un mode de réalisation, la composition est caractérisée en ce que le citrate substitué est choisi parmi les composés de formule II dans laquelle AA est issu de la L-phénylalanine.

Dans un mode de réalisation, la composition est caractérisée en ce que le citrate substitué est choisi parmi les composés de formule I dans laquelle R₂ ou R₃ est un radical AA, R₁ est OH, si R₂ = AA alors R₃ = OH et si R₃ = AA alors R₂ est OH.

Dans un mode de réalisation, la composition est caractérisée en ce que le citrate substitué est choisi parmi les composés de formule III suivante : 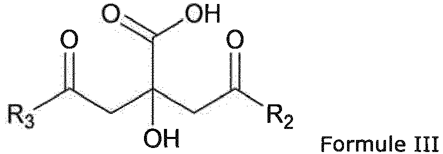 dans laquelle AA, R₂ et R₃ sont définis tel que ci-dessus et,
- si R₂ = AA, alors R₃ = OH,
- si R₃ = AA, alors R₂ = OH,
les fonctions acide carboxylique sont sous forme de sel d'un métal alcalin choisi parmi Na⁺ et K⁺.

Dans un mode de réalisation, la composition est caractérisée en ce que le citrate substitué est choisi parmi les composés de formule III dans laquelle AA est issu d'un acide aminé aromatique choisi dans le groupe constitué de la phénylalanine, de la tyrosine et du tryptophane.

Dans un mode de réalisation, la composition est caractérisée en ce que le citrate substitué est choisi parmi les composés de formule III dans laquelle AA est issu de la L-phénylalanine.

Dans un mode de réalisation, la composition est caractérisée en ce que le citrate substitué est choisi parmi les composés de formule IV : 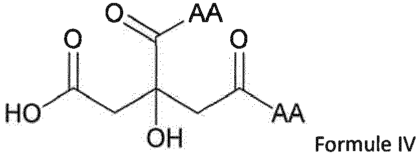 dans laquelle le radical AA est tel que défini ci-dessus et les fonctions acide carboxylique sont sous forme de sel d'un métal alcalin choisi parmi Na⁺ et K⁺.

Dans un mode de réalisation, la composition est caractérisée en ce que le citrate substitué est choisi parmi les composés de formule V : 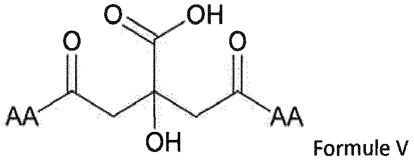 dans laquelle le radical AA est tel que défini ci-dessus et les fonctions acide carboxylique sont sous forme de sel d'un métal alcalin choisi parmi Na⁺ et K⁺.

Dans un mode de réalisation, la composition est caractérisée en ce que le citrate substitué est choisi parmi les composés de formule VI : 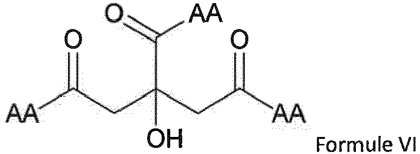 dans laquelle le radical AA est tel que défini ci-dessus et les fonctions acide carboxylique sont sous forme de sel d'un métal alcalin choisi parmi Na⁺ et K⁺.

Dans un mode de réalisation, la composition est caractérisée en ce que le citrate substitué répond à la Formule I dans laquelle R₁ est un radical AA, R₂ et R₃ sont OH et le radical AA est issu de la phénylalanine.

Dans un mode de réalisation, la composition est caractérisée en ce que le citrate substitué répond à la Formule I dans laquelle R₁ est un radical AA, R₂ et R₃ sont OH et le radical AA est issu du tryptophane.

Dans un mode de réalisation, la composition est caractérisée en ce que le citrate substitué répond à la Formule I dans laquelle R₁ est un radical AA, R₂ et R₃ sont OH et le radical AA est issu de la tyrosine.

Dans un mode de réalisation, la composition est caractérisée en ce que le citrate substitué répond à la Formule IV dans laquelle le radical AA est issu de la phénylalanine.

Dans un mode de réalisation, la composition est caractérisée en ce que le citrate substitué répond à la Formule I dans laquelle R₂ est un radical AA, R₁ et R₃ sont OH et le radical AA est issu de la phénylalanine.

Dans un mode de réalisation, la composition est caractérisée en ce que le citrate substitué répond à la Formule I dans laquelle R₂ est un radical AA, R₁ et R₃ sont OH et le radical AA est issu du tryptophane.

Dans un mode de réalisation, la composition est caractérisée en ce que le citrate substitué répond à la Formule I dans laquelle R₂ est un radical AA, R₁ et R₃ sont OH et le radical AA est issu de la tyrosine.

Dans un mode de réalisation, la composition est caractérisée en ce que le citrate substitué répond à la Formule I dans laquelle R₁ est OH, R₂ et R₃ sont un radical AA, et le radical AA est issu de la phénylalanine.

Dans un mode de réalisation, la composition est caractérisée en ce que le citrate substitué répond à la Formule V dans laquelle le radical AA est issu de la phénylalanine.

Dans un mode de réalisation, la composition est caractérisée en ce que le citrate substitué répond à la Formule I dans laquelle R₁, R₂ et R₃ sont un radical AA, et le radical AA est issu de la phénylalanine.

Dans un mode de réalisation, la composition est caractérisée en ce que le citrate substitué répond à la Formule VI dans laquelle le radical AA est issu de la phénylalanine.

Dans un mode de réalisation, la composition est caractérisée en ce que les ratios molaires citrate substitué/insuline sont compris entre 3 et 400.

Dans un mode de réalisation, la composition est caractérisée en ce que les ratios molaires citrate substitué/insuline sont compris entre 4 et 350.

Dans un mode de réalisation, la composition est caractérisée en ce que les ratios molaires citrate substitué/insuline sont compris entre 5 et 300.

Dans un mode de réalisation, la composition est caractérisée en ce que les ratios molaires citrate substitué/insuline sont compris entre 8 et 250.

Dans un mode de réalisation, la composition est caractérisée en ce que les ratios molaires citrate substitué/insuline sont compris entre 12 et 200.

Dans un mode de réalisation, la composition est caractérisée en ce que les ratios molaires citrate substitué/insuline sont compris entre 15 et 150.

Dans un mode de réalisation, la composition est caractérisée en ce que les ratios molaires citrate substitué/insuline sont compris entre 18 et 128.

Dans un mode de réalisation, la composition est caractérisée en ce que les ratios molaires citrate substitué/insuline sont compris entre 18 et 64.

Dans un mode de réalisation, la composition est caractérisée en ce que les ratios molaires citrate substitué/insuline sont compris entre 18 et 36.

Dans un mode de réalisation, la composition est caractérisée en ce que le ratio molaire citrate substitué/insuline est égal à 18, 25, 32, 36, 50 et 64.

Dans les ratios molaires ci-dessus, le nombre de moles d'insuline s'entend comme le nombre de moles de monomère d'insuline.

Dans un mode de réalisation, la composition est caractérisée en ce que les ratios massiques citrate substitué/insuline sont compris entre 0,5 et 30.

Dans un mode de réalisation, la composition est caractérisée en ce que les ratios massiques citrate substitué/insuline sont compris entre 0,5 et 20.

Dans un mode de réalisation, la composition est caractérisée en ce que les ratios massiques citrate substitué/insuline sont compris entre 0,5 et 16.

Dans un mode de réalisation, la composition est caractérisée en ce que les ratios massiques citrate substitué/insuline sont compris entre 0,6 et 12.

Dans un mode de réalisation, la composition est caractérisée en ce que les ratios massiques citrate substitué/insuline sont compris entre 1,2 et 10.

Dans un mode de réalisation, la composition est caractérisée en ce que les ratios massiques citrate substitué/insuline sont compris entre 1,6 et 8.

Dans un mode de réalisation, la composition est caractérisée en ce que les ratios massiques citrate substitué/insuline sont compris entre 2 et 8.

Dans un mode de réalisation, la composition est caractérisée en ce que les ratios massiques citrate substitué/insuline sont compris entre 2 et 6.

Dans un mode de réalisation, la composition est caractérisée en ce que les ratios massiques citrate substitué/insuline sont compris entre 2 et 4.

Dans un mode de réalisation, la composition est caractérisée en ce que le ratio massique citrate substitué/insuline est 2, 3, 4 ou 6.

Dans un mode de réalisation, la composition est caractérisée en ce que l'insuline est l'insuline humaine.

On entend par insuline humaine une insuline obtenue par synthèse ou recombinaison dont la séquence peptidique est la séquence de l'insuline humaine, incluant les variations alléliques et les homologues.

Dans un mode de réalisation, la composition est caractérisée en ce que l'insuline est une insuline humaine recombinante telle que décrite dans la Pharmacopée Européenne et la Pharmacopée Américaine (ou US).

Dans un mode de réalisation, la composition est caractérisée en ce que l'insuline est une insuline analogue.

On entend par insuline analogue une insuline recombinante dont la séquence primaire contient au moins une modification par rapport à la séquence primaire de l'insuline humaine.

Dans un mode de réalisation l'insuline analogue est choisie dans le groupe constitué par l'insuline lispro (Humalog®), l'insuline aspart (Novolog®, Novorapid®) et l'insuline glulisine (Apidra®).

Dans un mode de réalisation, la composition est caractérisée en ce que l'insuline analogue est l'insuline lispro (Humalog®).

Dans un mode de réalisation, la composition est caractérisée en ce que l'insuline analogue est l'insuline aspart (Novolog®, Novorapid®).

Dans un mode de réalisation, la composition est caractérisée en ce que l'insuline analogue est l'insuline glulisine (Apidra®).

Dans un mode de réalisation, la composition est caractérisée en ce que l'insuline est sous forme hexamérique.

Les unités recommandées par les pharmacopées pour les insulines sont présentées dans le tableau ci-après avec leurs correspondances en mg :

| **Insuline** | **Pharmacopée EP 8.0 (2014)** | **Pharmacopée** US - USP38 (2015**)** |
|---|---|---|
| Aspart | 1U = 0,0350 mg d'insuline aspart | 1 USP = 0,0350 mq insulin aspart |
| Lispro | 1U = 0,0347 mg d'insuline lispro | 1 USP = 0,0347 mg d'insuline lispro |
| Humaine | 1UI = 0,0347 mg d'insuline humaine | 1 USP = 0,0347 mg d'insuline humaine |

Dans le cas de l'insuline glulisine, 100U = 3,49 mg d'insuline glulisine (d'après "Annex 1 - Summary of product characteristics" relative à ADIPRA®).

Néanmoins dans la suite du texte UI est systématiquement utilisé indifféremment pour les quantités et les concentrations en toutes les insulines. Les valeurs respectives correspondant en mg sont celles données ci-dessus pour des valeurs exprimées en U, UI ou USP.

Dans le texte, lorsque « insuline » est utilisé sans qualificatif, il désigne une insuline choisie dans le groupe constitué des insulines humaine ou analogues.

Dans un mode de réalisation, la composition est caractérisée en ce que la composition pharmaceutique est caractérisée en ce que la concentration en insuline est comprise entre 240 et 3000 µM (40 à 500 UI/mL).

Dans un mode de réalisation, la composition est caractérisée en ce que la composition pharmaceutique est caractérisée en ce que la concentration en insuline est comprise entre 600 et 3000 µM (100 à 500 UI/mL).

Dans un mode de réalisation, la composition est caractérisée en ce que la composition pharmaceutique est caractérisée en ce que la concentration en insuline est comprise entre 600 et 2400 µM (100 à 400 UI/mL).

Dans un mode de réalisation, la composition est caractérisée en ce que la composition pharmaceutique est caractérisée en ce que la concentration en insuline est comprise entre 600 et 1800 µM (100 à 300 UI/mL).

Dans un mode de réalisation, la composition est caractérisée en ce que la composition pharmaceutique est caractérisée en ce que la concentration en insuline est comprise entre 600 et 1200 µM (100 à 200 UI/mL).

Dans un mode de réalisation, la composition est caractérisée en ce que elle concerne une composition pharmaceutique caractérisée en ce que la concentration en insuline est 600 µM (100 UI/mL), 1200 µM (200 UI/mL), 1800 µM (300 UI/mL), 2400 µM (400 UI/mL) ou 3000 µM (500 UI/mL).

Dans un mode de réalisation, la composition est caractérisée en ce que le composé polyanionique (composé PNP) présente une affinité pour le zinc inférieure à l'affinité de l'insuline pour le zinc et une constante de dissociation K_{d}Ca = [composé PNP]^{r∗} [Ca²⁺]^{s}/[(composé PNP)r-(Ca²⁺)s] est inférieure ou égale à 10^{-1,5}.
Cette constante de dissociation est la constante de réaction associée à la dissociation du complexe (composé PNP)r-(Ca²⁺)s, c'est-à-dire à la réaction suivante : (composé PNP)r-(Ca²⁺)s se dissocie en r(composé PNP) + sCa²⁺. Dans la présente demande le symbole * étant le signe multiplier.

Les constantes de dissociation (K_{d}) des différents composés polyanioniques vis-à-vis des ions de calcium sont déterminées par calibration externe à l'aide d'une électrode spécifique aux ions Calcium (Mettler Toledo) et d'une électrode de référence. Toutes les mesures sont effectuées dans 150 mM de NaCl à pH 7. Seules les concentrations en ions calcium libres sont déterminées ; les ions calcium liés au composé polyanionique n'induisent pas de potentiel d'électrode.

Dans un mode de réalisation, la composition est caractérisée en ce que le composé polyanionique est choisi dans le groupe constitué des polyacides carboxyliques et leurs sels de Na⁺, K⁺, Ca²⁺ ou Mg²⁺, de préférence Na⁺ ou K⁺.

Dans un mode de réalisation, la composition est caractérisée en ce que le polyacide est choisi dans le groupe constitué de l'acide citrique, l'acide aspartique, l'acide glutamique, l'acide malique, l'acide tartrique, l'acide succinique, l'acide adipique, l'acide oxalique, le phosphate, les polyacides phosphoriques, comme le triphosphate et leurs sels de Na⁺, K⁺, Ca²⁺ ou Mg²⁺, de préférence Na⁺ ou K⁺.

Dans un mode de réalisation, la composition est caractérisée en ce que le composé polyanionique est choisi dans le groupe constitué de l'acide citrique et ses sels de Na⁺, K⁺, Ca²⁺ ou Mg²⁺, de préférence Na⁺ ou K⁺.

Dans un mode de réalisation, la composition est caractérisée en ce que la concentration en citrate substitué est comprise entre 1,8 et 100 mg/mL.

Dans un mode de réalisation, la composition est caractérisée en ce que la concentration en citrate substitué est comprise entre 1,8 et 50 mg/mL.

Dans un mode de réalisation, la composition est caractérisée en ce que la concentration en citrate substitué est comprise entre 1,8 et 36 mg/mL.

Dans un mode de réalisation, la composition est caractérisée en ce que la concentration en citrate substitué est comprise entre 1,8 et 36,5 mg/mL.

Dans un mode de réalisation, la composition est caractérisée en ce que la concentration en citrate substitué est comprise entre 2,1 et 25 mg/mL.

Dans un mode de réalisation, la composition est caractérisée en ce que la concentration en citrate substitué est comprise entre 4,2 et 18 mg/mL.

Dans un mode de réalisation, la composition est caractérisée en ce que la concentration en citrate substitué est comprise entre 5,6 et 15 mg/mL.

Dans un mode de réalisation, la composition est caractérisée en ce que la concentration en citrate substitué est comprise entre 7 et 15 mg/mL.

Dans un mode de réalisation, la composition est caractérisée en ce que la concentration en citrate substitué est 7,3 mg/mL.

Dans un mode de réalisation, la composition est caractérisée en ce que la concentration en citrate substitué est 10,5 mg/mL.

Dans un mode de réalisation, la composition est caractérisée en ce que la concentration en citrate substitué est 14,6 mg/mL.

Dans un mode de réalisation, la composition est caractérisée en ce que la concentration en citrate substitué est 21,9 mg/mL.

Dans un mode de réalisation, la composition est caractérisée en ce que la concentration en citrate substitué est comprise entre 1,8 et 100 mg pour 100 UI d'insuline.

Dans un mode de réalisation, la composition est caractérisée en ce que la concentration en citrate substitué est comprise entre 1,8 et 50 mg pour 100 UI d'insuline.

Dans un mode de réalisation, la composition est caractérisée en ce que la concentration en citrate substitué est comprise entre 1,8 et 36 mg pour 100 UI d'insuline.

Dans un mode de réalisation, la composition est caractérisée en ce que la concentration en citrate substitué est comprise entre 1,8 et 36,5 mg pour 100 UI d'insuline.

Dans un mode de réalisation, la composition est caractérisée en ce que la concentration en citrate substitué est comprise entre 2,1 et 25 mg pour 100 UI d'insuline.

Dans un mode de réalisation, la composition est caractérisée en ce que la concentration en citrate substitué est comprise entre 4,2 et 18 mg pour 100 UI d'insuline.

Dans un mode de réalisation, la composition est caractérisée en ce que la concentration en citrate substitué est comprise entre 5,6 et 15 mg pour 100 UI d'insuline.

Dans un mode de réalisation, la composition est caractérisée en ce que la concentration en citrate substitué est comprise entre 7 et 15 mg pour 100 UI d'insuline.

Dans un mode de réalisation, la composition est caractérisée en ce que la concentration en citrate substitué est 7,3 mg pour 100 UI d'insuline.

Dans un mode de réalisation, la composition est caractérisée en ce que la concentration en citrate substitué est 10,5 mg pour 100 UI d'insuline.

Dans un mode de réalisation, la composition est caractérisée en ce que la concentration en citrate substitué est 14,6 mg pour 100 UI d'insuline.

Dans un mode de réalisation, la composition est caractérisée en ce que la concentration en citrate substitué est 21,9 mg pour 100 UI d'insuline.

Dans un mode de réalisation, la composition est caractérisée en ce que la concentration en composé polyanionique est comprise entre 2 et 150 mM.

Dans un mode de réalisation, la composition est caractérisée en ce que la concentration en composé polyanionique est comprise entre 2 et 100 mM.

Dans un mode de réalisation, la composition est caractérisée en ce que la concentration en composé polyanionique est comprise entre 2 et 75 mM.

Dans un mode de réalisation, la composition est caractérisée en ce que la concentration en composé polyanionique est comprise entre 2 et 50 mM.

Dans un mode de réalisation, la composition est caractérisée en ce que la concentration en composé polyanionique est comprise entre 2 et 30 mM.

Dans un mode de réalisation, la composition est caractérisée en ce que la concentration en composé polyanionique est comprise entre 2 et 20 mM.

Dans un mode de réalisation, la composition est caractérisée en ce que la concentration en composé polyanionique est comprise entre 2 et 10 mM.

Dans un mode de réalisation, la composition est caractérisée en ce que la concentration en composé polyanionique est comprise entre 5 et 150 mM.

Dans un mode de réalisation, la composition est caractérisée en ce que la concentration en composé polyanionique est comprise entre 5 et 100 mM.

Dans un mode de réalisation, la composition est caractérisée en ce que la concentration en composé polyanionique est comprise entre 5 et 75 mM.

Dans un mode de réalisation, la composition est caractérisée en ce que la concentration en composé polyanionique est comprise entre 5 et 50 mM.

Dans un mode de réalisation, la composition est caractérisée en ce que la concentration en composé polyanionique est comprise entre 5 et 30 mM.

Dans un mode de réalisation, la composition est caractérisée en ce que la concentration en composé polyanionique est comprise entre 5 et 20 mM.

Dans un mode de réalisation, la composition est caractérisée en ce que la concentration en composé polyanionique est comprise entre 5 et 10 mM.

Dans un mode de réalisation, la composition est caractérisée en ce que la concentration en composé polyanionique est comprise entre 0,5 et 30 mg/mL.

Dans un mode de réalisation, la composition est caractérisée en ce que la concentration en composé polyanionique est comprise entre 0,5 et 25 mg/mL.

[000210] Dans un mode de réalisation, la composition est caractérisée en ce que la concentration en composé polyanionique est comprise entre 0,5 et 10 mg/mL.

[000210] Dans un mode de réalisation, la composition est caractérisée en ce que la concentration en composé polyanionique est comprise entre 0,5 et 8 mg/mL.

Dans un mode de réalisation, la composition est caractérisée en ce que la concentration en composé polyanionique est comprise entre 1 et 30 mg/mL.

Dans un mode de réalisation, la composition est caractérisée en ce que la concentration en composé polyanionique est comprise entre 1,5 et 25 mg/mL.

Dans un mode de réalisation, la composition est caractérisée en ce que la concentration en composé polyanionique est comprise entre 2 et 25 mg/mL.

Dans un mode de réalisation, la composition est caractérisée en ce que la concentration en composé polyanionique est comprise entre 2 et 10 mg/mL.

Dans un mode de réalisation, la composition est caractérisée en ce que la concentration en composé polyanionique est comprise entre 2 et 8 mg/mL.

Dans un mode de réalisation, la composition est caractérisée en ce que le pH de la composition est compris entre 6 et 8.

Dans un mode de réalisation particulier, la composition selon l'invention comprend une insuline, notamment telle que définie ci-dessus, au moins un citrate substitué tel que défini ci-dessus, et de l'acide citrique ou ses sels de Na⁺, K⁺, Ca²⁺ ou Mg²⁺, notamment tels que définis ci-dessus.

Dans un mode de réalisation particulier, la composition selon l'invention comprend une insuline, notamment telle que définie ci-dessus, au moins un citrate substitué répondant à la Formule I telle que définie ci-dessus, et de l'acide citrique ou ses sels de Na⁺, K⁺, Ca²⁺ ou Mg²⁺, notamment tels que définis ci-dessus.

Il est connu de l'homme de l'art que le délai d'action des insulines est dépendant de la concentration en insuline. Seules les valeurs de délai d'action des compositions à 100 UI/mL sont documentées.

Les compositions d'insuline humaine « regular » sur le marché à une concentration de 600 µM (100 UI/mL) ont un délai d'action compris entre 50 et 90 minutes et une fin d'action d'environ 360 à 420 minutes chez l'humain. Le temps pour atteindre la concentration maximale en insuline dans le sang est compris entre 90 et 180 minutes chez l'humain.

Les compositions d'insulines analogues rapides sur le marché à une concentration de 600 µM (100 UI/mL) ont un délai d'action compris entre 30 et 60 minutes et une fin d'action d'environ 240-300 minutes chez l'humain. Le temps pour atteindre la concentration maximale en insuline dans le sang est compris entre 50 et 90 minutes chez l'humain.

L'invention concerne également une méthode de préparation d'une composition d'insuline humaine ayant une concentration en insuline comprise entre 240 et 3000 µM (40 et 500 UI/mL), dont le délai d'action chez l'humain est inférieur à celui de la composition de référence à la même concentration en insuline en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline humaine ayant une concentration en insuline comprise entre 600 et 1200 µM (100 et 200 UI/mL), dont le délai d'action chez l'humain est inférieur à celui de la composition de référence à la même concentration en insuline en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline humaine ayant une concentration en insuline de 600 µM (100 UI/mL), dont le délai d'action chez l'humain est inférieur à 60 minutes caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline humaine ayant une concentration en insuline de 1200 µM (200 UI/mL), dont le délai d'action chez l'humain est inférieur d'au moins 10% à celui de la composition de l'insuline humaine à la même concentration (200 UI/mL) et en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline humaine ayant une concentration en insuline de 1800 µM (300 UI/mL), dont le délai d'action chez l'humain est inférieur d'au moins 10% à celui de la composition de l'insuline humaine à la même concentration (300 UI/mL) et en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline humaine ayant une concentration en insuline de 2400 µM (400 UI/mL), dont le délai d'action chez l'humain est inférieur d'au moins 10% à celui de la composition de l'insuline humaine à la même concentration (400 UI/mL) et en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline humaine ayant une concentration en insuline de 3000 µM (500 UI/mL), dont le délai d'action chez l'humain est inférieur d'au moins 10% à celui de la composition de l'insuline humaine à la même concentration (500 UI/mL) et en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention consiste en la préparation d'une composition d'insuline humaine dite rapide caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation, l'insuline est sous forme hexamérique.

Dans un mode de réalisation, le pH de la composition est compris entre 6 et 8.

L'invention concerne également une méthode de préparation d'une composition d'insuline humaine à une concentration de 600 µM (100 UI/mL) dont le délai d'action chez l'humain est inférieur à 60 minutes, de préférence inférieur à 45 minutes, et encore de préférence inférieur à 30 minutes caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline analogue ayant une concentration en insuline comprise entre 240 et 3000 µM (40 et 500 UI/mL), dont le délai d'action chez l'humain est inférieur à celui de la composition de référence à la même concentration en insuline en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline analogue ayant une concentration en insuline comprise entre 600 et 1200 µM (100 et 200 UI/mL), dont le délai d'action chez l'humain est inférieur à celui de la composition de référence à la même concentration en insuline analogue en l'absence de citrate substitué et de composé polyanionique, caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline analogue ayant une concentration en insuline de 600 µmol/L (100 UI/mL), dont le délai d'action chez l'humain est inférieur à 30 minutes, caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline analogue ayant une concentration en insuline de 1200 µM (200 UI/mL), dont le délai d'action chez l'humain est inférieur d'au moins 10% à celui de la composition de l'insuline analogue en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline analogue ayant une concentration en insuline de 1800 µM (300 UI/mL), dont le délai d'action chez l'humain est inférieur d'au moins 10% à celui de la composition de l'insuline analogue en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline analogue ayant une concentration en insuline de 2400 µM (400 UI/mL), dont le délai d'action chez l'humain est inférieur d'au moins 10% à celui de la composition de l'insuline analogue en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention concerne également une méthode de préparation d'une composition d'insuline analogue ayant une concentration en insuline de 3000 µM (500 UI/mL), dont le délai d'action chez l'humain est inférieur d'au moins 10% à celui de la composition de l'insuline analogue en l'absence de citrate substitué et de composé polyanionique caractérisée en ce qu'elle comprend (1) une étape d'addition à ladite composition d'au moins un citrate substitué, et éventuellement en outre (2) une étape d'addition à ladite composition d'au moins un composé polyanionique.

Dans un mode de réalisation de la méthode, l'insuline est sous forme hexamérique.

Dans un mode de réalisation de la méthode, le pH de la composition est compris entre 6 et 8.

L'invention consiste en la préparation d'une composition d'insuline analogue dite très rapide caractérisée en ce qu'elle comprend une étape d'addition à ladite composition d'au moins un citrate substitué, les groupes fonctionnels carboxyles non substitués étant salifiables.

Dans un mode de réalisation, la préparation comprend en outre une étape d'addition à ladite composition d'au moins un composé polyanionique.

Dans un mode de réalisation, l'insuline est sous forme hexamérique.

Dans un mode de réalisation, le pH de la composition est compris entre 6 et 8.

Dans un mode de réalisation, l'invention concerne également l'utilisation d'au moins un citrate substitué, éventuellement en combinaison avec un composé polyanionique, pour préparer une composition pharmaceutique d'insuline humaine, permettant, après administration, d'accélérer le passage de l'insuline humaine dans le sang et de réduire plus rapidement la glycémie par rapport à une composition exempte de citrate substitué.

Dans un mode de réalisation, l'invention concerne l'utilisation d'au moins un citrate substitué, éventuellement en combinaison avec un composé polyanionique, pour préparer une composition d'insuline analogue, permettant, après administration, d'accélérer le passage de l'insuline analogue dans le sang et de réduire plus rapidement la glycémie par rapport à une composition exempte de citrate substitué.

Dans un mode de réalisation de l'utilisation, le pH de la composition est compris entre 6 et 8.

L'invention concerne également une composition pharmaceutique selon l'invention, caractérisée en ce qu'elle est obtenue par séchage et/ou lyophilisation.

Dans un mode de réalisation, les compositions selon l'invention comprennent en outre l'addition de sels de zinc à une concentration comprise entre 0 et 500 µM, notamment entre 0 et 300 µM, et en particulier entre 0 et 200 µM.

Dans un mode de réalisation, les compositions selon l'invention comprennent des tampons à des concentrations comprises entre 0 et 100 mM, de préférence entre 0 et 50 mM, voire entre 15 et 50 mM.

Dans un mode de réalisation le tampon est le Tris.

Dans un mode de réalisation, les compositions selon l'invention comprennent en outre des conservateurs.

Dans un mode de réalisation, les conservateurs sont choisis dans le groupe constitué par le m-crésol et le phénol seuls ou en mélange.

Dans un mode de réalisation, la concentration des conservateurs est comprise entre 10 et 50 mM, notamment entre 10 et 40 mM.

Les compositions selon l'invention peuvent en outre comprendre des additifs tels que des agents de tonicité comme la glycérine, le chlorure de sodium (NaCI), le mannitol et la glycine.

Les compositions selon l'invention peuvent en outre comprendre des additifs conformes aux pharmacopées comme des tensioactifs par exemple du polysorbate.

Les compositions selon l'invention peuvent comprendre en outre tous les excipients conformes aux pharmacopées et compatibles avec les insulines utilisées aux concentrations d'usage.

Dans le cas des libérations locale et systémique, les modes d'administration envisagés sont par voie intraveineuse, sous-cutanée, intradermique ou intramusculaire. Tout particulièrement, le mode d'administration est la voie sous-cutanée.

Les voies d'administration transdermique, orale, nasale, vaginale, oculaire, buccale, pulmonaire sont également envisagées.

L'invention concerne également l'utilisation d'une composition selon l'invention pour la composition d'une solution d'insuline humaine ou analogue de concentration de 100 UI/mL ou 200 UI/mL destinée aux pompes à insuline implantables ou transportables.

Selon un autre de ses aspects, l'invention porte également sur les citrates substitués tels que définis ci-dessus.

Dans un mode de réalisation, le citrate substitué répond à la Formule I suivante : 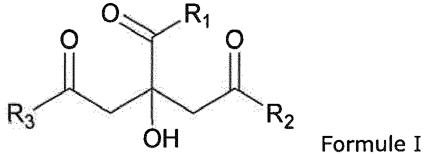 dans laquelle :
- R₁, R₂, R₃ identiques ou différents, représentent OH ou AA,
- un seul des R₁, R₂, R₃ est un radical AA,
- AA est un radical issu d'un acide aminé aromatique naturel ou synthétique comprenant au moins un groupement phényle ou un groupement indole, substitué ou non substitué, ledit radical AA présentant au moins une fonction acide carboxylique libre.

Selon un mode de réalisation, le citrate substitué est caractérisé en ce que le radical AA est issu d'un acide aminé aromatique naturel ou synthétique comprenant au moins un groupement phényle ou un groupement indole, substitué ou non substitué, choisi parmi les acides alpha- ou béta- aminés. Les acides aminés aromatiques comportant un phényle ou un indole, substitué ou non substitué, peuvent être choisis dans le groupe constitué de la phénylalanine, l'alpha-méthyl phénylalanine, la 3,4-dihydroxyphénylalanine, l'alpha phénylglycine, la 4-hydroxyphénylglycine, la 3,5-dihydroxyphénylglycine, la tyrosine, l'alpha-méthyl tyrosine, la O-méthyl tyrosine et le tryptophane.

Dans un mode de réalisation, le citrate substitué est caractérisé en ce que le radical AA est issu d'un acide aminé aromatique naturel.

Selon un mode de réalisation, l'acide aminé aromatique naturel est choisi dans le groupe constitué de la phénylalanine, la tyrosine et le tryptophane.

Dans un mode de réalisation préféré, l'acide aminé aromatique naturel est la phénylalanine.

Les acides aminés aromatiques peuvent, le cas échéant, être sous forme lévogyre, dextrogyre ou sous forme racémique. En particulier, ils sont sous forme lévogyre.

Dans un mode de réalisation préféré, l'acide aminé aromatique est la L-phénylalanine.

Dans un mode de réalisation préféré, l'acide aminé aromatique est le L-tryptophane.

Dans un mode de réalisation préféré, l'acide aminé aromatique est la L-tyrosine.

Dans un mode de réalisation, le citrate substitué est caractérisé en ce que le radical AA est issu d'un acide aminé aromatique synthétique.

Selon un mode de réalisation, l'acide aminé aromatique synthétique est l'alpha phénylglycine.

Dans un mode de réalisation, le citrate substitué est caractérisé en ce que R₁ est un radical AA et R₂ et R₃ sont OH.

Dans un mode de réalisation, le citrate substitué répond à la Formule II suivante : 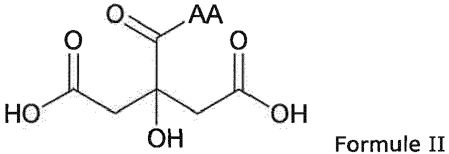 dans laquelle le radical AA est défini tel que ci-dessus.

Dans un mode de réalisation, le citrate substitué est choisi parmi les citrates substitués de formule II dans laquelle AA est issu d'un acide aminé aromatique choisi dans le groupe constitué de la phénylalanine, de la tyrosine et du tryptophane.

Dans un mode de réalisation, le citrate substitué est choisi parmi les citrates substitués de formule II dans laquelle AA est issu de la L-phénylalanine.

Dans un mode de réalisation, le citrate substitué est caractérisé en ce que R₂ ou R₃ est un radical AA, R₁ est OH, si R₂ = AA alors R₃ = OH et si R₃ = AA alors R₂ est OH.

Dans un mode de réalisation, le citrate substitué répond à la Formule III suivante : 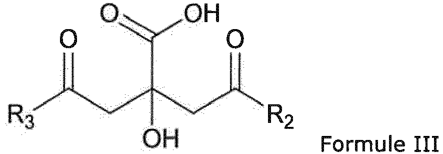 dans laquelle le radical AA, R₂ et R₃ sont définis tel que ci-dessus et,
- si R₂ = AA, alors R₃ = OH,
- si R₃ = AA, alors R₂ = OH.

Dans un mode de réalisation, le citrate substitué est choisi parmi les citrates substitués de formule III dans laquelle AA est issu d'un acide aminé aromatique choisi dans le groupe constitué de la phénylalanine, de la tyrosine et du tryptophane.

Dans un mode de réalisation, le citrate substitué est choisi parmi les citrates substitués de formule III dans laquelle AA est issu de la L-phénylalanine.

Dans un mode de réalisation, le citrate substitué répond à la formule IV suivante : 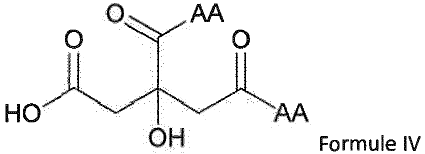 dans laquelle le radical AA est tel que défini ci-dessus et les fonctions acide carboxylique sont sous forme de sel d'un métal alcalin choisi parmi Na⁺ et K⁺.

Dans un mode de réalisation, le citrate substitué est choisi parmi les citrates substitués de formule IV dans laquelle le radical AA est issu d'un acide aminé aromatique choisi dans le groupe constitué de la phénylalanine, de la tyrosine et du tryptophane.

Dans un mode de réalisation, le citrate substitué est choisi parmi les citrates substitués de formule IV dans laquelle le radical AA est issu de la L-phénylalanine.

Dans un mode de réalisation, la composition est caractérisée en ce que le citrate substitué est choisi parmi les composés de formule V : 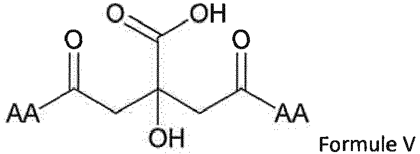 dans laquelle le radical AA est tel que défini ci-dessus et les fonctions acide carboxylique sont sous forme de sel d'un métal alcalin choisi parmi Na⁺ et K⁺.

Dans un mode de réalisation, le citrate substitué est choisi parmi les citrates substitués de formule V dans laquelle le radical AA est issu d'un acide aminé aromatique choisi dans le groupe constitué de la phénylalanine, de la tyrosine et du tryptophane.

Dans un mode de réalisation, le citrate substitué est choisi parmi les citrates substitués de formule V dans laquelle le radical AA est issu de la L-phénylalanine.

Dans un mode de réalisation, la composition est caractérisée en ce que le citrate substitué est choisi parmi les composés de formule VI : 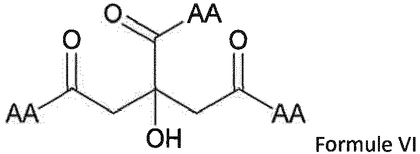 dans laquelle le radical AA est tel que défini ci-dessus et les fonctions acide carboxylique sont sous forme de sel d'un métal alcalin choisi parmi Na⁺ et K⁺.

Dans un mode de réalisation, le citrate substitué est choisi parmi les citrates substitués de formule VI dans laquelle le radical AA est issu d'un acide aminé aromatique choisi dans le groupe constitué de la phénylalanine, de la tyrosine et du tryptophane.

Dans un mode de réalisation, le citrate substitué est choisi parmi les citrates substitués de formule VI dans laquelle le radical AA est issu de la L-phénylalanine.

Les citrates substitués selon l'invention sont par exemple synthétisés comme dans les exemples ci-dessous. La voie de synthèse utilisée peut être revue et adaptée selon la nature des radicaux AA que l'on souhaite avoir sur le citrate substitué.

Des méthodes classiques de l'état de la technique faisant intervenir des étapes de protection/déprotection des fonctions carboxylates du citrate peuvent être utilisées pour coupler les fonctions d'intérêt avec le précurseur du radical AA.

Ainsi, pour obtenir un citrate substitué portant deux radicaux AA, on peut par exemple protéger la fonction carboxylate du citrate que l'on souhaite garder sous forme de -COOH, former ensuite la liaison amide par réaction entre les fonctions carboxylate désirées du citrate et une fonction amine portée par le précurseur du radical AA puis enfin déprotéger la fonction carboxylate du citrate que l'on souhaite conserver sous forme de -COOH. Cette étape de déprotection finale peut également servir d'étape de déprotection de la ou des fonctions carboxylates portées par les précurseurs des radicaux AA sous forme de -COOH. La déprotection de la ou des fonctions carboxylates portées par les précurseurs des radicaux AA portés par le citrate peut éventuellement se faire indépendamment lors d'une étape spécifique de synthèse avant ou après l'étape de déprotection conduisant à la forme -COOH de la fonction carboxylate du citrate laissée libre.

Dans le cas où les R₁, R₂ et R₃ sont des radicaux AA identiques, ils sont introduits en une seule étape par réaction entre une fonction amine portée par leur précurseur et les trois fonctions carboxylate du citrate.

Dans le cas où les R₁, R₂ et R₃ sont des radicaux AA différents, des étapes de protection/déprotection orthogonales des fonctions carboxylates du citrate sont nécessaires afin d'obtenir un citrate substitué avec des radicaux AA différents sur les fonctions carboxylate du citrate.

Dans un mode de réalisation, la voie de synthèse des citrates substitués fait intervenir une ou plusieurs étapes de déprotection de fonctions carboxylates qui passent alors sous la forme -COOH ou sous la forme de sel de carboxylate.

Dans un mode de réalisation, la synthèse des citrates substitués fait intervenir une étape finale de passage des fonctions carboxylates du citrate substitué de la forme -COOH à la forme de sel de carboxylate.

### Description des figures

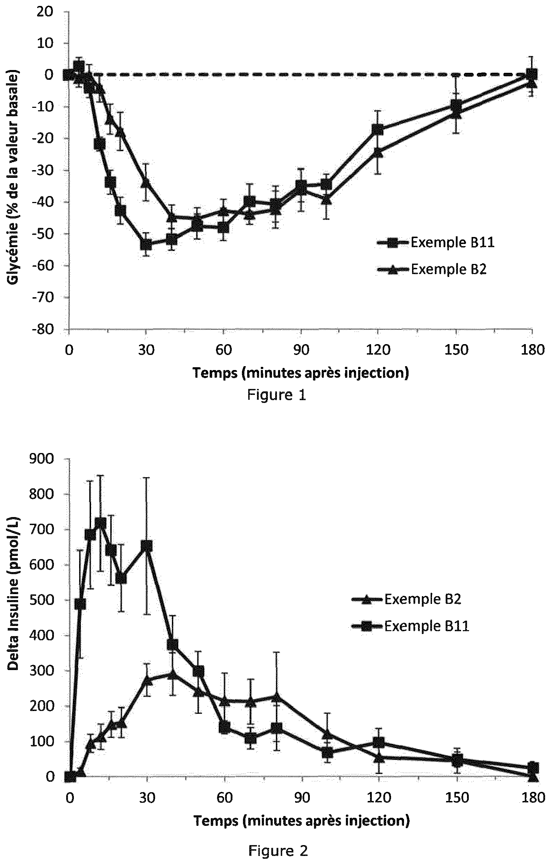

Figure 1 : Glycémie (% de la valeur basale) en fonction du temps (minutes après injection). L'analyse de ces courbes montre que la composition de l'exemple B11 comprenant le citrate substitué A1 et le citrate comme excipient (courbe tracée avec les carrés correspondant à l'exemple B11, Tmin glucose = 44 ± 18 min et T50%Rmin glucose = 15 ± 3 min) permet d'obtenir une action plus rapide que celle de la composition commerciale Humalog® de l'exemple B2 (courbe tracée avec les triangles correspondant à l'exemple B2, Tmin glucose = 61 ± 23 min et T50%Rmin glucose = 26 ± 9 min).
Figure 2 : Delta insuline (pmol/L) en fonction du temps (minutes après injection). L'analyse de ces courbes montre que la composition de l'exemple B11 comprenant le citrate substitué A1 et le citrate comme excipient (courbe tracée avec les carrés correspondant à l'exemple B11, Tmax insuline = 20 ± 23 min et T50%Cmax insuline = 6 ± 5 min) induit une absorption plus rapide de l'insuline lispro que la composition commerciale Humalog® de l'exemple B2 (courbe tracée avec les triangles correspondant à l'exemple B2, Tmax insuline = 39 ± 18 min et T50%Cmax insuline = 24 ± 14 min).

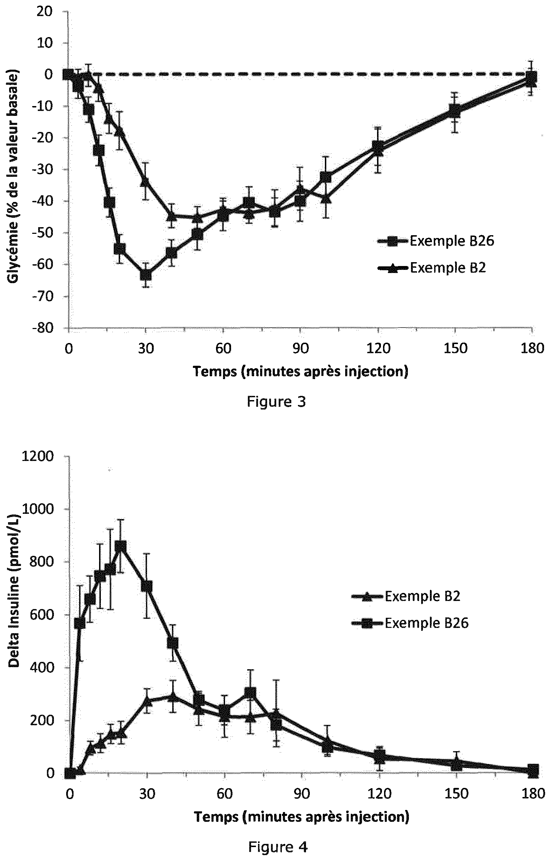

Figure 3 : Glycémie (% de la valeur basale) en fonction du temps (minutes après injection). L'analyse de ces courbes montre que la composition de l'exemple B26 comprenant le citrate substitué A1 à 14,6 mg/ml (courbe tracée avec les carrés correspondant à l'exemple B26, Tmin glucose = 37 ± 18 min et T50%Rmin glucose = 14 ± 3 min) permet d'obtenir une action plus rapide que celle de la composition commerciale Humalog® de l'exemple B2 (courbe tracée avec les triangles correspondant à l'exemple B2, Tmin glucose = 61 ± 23 min et T50%Rmin glucose = 26 ± 9 min).
Figure 4 : Delta insuline (pmol/L) en fonction du temps (minutes après injection). L'analyse de ces courbes montre que la composition de l'exemple B26 comprenant le citrate substitué A1 à 14,6 mg/ml (courbe tracée avec les carrés correspondant à l'exemple B26, Tmax insuline = 22 ± 17 min et T50%Cmax insuline = 5 ± 3 min) induit une absorption plus rapide de l'insuline lispro que la composition commerciale Humalog® de l'exemple B2 (courbe tracée avec les triangles correspondant à l'exemple B2, Tmax insuline = 39 ± 18 min et T50%Cmax insuline = 24 ± 14 min).

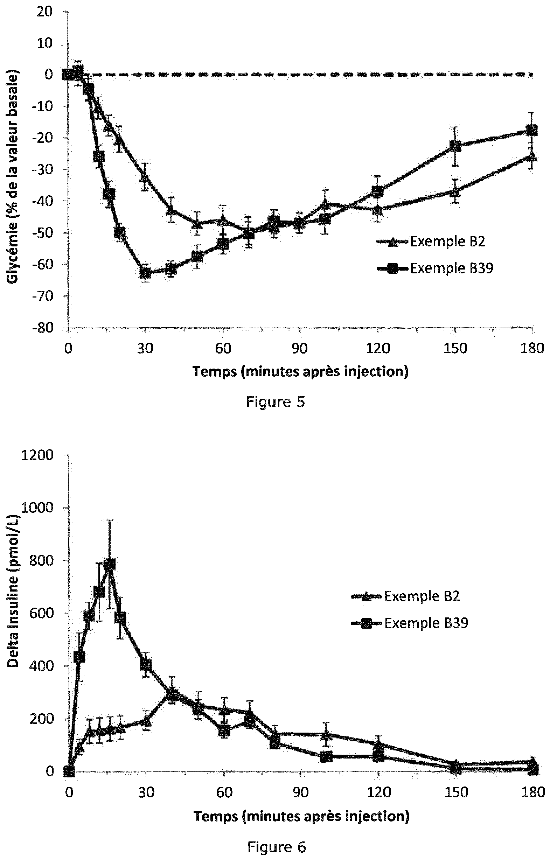

Figure 5 : Glycémie (% de la valeur basale) en fonction du temps (minutes après injection). L'analyse de ces courbes montre que la composition de l'exemple B39 comprenant le citrate substitué A2 et le citrate comme excipient (courbe tracée avec les carrés correspondant à l'exemple B39, Tmin glucose = 37 ± 12 min et T50%Rmin glucose = 14 ± 3 min) permet d'obtenir une action plus rapide que celle de la composition commerciale Humalog® de l'exemple B2 (courbe tracée avec les triangles correspondant à l'exemple B2, Tmin glucose = 76 ± 40 min et T50%Rmin glucose = 29 ± 13 min).
Figure 6 : Delta insuline (pmol/L) en fonction du temps (minutes après injection). L'analyse de ces courbes montre que la composition de l'exemple B39 comprenant le citrate substitué A2 et le citrate comme excipient (courbe tracée avec les carrés correspondant à l'exemple B39, Tmax insuline = 34 ± 26 min et T50%Cmax insuline = 9 ± 6 min) induit une absorption plus rapide de l'insuline lispro que la composition commerciale Humalog® de l'exemple B2 (courbe tracée avec les triangles correspondant à l'exemple B2, Tmax insuline = 43 ± 24 min et T50%Cmax insuline = 20 ± 19 min).

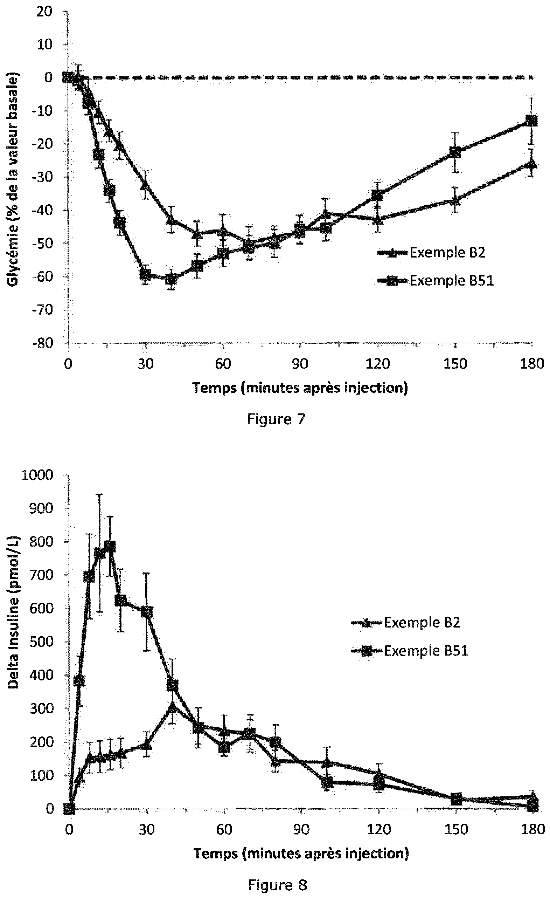

Figure 7 : Glycémie (% de la valeur basale) en fonction du temps (minutes après injection). L'analyse de ces courbes montre que la composition de l'exemple B51 comprenant le citrate substitué A3 et le citrate comme excipient (courbe tracée avec les carrés correspondant à l'exemple B51, Tmin glucose = 46 ± 14 min et T50%Rmin glucose = 15 ± 4 min) permet d'obtenir une action plus rapide que celle de la composition commerciale Humalog® de l'exemple B2 (courbe tracée avec les triangles correspondant à l'exemple B2, Tmin glucose = 76 ± 40 min et T50%Rmin glucose = 29 ± 13 min).
Figure 8 : Delta insuline (pmol/L) en fonction du temps (minutes après injection). L'analyse de ces courbes montre que la composition de l'exemple B51 comprenant le citrate substitué A3 et le citrate comme excipient (courbe tracée avec les carrés correspondant à l'exemple B51, Tmax insuline = 17 ± 9 min et T50%Cmax insuline = 6 ± 3 min) induit une absorption plus rapide de l'insuline lispro que la composition commerciale Humalog® de l'exemple B2 (courbe tracée avec les triangles correspondant à l'exemple B2, Tmax insuline = 43 ± 24 min et T50%Cmax insuline = 20 ± 19 min).

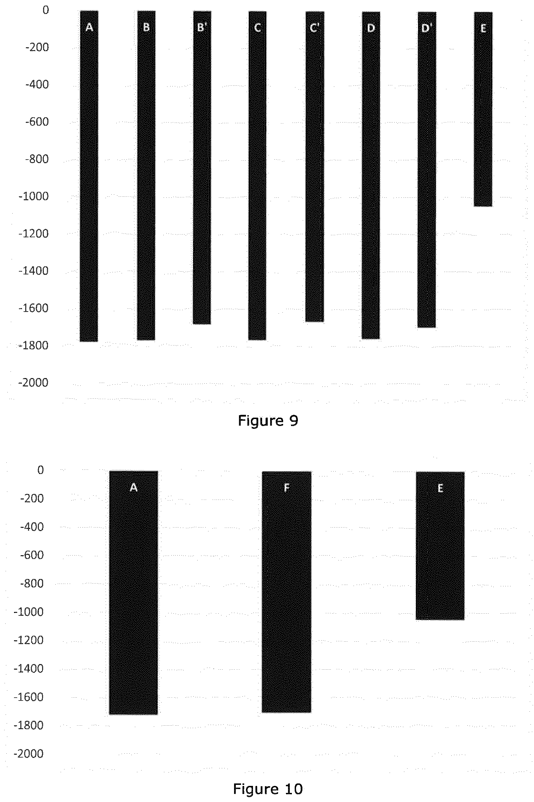

Figure 9 : La figure 9 décrit en ordonnée le signal CD à 240 nm (deg.cm².dmol⁻¹) et en abscisse :
   A : insuline lispro 100 UI/ml
   B : insuline lispro 100 UI/ml + 7,3 mg de citrate substitué A1
   B' : insuline lispro 100 UI/ml + 7,3 mg de citrate substitué A1 et citrate à 9,3 mM
   C : insuline lispro 100 UI/ml + 7,3 mg de citrate substitué A2
   C' : insuline lispro 100 UI/ml + 7,3 mg de citrate substitué A2 et citrate à 9,3 mM
   D : insuline lispro 100 UI/ml + 7,3 mg de citrate substitué A3
   D' : insuline lispro 100 UI/ml + 7,3 mg de citrate substitué A3 et citrate à 9,3 mM
   E : insuline lispro + EDTA à 300 µM
Figure 10 : La figure 10 décrit en ordonnée le signal CD à 240 nm (deg.cm².dmol⁻¹) et en abscisse :
   A : insuline lispro 100 UI/ml
   F : insuline lispro 100 UI/ml + 14.6 mg de citrate substitué A1
   E : Insuline Lispro + EDTA à 300 µM
Figure 11 : Glycémie (% de la valeur basale) en fonction du temps (minutes après injection). L'analyse de ces courbes montre que la composition de l'exemple B62 comprenant le citrate substitué A3 (courbe tracée avec les carrés correspondant à l'exemple B62, Tmin glucose = 46 ± 24 min et T50%Rmin glucose = 16 ± 6 min) permet d'obtenir une action plus rapide que celle de la composition commerciale Humalog® de l'exemple B2 (courbe tracée avec les triangles correspondant à l'exemple B2, Tmin glucose = 63 ± 35 min et T50%Rmin glucose = 19 ± 6 min).
Figure 12 : Delta insuline (pmol/L) en fonction du temps (minutes après injection). L'analyse de ces courbes montre que la composition de l'exemple B62 comprenant le citrate substitué A3 (courbe tracée avec les carrés correspondant à l'exemple B62, Tmax insuline = 24 ± 20 min et T50%Cmax insuline = 7 ± 4 min) induit une absorption plus rapide de l'insuline lispro que la composition commerciale Humalog® de l'exemple B2 (courbe tracée avec les triangles correspondant à l'exemple B2, Tmax insuline = 26 + 19 min et T50%Cmax insuline = 9 ± 7 min).
Figure 13 : La figure 13 décrit en ordonnée le signal CD à 240 nm (deg.cm².dmol⁻¹) et en abscisse :
   A : insuline lispro 100 UI/ml
   D : insuline lispro 100 UI/ml + 7,3 mg/ml de citrate substitué A3
   D' : insuline lispro 100 UI/ml + 7,3 mg/ml de citrate substitué A3 et citrate à 9,3 mM
   E : insuline lispro + EDTA à 300 µM
Figure 14 : La figure 14 décrit en ordonnée le signal CD à 240 nm (deg.cm².dmol⁻¹) et en abscisse :
   A : insuline lispro 100 UI/ml
   E : Insuline Lispro + EDTA à 300 µM
   G : insuline lispro 100 UI/ml + 14,6 mg/ml de citrate substitué A3
Figure 15 : La figure 15 décrit en ordonnée le signal CD à 240 nm (deg.cm².dmol-¹) et en abscisse :
   A : insuline humaine 100 UI/ml
   B : insuline humaine 100 UI/ml + 7,3 mg/ml de citrate substitué A3
   C : insuline humaine 100 UI/ml + 7,3 mg/ml de citrate substitué A3 et citrate à 9,3 mM
   D : insuline humaine 100 UI/ml + EDTA à 300 µM
   D' : insuline humaine 100 UI/ml + EDTA à 6 mM
Figure 16 : La figure 16 décrit en ordonnée le signal CD à 240 nm (deg.cm².dmol⁻¹) et en abscisse :
   A : insuline humaine 100 UI/ml (Humulin® R)
   B : insuline humaine 100 UI/ml + EDTA à 300 µM
   B': insuline humaine 100 UI/ml + EDTA à 6 mM
   C : insuline humaine 100 UI/ml + 14,6 mg/ml de citrate substitué A3.

### Exemples.

Le Tableau 1 ci-après présente, de façon non limitative, des exemples de composés selon l'invention.

**Tableau 1**

| citrates substitués | Formules |
|---|---|
| A1 | 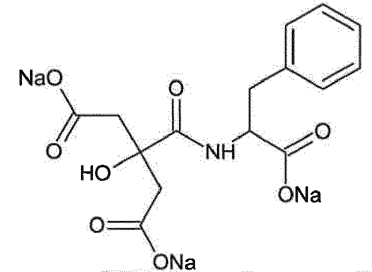 |
| A2 | 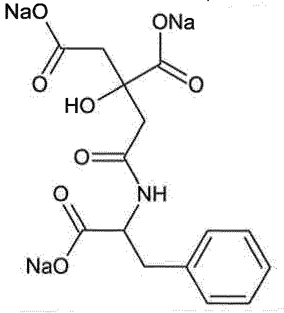 |
| A3 | 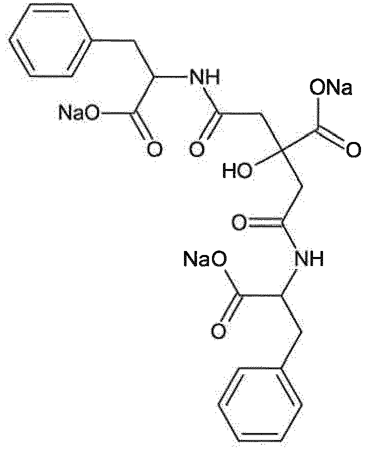 |
| A4 | 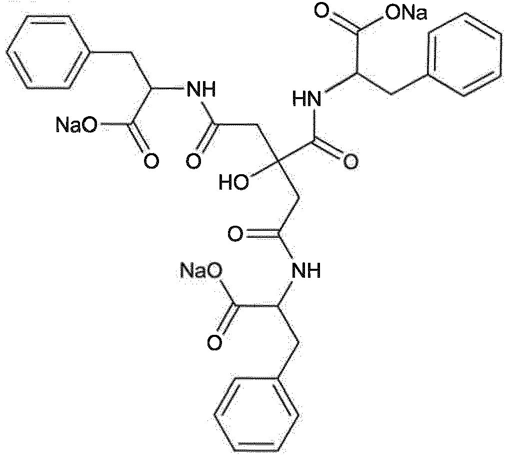 |

**Tableau 2 : contre-exemples**

| Composés de l'art antérieur | Formules | Nom CAS Origine |
|---|---|---|
| CE1 | 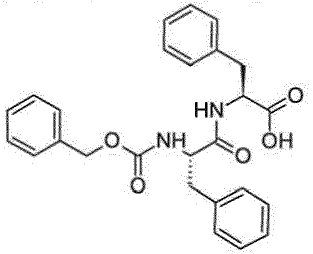 | CAS : 13122-91-3 N-Carbobenzoxy-L-phenylalanyl-L-phenylalanine Fournisseur : TCI |
| CE2 | 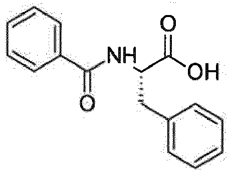 | CAS : 2566-22-5 Benzoyl-L-phenylalanine Fournisseur : BACHEM |

### Exemple A1 : citrate substitué A1

### Molécule 1 : acide 1,3-diméthyle citrate

Une solution d'acide citrique (50 g, 260 mmol), d'acide borique (1,5 g, 24,7 mmol) dans 70 mL de méthanol et 80 mL d'acétone est agitée à température ambiante pendant 3 jours. De l'acétone (80 mL) est ajouté au milieu réactionnel qui est refroidi à 0°C pendant 2 h. Le solide est récupéré par filtration sur fritté, lavé avec un mélange acétone/dichlorométhane (1/1) et séché sous vide.
Rendement : 48,8 g (85%)
RMN ¹H (DMSO-d₆, ppm) : 2,74 (2H) ; 2,85 (2H) ; 3,56 (6H).
LC/MS (ESI): 221,2 ; (calculé ([M+H]⁺): 221,2).

### Molécule 2 : produit obtenu par la réaction entre la molécule 1 et le sel chlorhydrate de l'éthyle ester de la L-phénylalanine

A une solution de molécule 1 (25 g, 113,5 mmol) dans le tétrahydrofuranne (THF) (1,1 L) à 0°C sont ajoutés successivement du dicyclohexyle carbodiimide (DCC) (24,6 g, 119,2 mmol) et du N-hydroxysuccinimide (NHS) (13,72 g, 119,2 mmol). Après 16 h d'agitation à température ambiante, le milieu est refroidi à 0°C pendant 20 min, filtré sur fritté et ajouté à une suspension de sel chlorhydrate de l'éthyle ester de la L-phénylalanine (26,8 g, 113,5 mmol) et de triéthylamine (110,8 mL, 794,8 mmol) dans le THF (150 mL). 50 mL sont ajoutés pour avoir un milieu homogène et la solution est agitée à température ambiante pendant 1 h. Le milieu est évaporé sous vide, dilué avec de l'acétate d'éthyle et lavé avec une solution aqueuse saturée de NaHCO₃. La phase aqueuse est extraite à l'acétate d'éthyle. Les phases organiques combinées sont lavées avec une solution aqueuse de HCl 10% puis une solution aqueuse saturée en NaCl. Après séchage sur Na₂SO₄, la phase organique est filtrée, concentrée sous vide et le résidu est purifié par chromatographie flash (cyclohexane, acétate d'éthyle) pour donner une huile incolore.
Rendement : 20,4 g (45%)
RMN ¹H (DMSO-d₆, ppm) : 1,26 (3H) ; 2,78-2,90 (4H) ; 3,14 (2H) ; 3,70 (6H) ; 4,15 (2H) ; 4,82 (1H) ; 4,93 (1H) ; 7,20-7,33 (5H) ; 7,40 (1H).
LC/MS (ESI): 396,3 ; (calculé ([M+H]⁺): 396,4).

### Citrate substitué A1

A une solution de molécule 2 (20,34 g, 51,44 mmol) dans le méthanol (514 mL) à 0°C est ajoutée une solution aqueuse de soude 2N (128,6 mL). Le mélange est agité à 0°C pendant 2 h puis à température ambiante pendant 2 h. 25,7 mL de soude 2N supplémentaires sont ajoutés et le milieu est agité à température ambiante pendant 2 h. Le solide formé est filtré sur fritté, azéotropé à l'eau (150 mL) et séché sous vide pour donner un solide blanc de citrate substitué A1.
Rendement : 13,6 g (64%)
RMN ¹H (DMSO-d₆, ppm) : 1,95-2,65 (4H) ; 2,99 (1H) ; 3,20 (1H) ; 4,49 (1H) ; 7,30-7,42 (5H).
LC/MS (ESI): 340,1 ; (calculé ([M+4H-3Na]⁺): 340,2).

### Exemple A2 : citrate substitué A2

### Molécule 3 : tert-butyle ester de la molécule 1

A une solution de la molécule 1 (7 g, 31,8 mmol) dans le dichlorométhane (318 mL) à température ambiante est ajouté le tert-butyl-2,2,2-trichloroacétimidate (9,3 mL). Après 16 h d'agitation, une portion supplémentaire de tert-butyl-2,2,2-trichloroacétimidate (3,09 mL) est ajoutée. Après 6 h d'agitation, une portion supplémentaire de *tert*-butyl-2,2,2-trichloroacétimidate (3,09 mL) est ajoutée. Après 16 h d'agitation, le milieu est évaporé sous vide à sec pour donner un solide qui est repris dans l'hexane (100 mL). La suspension est agitée à chaud pendant 30 min, refroidie à 5°C et filtrée sur fritté. Le solide est rincé avec du dichlorométhane (50 mL) et le filtrat est évaporé sous vide pour donner une huile qui est purifiée par chromatographie flash (cyclohexane, acétate d'éthyle).
Rendement : 4,76 g (50%)
RMN ¹H (CDCl₃, ppm) : 1,48 (9H) ; 2,70-2,86 (4H) ; 3,67 (6H) ; 4,02 (1H).
RMN ¹³C (CDCl₃, ppm) : 27,67 ; 43,27 ; 51,75 ; 72,98 ; 83,22 ; 170,04 ; 172,23.

### Molécule 4: produit obtenu par saponification de la molécule 3

A une solution de molécule 3 (3,35 g, 12,13 mmol) dans le méthanol (120 mL) à 0°C est ajoutée une solution aqueuse de soude 2N (18,2 mL) préalablement refroidie à 0°C. Le milieu est agité à 0°C pendant 15 min puis à température ambiante pendant 5 h. Après évaporation de la phase organique sous vide, le milieu est dilué avec de l'eau (50 mL) et la phase aqueuse est lavée avec de l'acétate d'éthyle (100 mL) puis acidifiée par une solution aqueuse d'HCI 10% et extraite avec de l'acétate d'éthyle (2x150 mL). La phase organique est lavée avec de l'eau (100 mL), une solution aqueuse saturée en NaCl (150 mL) puis séchée sur Na₂SO₄, filtrée et évaporée sous vide pour donner un solide blanc. La phase aqueuse est de nouveau extraite à l'acétate d'éthyle (2x150 mL), séchée sur Na₂SO₄, filtrée et évaporée sous vide pour donner une deuxième portion du produit désiré.
Rendement : 15,6 g (86%)
RMN ¹H (CD₃OD, ppm) : 1,46 (9H) ; 2,71 (2H) ; 2,86 (2H).
LC/MS (ESI): 247,1 ; (calculé ([M-H]⁻): 247,3).

### Molécule 5 : produit obtenu par la réaction entre la molécule 4 et le DCC

A une solution de molécule 4 (15,6 g, 62,8 mmol) dans un mélange dichlorométhane/THF (310/100 mL) est ajouté le DCC (14,26 g, 69,1 mmol) et le milieu est agité pendant 5 h à température ambiante. Après filtration sur célite, le milieu est évaporé à sec. Le résidu est agité à chaud dans un mélange hexane/acétate d'éthyle (320/80 mL) puis refroidi à -20°C sur une nuit. Après filtration sur fritté, un solide orangé est isolé.
Rendement : 13,3 g (91%)
RMN ¹H (CDCl₃, ppm) : 1,50 (9H) ; 2,94 (2H) ; 3,03 (2H).

### Molécule 6 : produit obtenu par la réaction entre la molécule 5 et le sel chlorhydrate du tert-butyle ester de la L-phénylalanine

A une solution de sel chlorhydrate du tert-butyle ester de la L-phénylalanine (16,4 g, 63,55 mmol) dans le dichlorométhane (100 mL) est ajoutée une solution aqueuse saturée de NaHCO₃ (150 mL). La phase organique est séparée et la phase aqueuse est extraite au dichlorométhane (50 mL). Les phases organiques combinées sont séchées sur Na₂SO₄, filtrées et partiellement concentrées sous vide à un volume de 40 mL. La solution obtenue est coulée sur une solution de molécule 5 (13,3 g, 57,8 mmol) dans le THF (577 mL) à 0°C. Après 3 h d'agitation à 0°C, le milieu est concentré sous vide. Le résidu est solubilisé dans l'acétate d'éthyle (200 mL), filtré sur membrane PTFE (0,45 µm), lavé avec une solution aqueuse saturée en NH₄Cl (100 mL), de l'eau (100 mL) et une solution aqueuse saturée en NaCl (100 mL). Après séchage sur Na₂SO₄ et filtration, la phase organique est évaporée sous vide. Le résidu est purifié par chromatographie flash (cyclohexane, acétate d'éthyle). L'huile obtenue est azéotropée à l'eau et à l'acétone pour donner un solide beige.
Rendement : 24,3 g (93%)
RMN ¹H (CDCl₃, ppm) : 1,40 (9H) ; 1,45 (9H) ; 2,60-2,85 (4H) ; 3,08 (2H) ; 4,73 (1H) ; 6,73 (1H) ; 7,15-7,30 (5H).
LC/MS (ESI): 453,2 ; (calculé ([M+H]⁺): 452,5).

### Citrate substitué A2

A une solution de molécule 6 (24,3 g, 53,8 mmol) dans le dichlorométhane (110 mL) à 0°C est ajouté de l'acide trifluoroacétique (TFA) (82,4 mL). Le milieu est laissé remonter à température ambiante et est agité pendant 5 h. Après évaporation à sec sous vide, le résidu est solubilisé dans l'acétate d'éthyle (100 mL), refroidi à -20°C pendant 1 h et filtré sur une membrane PVDF (0,45 µm). Le filtrat est concentré sous vide pour donner le citrate substitué A2 sous forme acide.
Rendement : 18,4 g (quantitatif)
RMN ¹H (DMSO-d₆, ppm) : 2,50-2,65 (4H) ; 2,90 (1H) ; 3,02 (1H) ; 4,43 (1H) ; 7,20-7,30 (5H) ; 8,24 (1H).
LC/MS (ESI): 338,0 ; (calculé ([M-H]⁻): 338,3).

A une suspension de citrate substitué A2 sous forme acide (18,2 g, 53,6 mmol) dans 250 mL d'eau est ajoutée au goutte à goutte une solution aqueuse de soude 2N (80,5 mL). La solution est évaporée. Le résidu est solubilisé dans de l'eau à reflux (40 mL) puis de l'éthanol (400 mL) est ajouté. Le milieu qui prend en masse est agité à chaud pendant 15 min puis est refroidi dans un bain de glace. Le précipité est récupéré par filtration sur fritté puis repris dans de l'eau pour donner un solide. Après 3 traitements eau/éthanol/filtration, un solide blanc de citrate substitué A2 est obtenu.
Rendement : 11,74 g (54%)
RMN ¹H (D₂O, ppm) : 2,10-2,70 (4H) ; 2,90 (1H) ; 3,02 (1H) ; 3,16 (1H) ; 4,48 (1H) ; 7,32-7,42 (5H).

### Exemple A3 : citrate substitué A3

### Molécule 7 : produit obtenu par la réaction entre la molécule 4 et le sel chlorhydrate du tert-butyle ester de la L-phénylalanine

A une solution de molécule 4 (1,0 g, 4,03 mmol) dans le THF (40 mL) sont ajoutés successivement du DCC (2,49 g, 12,09 mmol) et du NHS (1,27 g, 11,08 mmol). Après 4 h d'agitation à température ambiante, le milieu est évaporé sous vide pour donner un solide qui est repris dans du dioxane sec et est refroidi à 0°C.

Le sel chlorhydrate du tert-butyle ester de la L-phénylalanine (2,28 g, 8,85 mmol) est solubilisé dans un mélange dichlorométhane/solution aqueuse saturée de NaHCO₃ (15/20 mL). Après séparation des phases, la phase aqueuse est extraite par le dichlorométhane. Les phases organiques sont séchées sur Na₂SO₄ et filtrées. La solution obtenue est ajoutée au goutte-à-goutte à la solution de molécule 4 activée à 0°C. Le mélange est laissé revenir à température ambiante et est agité pendant 16 h. Trois équivalents supplémentaires d'amine libre préparée comme ci-dessus dans 20 mL de dichlorométhane sont alors ajoutés à 24 h d'intervalle. Le milieu réactionnel est filtré sur fritté et les solvants organiques sont évaporés sous vide. Le milieu est dilué avec de l'acétate d'éthyle (100 mL), lavé avec une solution aqueuse de HCl 10% (50 mL), une solution aqueuse saturée de NaHCO₃ (50 mL) et une solution aqueuse saturée en NaCl (50 mL). Après séchage sur Na₂SO₄, la phase organique est filtrée et concentrée sous vide pour donner une huile. Le résidu est purifié par chromatographie flash (cyclohexane, acétate d'éthyle) pour donner une huile jaune qui est reprise dans de l'acétate d'éthyle (20 mL). La solution est refroidie à -20°C pendant 1 h, filtrée sur membrane PVDF (0,45 µm). Le filtrat est évaporé sous vide pour donner une laque jaune.
Rendement : 1,84 g (70%)
RMN ¹H (CDCl₃, ppm) : 1,43 (18H) ; 1,47 (9H) ; 2,49 (1H) ; 2,60 (3H) ; 2,95-3,10 (4H) ; 4,68 (2H) ; 5,02 (1H) ; 7,13 (1H) ; 7,19-7,30 (12H).
LC/MS (ESI): 655,5 ; (calculé ([M+H]⁺): 655,8).

### Citrate substitué A3

A une solution de molécule 7 (1,72 g, 2,63 mmol) dans le dichlorométhane (13 mL) à 0°C est ajouté du TFA (5,7 mL). Le milieu est laissé remonter à température ambiante et est agité pendant 5 h. Le milieu est refroidi à -20°C pendant une nuit puis remis à température ambiante et 2 mL supplémentaires de TFA sont ajoutés. Après 1 h d'agitation et évaporation sous vide à 30°C, le milieu est azéotropé à l'eau (50 mL). Le résidu est solubilisé dans l'acétate d'éthyle (20 mL) et la phase organique est extraite avec une solution aqueuse de soude 1N (20 mL). La phase aqueuse est acidifiée avec une solution aqueuse de HCl 10% et extraite à l'acétate d'éthyle (30 mL). La phase organique est lavée avec de l'eau (25 mL), une solution aqueuse saturée en NaCl (2x25 mL), séchée sur Na₂SO₄, filtrée et évaporée sous vide pour donner le citrate substitué A3 sous forme acide.
Rendement : 0,83 g (64%)
RMN ¹H (DMSO-d₆, ppm) : 2,44-2,56 (4H) ; 2,87 (2H) ; 2,99 (2H) ; 4,41 (2H) ; 7,19-7,30 (10H) ; 8,19 (2H).
LC/MS (ESI): 487,2 ; (calculé ([M+H]⁺): 487,5).

Après un azéotrope à l'eau (2x50 mL), le citrate substitué A3 sous forme acide (828 mg, 1,7 mmol) est dissous dans 50 mL d'eau et une solution de soude 2N est ajoutée au goutte-à-goutte jusqu'à pH 7,27. La solution est évaporée à sec et le solide obtenu est trituré dans un mélange éthanol/eau (10/1 mL) à chaud pendant 30 min. Après refroidissement à l'aide d'un bain de glace, la suspension est filtrée sur fritté. Le solide obtenu est azéotropé dans l'eau (150 mL). Après séchage sous vide, le solide est dissous dans 2,5 mL d'eau à chaud puis 25 mL d'éthanol sont ajoutés à chaud. Après refroidissement à -20°C pendant 1 h, le précipité formé est récupéré par filtration sur fritté et azéotropé dans l'eau (50 mL). Après séchage sous cloche (16 h, 55°C, 0,2 mbar), un solide blanc de citrate substitué A3 est obtenu.
Rendement : 396 mg (42%)
RMN ¹H (D₂O, ppm) : 2,22-2,31 (2H) ; 2,50 (2H) ; 2,94-3,01 (2H) ; 3,20 (2H) ; 4,48 (2H) ; 7,26-7,39 (10H).

### Exemple A4 : citrate substitué A4

### Molécule 8 : produit obtenu par la réaction entre l'acide citrique et le sel chlorhydrate de l'éthyle ester de la L-phénylalanine

A une solution d'acide citrique (1,0 g, 5,20 mmol) dans le N,N-diméthylformamide (DMF) (50 mL) sont ajoutés successivement de la diisopropyléthylamine (DIPEA) (2,69 g, 20,8 mmol), du sel chlorhydrate de 1-éthyl-3-(3-diméthylaminopropyl)carbodiimide (EDCI.HCl) (3,29 g, 17,18 mmol) et du 2-hydroxypyridine-N-oxyde (HOPO) (1,82 g, 16,4 mmol). Après 5 min d'agitation, le sel chlorhydrate de l'éthyle ester de la L-phénylalanine (3,95 g, 17,18 mmol) est ajouté et le milieu réactionnel est agité à température ambiante. Après 16 h d'agitation, le DMF est évaporé sous vide. Le résidu est dilué dans l'acétate d'éthyle (250 mL) et lavé avec une solution aqueuse saturée de NaHCO₃ (2 x 120 mL). Les phases sont séparées. La phase aqueuse est lavée à l'acétate d'éthyle (50 mL). Les phases organiques combinées sont lavées avec une solution aqueuse de HCl 1N (120 mL), de l'eau (2 x 120 mL), une solution aqueuse saturée de NaCl (120 mL). Après séchage sur Na₂SO₄, la phase organique est filtrée et concentrée sous vide. Le résidu est purifié par chromatographie flash (cyclohexane, acétate d'éthyle) pour donner un solide blanc après évaporation sous vide suivi d'un azéotrope avec du dichlorométhane par deux fois.
Rendement : 2,49 g (66%)
RMN ¹H (CDCl₃, ppm) : 1,21 (9H) ; 2,47 (1H) ; 2,58 (3H) ; 3,03 (6H) ; 4,14 (6H) ; 4,75 (3H) ; 6,09 (1H) ; 6,86 (2H) ; 7,14 (6H) ; 7,20-7,35 (9H) ; 7,50 (1H).
LC/MS (ESI): 718,6 ; (calculé ([M+H]⁺): 718,8).

### Citrate substitué A4

A une solution de molécule 8 (2,77 g, 3,86 mmol) dans un mélange THF/méthanol (1/1, 26 mL) refroidie à 0°C est ajoutée une solution d'hydrate d'hydroxyde de lithium (LiOH.H₂O) (0,57 g, 13,51 mmol) dans l'eau (13 mL). Le milieu réactionnel est agité à 0°C. Après 3 h d'agitation, les solvants sont évaporés sous vide et le résidu aqueux est acidifié jusqu'à pH 1 par ajout d'une solution aqueuse de HCl 1N. La phase aqueuse est extraite avec du méthyl tert-butyl éther (MTBE) (4 x 20 mL). Les phases organiques sont rassemblées, lavées avec une solution aqueuse saturée de NaCl (20 mL), séchées sur Na₂SO₄, filtrées et concentrées sous vide. Le résidu est repris dans l'acétonitrile (120 mL) et est co-évaporé avec du MTBE pour donner un solide blanc de citrate substitué A4 sous forme acide après séchage sous vide.
Rendement : 2,35 g (66%)
RMN ¹H (DMSO-d₆, ppm) : 2,34-2,49 (4H) ; 2,85 (2H) ; 2,97 (4H) ; 4,42 (3H) ; 6,25 (1H) ; 7,15-7,21 (15H) ; 7,58 (1H) ; 8,19 (1H) ; 8,24 (1H) ; 12,77 (3H).
LC/MS (ESI): 634,5 ; (calculé ([M+H]⁺): 634,6).

A une solution de citrate substitué A4 sous forme acide (2,35 g, 3,71 mmol) dans l'eau (100 mL) est ajoutée au goutte-à-goutte une solution aqueuse de soude 2M jusqu'à pH 7,04. La solution est alors filtrée sur une membrane en nitrocellulose 0,22 µm, congelée et lyophilisée pour donner un solide blanc de citrate substitué A4.
Rendement : 2,57 g (99%)
RMN ¹H (D₂O, ppm) : 1,94 (1H) ; 2,35 (2H) ; 2,54 (1H) ; 2,88-3,13 (5H) ; 3,20 (1H) ; 4,37-4,43 (2H) ; 4,50 (1H) ; 7,18-7,36 (15H).

### Partie B : Préparation des solutions

### B1. Solution d'insuline analogue rapide Novoloa® à 100 UI/mL

Cette solution est une solution commerciale d'insuline aspart de Novo Nordisk vendue sous le nom de Novolog®. Ce produit est une insuline analogue rapide.

### B2. Solution d'insuline analogue rapide Humalog® à 100 UI/mL

Cette solution est une solution commerciale d'insuline lispro de Eli Lilly vendue sous le nom de Humalog® U100. Ce produit est une insuline analogue rapide. Dans le présent texte lorsque le terme Humalog est utilisé sans autre précision, il fait référence à l'Humalog® U100 et lorsque l'expression « la formulation commercial d'insuline lispro » est utilisée sans autre précision, elle fait référence à la formulation commerciale d'insuline lispro à 100 UI/ml.

### B3. Solution d'insuline humaine Humulin® R à 100 UI/mL

Cette solution est une solution commerciale d'insuline humaine de Eli Lilly vendue sous le nom d'Humulin® R. Ce produit est une composition d'insuline humaine.

### B4. Solution d'insuline analogue rapide Apidra® à 100 UI/mL

Cette solution est une solution commerciale d'insuline glulisine de Sanofi vendue sous le nom d'Apidra®. Ce produit est une insuline analogue rapide.

### B5. Préparation d'une solution de citrate de sodium à 1,188 M

La solution de citrate de sodium est obtenue en solubilisant 9,0811 g de citrate de sodium (30,9 mmol) dans 25 mL d'eau dans une fiole jaugée. Le pH est ajusté à 7,4 par ajout de 1 mL d'HCI 1M. La solution est filtrée sur 0,22 µm.

### B6. Préparation d'une solution d'insuline analogue (insuline lispro) à 200 UI/mL.

La formulation commerciale d'insuline lispro (Humalog® U100) a été concentrée en utilisant des tubes de centrifugation AMICON Ultra-15 avec une coupure à 3 kDa. Les tubes amicon ont tout d'abord été rincés avec 12 mL d'eau déionisée. 12 mL de la formulation commerciale ont été centrifugés pendant 35 minutes à 4000 g à 20°C. Le volume du rétentat a été mesuré et la concentration ainsi estimée. Tous les rétentats ont été mis en commun et la concentration globale a été estimée (> 200 UI/mL).

La concentration de cette solution d'insuline lispro concentrée a été ajustée à 200 UI/mL par addition de la formulation commerciale d'insuline lispro (Humalog®). La formulation d'insuline lispro concentrée présente les mêmes concentrations en excipients (m-crésol, glycérine, phosphate) que la formulation commerciale à 100 UI/mL.

Le pH final est ajusté à 7,4 ± 0,4. La solution limpide est filtrée sur une membrane 0,22 µm et stockée à 4°C.

### B7_{.} Préparation d'une solution d'insuline humaine à 200 UI/mL.

La formulation commerciale d'insuline humaine (Humulin® R) a été concentrée en utilisant des tubes de centrifugation AMICON Ultra-15 avec une coupure à 3 kDa. Les tubes amicon ont tout d'abord été rincés avec 12 mL d'eau déionisée. 12 mL de la formulation commerciale ont été centrifugés pendant 35 minutes à 4000 g à 20°C. Le volume du rétentat a été mesuré et la concentration ainsi estimée. Tous les rétentats ont été mis en commun et la concentration globale a été estimée (> 200 UI/mL).

La concentration de cette solution d'insuline humaine concentrée a été ajustée à 200 UI/mL par addition de la formulation commerciale d'insuline humaine (Humulin® R). La formulation d'insuline humaine concentrée présente les mêmes concentrations en excipients (m-crésol, glycérine) que la formulation commerciale à 100 UI/mL.

Le pH final est ajusté à 7,4 ± 0,4. La solution limpide est filtrée sur une membrane 0,22 µm et stockée à 4°C.

### B8. Préparation d'une solution d'insuline aspart à 200 UI/mL.

La formulation commerciale d'insuline aspart (Novolog®) a été concentrée en utilisant des tubes de centrifugation AMICON Ultra-15 avec une coupure à 3 kDa. Les tubes amicon ont tout d'abord été rincés avec 12 mL d'eau déionisée. 12 mL de la formulation commerciale ont été centrifugés pendant 35 minutes à 4000 g à 20°C. Le volume du rétentat a été mesuré et la concentration ainsi estimée. Tous les rétentats ont été mis en commun et la concentration globale a été estimée (> 200 UI/mL).

La concentration de cette solution d'insuline aspart concentrée a été ajustée à 200 UI/mL par addition de la formulation commerciale d'insuline aspart (Novolog®). La formulation concentrée d'insuline aspart concentrée présente les mêmes concentrations en excipients (m-crésol, glycérine) que la formulation commerciale à 100 UI/mL.

Le pH final est ajusté à 7,4 ± 0,4. La solution limpide est filtrée sur une membrane 0,22 µm et stockée à 4°C.

### B9_{.} Préparation d'une solution d'insuline glulisine à 200 UI/mL.

La formulation commerciale d'insuline glulisine (Apidra®) a été concentrée en utilisant des tubes de centrifugation AMICON Ultra-15 avec une coupure à 3 kDa. Les tubes amicon ont tout d'abord été rincés avec 12 mL d'eau déionisée. 12 mL de la formulation commerciale ont été centrifugés pendant 35 minutes à 4000 g à 20°C. Le volume du rétentat a été mesuré et la concentration ainsi estimée. Tous les rétentats ont été mis en commun et la concentration globale a été estimée (> 200 UI/mL).

La concentration de cette solution d'insuline glulisine concentrée a été ajustée à 200 UI/mL par addition de la formulation commerciale d'insuline glulisine (Apidra®). La formulation d'insuline glulisine concentrée présente les mêmes concentrations en excipients (m-crésol, NaCl, TRIS) que la formulation commerciale à 100 UI/mL.

Le pH final est ajusté à 7,4 ± 0,4. La solution limpide est filtrée sur une membrane 0,22 µm et stockée à 4°C.

### B10. Préparation d'une solution d'insuline humaine, d'insuline lispro, d'insuline aspart ou d'insuline glulisine à 300, 400 et 500 UI/mL.

Des formulations concentrées d'insuline humaine, d'insuline lispro, d'insuline aspart ou d'insuline glulisine à 300 UI/mL, 400 UI/mL ou 500 UI/mL (ainsi qu'à toutes concentrations intermédiaires) sont préparées sur la base du protocole de l'exemple B9 relatif à la préparation d'une solution d'insuline glulisine à 200 UI/mL. La formulation commerciale d'insuline est concentrée en utilisant des tubes de centrifugation AMICON Ultra-15 avec une coupure à 3 kDa. Les tubes amicon sont tout d'abord rincés avec 12 mL d'eau déionisée. 12 mL de la formulation commerciale sont centrifugés à 4000g et 20°C. En jouant sur le temps de centrifugation il est possible d'ajuster le volume final et donc la concentration finale en insuline dans la formulation. Le volume du rétentat est mesuré et la concentration ainsi estimée. Tous les rétentats sont mis en commun et la concentration globale est estimée (> 300, 400 ou 500 UI/mL).

La concentration de cette solution d'insuline concentrée est ajustée à la concentration désirée (e.g. 300 UI/mL, 400 UI/mL ou 500 UI/mL) par addition de la formulation d'insuline (Humulin® R, Novolog®, Humalog® ou Apidra®). La formulation concentrée d'insuline concentrée présente les mêmes concentrations en excipients que la formulation commerciale à 100 UI/mL.

Le pH final est ajusté à 7,4 ± 0,4. La solution limpide est filtrée sur une membrane 0,22 µm et stockée à 4°C.

### B11. Préparation d'une solution d'insuline lispro à 100 UI/mL en présence du citrate substitué A1 et de citrate

Pour un volume final de 100 mL de composition, avec un ratio massique [citrate substitué A1]/[insuline lispro] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A1) | 730 mg |
| - Solution commerciale d'Humalog® | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B12. Préparation d'une solution d'insuline humaine à 100 UI/mL en présence du citrate substitué A1 et de citrate

Pour un volume final de 100 mL de composition, avec un ratio massique [citrate substitué A1]/[insuline humaine] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A1) | 730 mg |
| - Solution commerciale d'Humulin® R | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B13. Préparation d'une solution d'insuline aspart à 100 UI/mL en présence du citrate substitué A1 et de citrate

Pour un volume final de 100 mL de composition, avec un ratio massique [citrate substitué A1]/[insuline aspart] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A1) | 730 mg |
| - Solution commerciale de Novolog® | 100 mL |
| - Solution de citrate de sodium à 1,188 M | 783 µL |

Le pH final est ajusté à 7,4 ± 0,4. La solution limpide est filtrée sur une membrane 0,22 µm et stockée à 4°C.

### B14. Préparation d'une solution d'insuline glulisine à 100 UI/mL en présence du citrate substitué A1 et de citrate

Pour un volume final de 100 mL de composition, avec un ratio massique [citrate substitué A1]/[insuline glulisine] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A1) | 730 mg |
| - Solution commerciale d'Apidra® | 100 mL |
| - Solution de citrate de sodium à 1,188M | 783 µL |

Le pH final est ajusté à 7,4 ± 0,4.

La solution limpide est filtré sur une membrane 0,22 µm et stockée à 4°C.

### B15_{.} Préparation d'une solution d'insuline lispro à 200 UI/mL en présence du citrate substitué A1 et de citrate.

Pour un volume final de 100 mL de formulation avec un ratio massique [citrate substitué A1]/[insuline lispro] de 2 et une concentration de 18,6 mM de citrate, les différents réactifs sont additionnés dans les quantités spécifiées ci-dessous et dans l'ordre suivant :

| | |
|---|---|
| - Composé lyophilisé (citrate substitué A1) | 1460 mg |
| - Insuline lispro à 200 UI/mL | 100 mL |
| - Solution de citrate de sodium à 1,188 M | 1566 µL |

Le pH final est ajusté à 7,4 ± 0,4.La solution limpide est filtrée sur une membrane 0,22 µm et stockée à 4°C.

### B16. Préparation d'une solution d'insuline humaine à 200 UI/mL en présence du citrate substitué A1 et de citrate.

Pour un volume final de 100 mL de formulation avec un ratio massique [citrate substitué A1]/[insuline humaine] de 2 et une concentration de 18,6 mM de citrate, les différents réactifs sont additionnés en quantités précisées ci-dessous et dans l'ordre suivant :

| | |
|---|---|
| - Composé lyophilisé (citrate substitué A1) | 1460 mg |
| - Insuline humaine à 200 UI/mL | 100 mL |
| - Solution de citrate de sodium à 1.188 M | 1566 µL |

Le pH final est ajusté à 7,4 ± 0,4. La solution limpide est filtrée sur une membrane 0,22 µm et stockée à 4°C.

### B17. Préparation d'une solution d'insuline aspart à 200 UI/mL en présence du citrate substitué A1 et de citrate.

Pour un volume final de 100 mL de formulation avec un ratio massique [citrate substitué A1]/[insuline aspart] de 2,0 et une concentration de 18,6 mM de citrate, les différents réactifs sont additionnés en quantités précisées ci-dessous et dans l'ordre suivant :

| | |
|---|---|
| - Composé lyophilisé (citrate substitué A1) | 1460 mg |
| - Insuline aspart à 200 UI/mL | 100 mL |
| - Solution de citrate de sodium à 1.188 M | 1566 µL |

Le pH final est ajusté à 7,4 ± 0,4. La solution limpide est filtrée sur une membrane 0,22 µm et stockée à 4°C.

### B18_{.} Préparation d'une solution d'insuline glulisine à 200 UI/mL en présence du citrate substitué A1 et de citrate.

Pour un volume final de 100 mL de formulation avec un ratio massique [citrate substitué A1]/[insuline glulisine] de 2 et une concentration de 18,6 mM de citrate, les différents réactifs sont additionnés en quantités précisées ci-dessous et dans l'ordre suivant :

| | |
|---|---|
| - Composé lyophilisé (citrate substitué A1) | 1460 mg |
| - Insuline glulisine à 200 UI/mL | 100 mL |
| - Solution de citrate de sodium à 1.188 M | 1566 µL |

Le pH final est ajusté à 7,4 ± 0,4. La solution limpide est filtrée sur une membrane 0,22 µm et stockée à 4°C.

### B19. Préparation d'une solution d'insuline lispro à 300 UI/mL en présence du citrate substitué A1 et de citrate.

Pour un volume final de 100 mL de formulation avec un ratio massique [citrate substitué A1]/[insuline lispro] de 2,0 et une concentration de 27,9 mM de citrate, les différents réactifs sont additionnés en quantités précisées ci-dessous et dans l'ordre suivant :

| | |
|---|---|
| Composé lyophilisé (citrate substitué A1) | 2190 mg |
| Insuline lispro à 300 UI/mL | 100 mL |
| Citrate de sodium | 720 mg |

Le pH final est ajusté à 7,4 ± 0,4. La solution limpide est filtrée sur une membrane 0,22 µm et stockée à 4°C.

### B20. Préparation d'une solution d'insuline humaine à 300 UI/mL en présence du citrate substitué A1 et de citrate.

Pour un volume final de 100 mL de formulation avec un ratio massique [citrate substitué A1]/[insuline humaine] de 2,0 et une concentration de 27,9 mM de citrate, les différents réactifs sont additionnés en quantités précisées ci-dessous et dans l'ordre suivant :

| | |
|---|---|
| - Composé lyophilisé (citrate substitué A1) | 2190 mg |
| - Insuline humaine à 300 UI/mL | 100 mL |
| - Citrate de sodium | 720 mg |

Le pH final est ajusté à 7,4 ± 0,4. La solution limpide est filtrée sur une membrane 0,22 µm et stockée à 4°C.

### B21. Préparation d'une solution d'insuline lispro à 400 UI/mL en présence du citrate substitué A1 et de citrate.

Pour un volume final de 100 mL de formulation avec un ratio massique [citrate substitué A1]/[insuline lispro] de 2,0 et une concentration de 37,2 mM de citrate, les différents réactifs sont additionnés en quantités précisées ci-dessous et dans l'ordre suivant :

| | |
|---|---|
| Composé lyophilisé (citrate substitué A1) | 2920 mg |
| Insuline lispro à 400 UI/mL | 100 mL |
| Citrate de sodium | 960 mg |

Le pH final est ajusté à 7,4 ± 0,4. La solution limpide est filtrée sur une membrane 0,22 µm et stockée à 4°C.

### B22_{.} Préparation d'une solution d'insuline humaine à 400 UI/mL en présence du citrate substitué A1 et de citrate.

Pour un volume final de 100 mL de formulation avec un ratio massique [citrate substitué A1]/[insuline humaine] de 2,0 et une concentration de 37,2 mM de citrate, les différents réactifs sont additionnés en quantités précisées ci-dessous et dans l'ordre suivant :

| | |
|---|---|
| Composé lyophilisé (citrate substitué A1) | 2920 mg |
| Insuline humaine à 400 UI/mL | 100 mL |
| Citrate de sodium | 960 mg |

Le pH final est ajusté à 7,4 ± 0,4. La solution limpide est filtrée sur une membrane 0,22 µm et stockée à 4°C.

### B23. Préparation d'une solution d'insuline lispro à 500 UI/mL en présence du citrate substitué A1 et de citrate.

Pour un volume final de 100 mL de formulation avec un ratio massique [citrate substitué A1]/[insuline lispro] de 2,0 et une concentration de 46,5 mM de citrate, les différents réactifs sont additionnés en quantités précisées ci-dessous et dans l'ordre suivant :

| | |
|---|---|
| Composé lyophilisé (citrate substitué A1) | 3650 mg |
| Insuline lispro à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B24. Préparation d'une solution d'insuline humaine à 500 UI/mL en présence du citrate substitué A1 et de citrate.

Pour un volume final de 100 mL de formulation avec un ratio massique [citrate substitué A1]/[insuline humaine] de 2,0 et une concentration de 46,5 mM de citrate, les différents réactifs sont additionnés en quantités précisées ci-dessous et dans l'ordre suivant :

| | |
|---|---|
| Composé lyophilisé (citrate substitué A1) | 3650 mg |
| Insuline humaine à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B25_{.} Préparation d'une solution d'insuline aspart à 500 UI/mL en présence du citrate substitué A1 et de citrate.

Pour un volume final de 100 mL de formulation avec un ratio massique [citrate substitué A1]/[insuline aspart] de 2,0 et une concentration de 46,5 mM de citrate, les différents réactifs sont additionnés en quantités précisées ci-dessous et dans l'ordre suivant :

| | |
|---|---|
| Composé lyophilisé (citrate substitué A1) | 3650 mg |
| Insuline aspart à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B26. Préparation d'une solution -d'insuline lispro à 100 UI/mL en présence du citrate substitué A1 à 14.6 mg/ml.

Pour un volume final de 100 mL de composition, avec un ratio massique [citrate substitué A1]/[insuline lispro] de 4,0, les différents réactifs sont additionnés dans les quantités spécifiées ci-dessous et dans l'ordre suivant :

| | |
|---|---|
| - Composé lyophilisé (citrate substitué A1) | 1460 mg |
| - Solution commerciale d'Humalog® | 100 mL |

Le pH final est ajusté à 7,4 ± 0,4.

La solution limpide est filtrée sur une membrane 0,22 µm et stockée à 4°C.

### B27_{.} Préparation d'une solution d'insuline humaine à 100 UI/mL en présence du citrate substitué A1 à 14.6 mg/ml.

Pour un volume final de 100 mL de composition, avec un ratio massique [citrate substitué A1]/[insuline humaine] de 4,0, les différents réactifs sont additionnés dans les quantités spécifiées ci-dessous et dans l'ordre suivant :

| | |
|---|---|
| - Composé lyophilisé (citrate substitué A1) | 1460 mg |
| - Solution commerciale d'Humulin® R | 100 mL |

Le pH final est ajusté à 7,4 ± 0,4.

La solution limpide est filtrée sur une membrane 0,22 µm et stockée à 4°C.

### B28. Préparation d'une solution d'insuline aspart à 100 UI/mL en présence du citrate substitué A1 à 14,6 mg/ml.

Pour un volume final de 100 mL de composition, avec un ratio massique [citrate substitué A1]/[insuline aspart] de 4,0, les différents réactifs sont additionnés dans les quantités spécifiés ci-dessous et dans l'ordre suivant :

| | |
|---|---|
| - Composé lyophilisé (citrate substitué A1) | 1460 mg |
| - Solution commerciale de Novolog® | 100 mL |

Le pH final est ajusté à 7,4 ± 0,4.

La solution limpide est filtrée sur une membrane 0,22 µm et stockée à 4°C

### B29. Préparation d'une solution d'insuline glulisine à 100 UI/mL en présence du citrate substitué A1 à 14,6 mg/ml.

Pour un volume final de 100 mL de composition, avec un ratio massique [citrate substitué A1]/[insuline glulisine] de 4,0, les différents réactifs sont additionnés dans les quantités spécifiées ci-dessous et dans l'ordre suivant :

| | |
|---|---|
| - Composé lyophilisé (citrate substitué A1) | 1460 mg |
| - Solution commerciale d'Apidra® | 100 mL |

Le pH final est ajusté à 7,4 ± 0,4.

La solution limpide est filtré sur une membrane 0,22 µm et stockée à 4°C.

### B30. Préparation d'une solution d'insuline lispro à 200 UI/mL en présence du citrate substitué A1 à 29,2 mg/ml.

Pour un volume final de 100 mL de formulation avec un ratio massique [citrate substitué A1]/[insuline lispro] de 4, les différents réactifs sont additionnés dans les quantités spécifiées ci-dessous et dans l'ordre suivant :

| | |
|---|---|
| - Composé lyophilisé (citrate substitué A1) | 2920 mg |
| - Insuline lispro à 200 UI/mL | 100 mL |

Le pH final est ajusté à 7,4 ± 0,4.La solution limpide est filtrée sur une membrane 0,22 µm et stockée à 4°C.

### B31_{.} Préparation d'une solution d'insuline humaine à 200 UI/mL en présence du citrate substitué A1 à 29,2 mg/ml.

Pour un volume final de 100 mL de formulation avec un ratio massique [citrate substitué A1]/[insuline humaine] de 4, les différents réactifs sont additionnés en quantités précisées ci-dessous et dans l'ordre suivant :

| | |
|---|---|
| - Composé lyophilisé (citrate substitué A1) | 2920 mg |
| - Insuline humaine à 200 UI/mL | 100 mL |

Le pH final est ajusté à 7,4 ± 0,4. La solution limpide est filtrée sur une membrane 0,22 µm et stockée à 4°C.

### B32. Préparation d'une solution d'insuline aspart à 200 UI/mL en présence du citrate substitué A1 à 29.2 mg/ml.

Pour un volume final de 100 mL de formulation avec un ratio massique [citrate substitué A1]/[insuline aspart] de 4,0 les différents réactifs sont additionnés en quantités précisées ci-dessous et dans l'ordre suivant :

| | |
|---|---|
| - Composé lyophilisé (citrate substitué A1) | 2920 mg |
| - Insuline aspart à 200 UI/mL | 100 mL |

Le pH final est ajusté à 7,4 ± 0,4. La solution limpide est filtrée sur une membrane 0,22 µm et stockée à 4°C.

### B32. Préparation d'une solution d'insuline lisoro à 300 UI/mL en présence du citrate substitué A1 à 43.8 mg/ml.

Pour un volume final de 100 mL de formulation avec un ratio massique [citrate substitué A1]/[insuline lispro] de 4,0 les différents réactifs sont additionnés en quantités précisées ci-dessous et dans l'ordre suivant :

| | |
|---|---|
| Composé lyophilisé (citrate substitué A1) | 4380 mg |
| Insuline lispro à 300 UI/mL | 100 mL |

Le pH final est ajusté à 7,4 ± 0,4. La solution limpide est filtrée sur une membrane 0,22 µm et stockée à 4°C.

### B33. Préparation d'une solution d'insuline humaine à 300 UI/mL en présence du citrate substitué A1 à 43.8 mg/ml.

Pour un volume final de 100 mL de formulation avec un ratio massique [citrate substitué A1]/[insuline humaine] de 4,0 les différents réactifs sont additionnés en quantités précisées ci-dessous et dans l'ordre suivant :

| | |
|---|---|
| Composé lyophilisé (citrate substitué A1) | 4380 mg |
| Insuline humaine à 300 UI/mL | 100 mL |

Le pH final est ajusté à 7,4 ± 0,4. La solution limpide est filtrée sur une membrane 0,22 µm et stockée à 4°C.

### B34. Préparation d'une solution d'insuline lisoro à 400 UI/mL en présence du citrate substitué A1 à 58,4 mg/ml.

Pour un volume final de 100 mL de formulation avec un ratio massique [citrate substitué A1]/[insuline lispro] de 4,0 les différents réactifs sont additionnés en quantités précisées ci-dessous et dans l'ordre suivant :

| | |
|---|---|
| Composé lyophilisé (citrate **substitué** A1) | 5840 mg |
| Insuline lispro à 400 UI/mL | 100 mL |

Le pH final est ajusté à 7,4 ± 0,4. La solution limpide est filtrée sur une membrane 0,22 µm et stockée à 4°C.

### B35. Préparation d'une solution d'insuline humaine à 400 UI/mL en présence du citrate substitué A1 à 58.4 mg/ml.

Pour un volume final de 100 mL de formulation avec un ratio massique [citrate substitué A1]/[insuline humaine] de 4,0 les différents réactifs sont additionnés en quantités précisées ci-dessous et dans l'ordre suivant :

| | |
|---|---|
| Composé lyophilisé (citrate substitué A1) | 5840 mg |
| Insuline humaine à 400 UI/mL | 100 mL |

Le pH final est ajusté à 7,4 ± 0,4. La solution limpide est filtrée sur une membrane 0,22 µm et stockée à 4°C.

### 836. Préparation d'une solution d'insuline lispro à 500 UI/mL en présence du citrate substitué A1 à 73 mg/ml.

Pour un volume final de 100 mL de formulation avec un ratio massique [citrate substitué A1]/[insuline lispro] de 4,0 les différents réactifs sont additionnés en quantités précisées ci-dessous et dans l'ordre suivant :

| | |
|---|---|
| Composé lyophilisé (citrate substitué A1) | 7300 mg |
| Insuline lispro à 500 UI/mL | 100 mL |

Le pH final est ajusté à 7,4 ± 0,4. La solution limpide est filtrée sur une membrane 0,22 µm et stockée à 4°C.

### B37. Préparation d'une solution d'insuline humaine à 500 UI/mL en présence du citrate substitué A1 à 73 mg/ml.

Pour un volume final de 100 mL de formulation avec un ratio massique [citrate substitué A1]/[insuline humaine] de 4,0 les différents réactifs sont additionnés en quantités précisées ci-dessous et dans l'ordre suivant :

| | |
|---|---|
| Composé lyophilisé (citrate substitué A1) | 7300 mg |
| Insuline humaine à 500 UI/mL | 100 mL |

Le pH final est ajusté à 7,4 ± 0,4. La solution limpide est filtrée sur une membrane 0,22 µm et stockée à 4°C.

### B38. Préparation d'une solution d'insuline aspart à 500 UI/mL en présence du citrate substitué A1 à 73 ma/ml.

Pour un volume final de 100 mL de formulation avec un ratio massique [citrate substitué A1]/[insuline aspart] de 4,0 les différents réactifs sont additionnés en quantités précisées ci-dessous et dans l'ordre suivant :

| | |
|---|---|
| Composé lyophilisé (citrate substitué A1) | 7300 mg |
| Insuline aspart à 500 UI/mL | 100 mL |

Le pH final est ajusté à 7,4 ± 0,4.

La solution limpide est filtrée sur une membrane 0,22 µm et stockée à 4°C.

### B39. Préparation d'une solution d'insuline lispro à 100 UI/mL en présence du citrate substitué A2 et de citrate

Pour un volume final de 100 mL de composition, avec un ratio massique [citrate substitué A2]/[insuline lispro] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate **substitué** A2) | 730 mg |
| - Solution commerciale d'Humalog® | 100 mL |
| - Solution de citrate de sodium à 1,188 M | 783 µL |

Le pH final est ajusté à 7,4 ± 0,4.

La solution limpide est filtré sur une membrane 0,22 µm et stockée à 4°C.

### B40. Préparation d'une solution d'insuline humaine à 100 UI/mL en présence du citrate substitué A2 et de citrate

Pour un volume final de 100 mL de composition, avec un ratio massique [citrate substitué A2]/[insuline humaine] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate **substitué** A2) | 730 mg |
| - Solution commerciale d'Humulin® R | 100 mL |
| - Solution de citrate de sodium à 1,188 M | 783 µL |

Le pH final est ajusté à 7,4 ± 0,4.

La solution limpide est filtré sur une membrane 0,22 µm et stockée à 4°C.

### B41. Préparation d'une solution d'insuline aspart à 100 UI/mL en présence du citrate substitué A2 et de citrate

Pour un volume final de 100 mL de composition, avec un ratio massique [citrate substitué A2]/[insuline aspart] de 2,0 et une concentration de 9,3 mM de citrate, les différents réactifs sont additionnés dans les quantités spécifiés ci-dessous et dans l'ordre suivant :

| | |
|---|---|
| - Composé lyophilisé (citrate substitué A2) | 730 mg |
| - Solution commerciale de Novolog® | 100 mL |
| - Solution de citrate de sodium à 1,188 M | 783 µL |

Le pH final est ajusté à 7,4 ± 0,4.

La solution limpide est filtré sur une membrane 0,22 µm et stockée à 4°C.

### B42. Préparation d'une solution d'insuline lisoro à 200 UI/mL en présence du citrate substitué A2 et de citrate.

Pour un volume final de 100 mL de formulation avec un ratio massique [citrate substitué A2]/[insuline lispro] de 2,0 et une concentration de 18,6 mM de citrate, les différents réactifs sont additionnés dans les quantités spécifiées ci-dessous et dans l'ordre suivant :

| | |
|---|---|
| - Composé lyophilisé (citrate substitué A2) | 1460 mg |
| - Insuline lispro à 200 UI/mL | 100 mL |
| - Solution de citrate de sodium à 1,188 M | 1566 µL |

Le pH final est ajusté à 7,4 ± 0,4.La solution limpide est filtrée sur une membrane 0,22 µm et stockée à 4°C.

### B43. Préparation d'une solution d'insuline humaine à 200 UI/mL en présence du citrate substitué A2 et de citrate.

Pour un volume final de 100 mL de formulation avec un ratio massique [citrate substitué A2]/[insuline humaine] de 2,0 et une concentration de 18,6 mM de citrate, les différents réactifs sont additionnés en quantités précisées ci-dessous et dans l'ordre suivant :

| | |
|---|---|
| - Composé lyophilisé (citrate substitué A2) | 1460 mg |
| - Insuline humaine à 200 UI/mL | 100 mL |
| - Solution de citrate de sodium à 1.188 M | 1566 µL |

Le pH final est ajusté à 7,4 ± 0,4. La solution limpide est filtrée sur une membrane 0,22 µm et stockée à 4°C.

### B44. Préparation d'une solution d'Insuline a spart à 200 UI/mL en présence du citrate substitué A2 et de citrate.

Pour un volume final de 100 mL de formulation avec un ratio massique [citrate substitué A2]/[insuline aspart] de 2,0 et une concentration de 18,6 mM de citrate, les différents réactifs sont additionnés en quantités précisées ci-dessous et dans l'ordre suivant :

| | |
|---|---|
| - Composé lyophilisé (citrate **substitué** A2) | 1460 mg |
| - Insuline aspart à 200 UI/mL | 100 mL |
| - Solution de citrate de sodium à 1.188 M | 1566 µL |

Le pH final est ajusté à 7,4 ± 0,4. La solution limpide est filtrée sur une membrane 0,22 µm et stockée à 4°C.

### B45. Préparation d'une solution d'insuline lispro à 300 UI/mL en présence du citrate substitué A2 et de citrate.

Pour un volume final de 100 mL de formulation avec un ratio massique [citrate substitué A2]/[insuline lispro] de 2,0 et une concentration de 27,9 mM de citrate, les différents réactifs sont additionnés en quantités précisées ci-dessous et dans l'ordre suivant :

| | |
|---|---|
| Composé lyophilisé (citrate substitué A2) | 2190 mg |
| Insuline lispro à 300 UI/mL | 100 mL |
| Citrate de sodium | 720 mg |

Le pH final est ajusté à 7,4 ± 0,4. La solution limpide est filtrée sur une membrane 0,22 µm et stockée à 4°C.

### B46. Préparation d'une solution d'insuline humaine à 300 UI/mL en présence du citrate substitué A2 et de citrate.

Pour un volume final de 100 mL de formulation avec un ratio massique [citrate substitué A2]/[insuline humaine] de 2,0 et une concentration de 27,9 mM de citrate, les différents réactifs sont additionnés en quantités précisées ci-dessous et dans l'ordre suivant :

| | |
|---|---|
| Composé lyophilisé (citrate **substitué** A2) | 2190 mg |
| Insuline humaine à 300 UI/mL | 100 mL |
| Citrate de sodium | 720 mg |

Le pH final est ajusté à 7,4 ± 0,4. La solution limpide est filtrée sur une membrane 0,22 µm et stockée à 4°C.

### B47. Préparation d'une solution d'insuline lispro à 400 UI/mL en présence du citrate substitué A2 et de citrate.

Pour un volume final de 100 mL de formulation avec un ratio massique [citrate substitué A2]/[insuline lispro] de 2,0 et une concentration de 37,2 mM de citrate, les différents réactifs sont additionnés en quantités précisées ci-dessous et dans l'ordre suivant :

| | |
|---|---|
| Composé lyophilisé (citrate substitué A2) | 2920 mg |
| Insuline lispro à 400 UI/mL | 100 mL |
| Citrate de sodium | 960 mg |

Le pH final est ajusté à 7,4 ± 0,4. La solution limpide est filtrée sur une membrane 0,22 µm et stockée à 4°C.

### B48. Préparation d'une solution d'insuline lispro à 500 UI/mL en présence du citrate substitué A2 et de citrate.

Pour un volume final de 100 mL de formulation avec un ratio massique [citrate substitué A2]/[insuline lispro] de 2,0 et une concentration de 46,5 mM de citrate, les différents réactifs sont additionnés en quantités précisées ci-dessous et dans l'ordre suivant :

| | |
|---|---|
| Composé lyophilisé (citrate substitué A2) | 3650 mg |
| Insuline lispro à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B49. Préparation d'une solution d'insuline humaine à 500 UI/mL en présence du citrate substitué A2 et de citrate.

Pour un volume final de 100 mL de formulation avec un ratio massique [citrate substitué A2]/[insuline humaine] de 2,0 et une concentration de 46,5 mM de citrate, les différents réactifs sont additionnés en quantités précisées ci-dessous et dans l'ordre suivant :

| | |
|---|---|
| Composé lyophilisé (citrate substitué A2) | 3650 mg |
| Insuline humaine à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B50. Préparation d'une solution d'insuline aspart à 500 UI/mL en présence du citrate substitué A2 et de citrate.

Pour un volume final de 100 mL de formulation avec un ratio massique [citrate substitué A2]/[insuline aspart] de 2,0 et une concentration de 46,5 mM de citrate, les différents réactifs sont additionnés en quantités précisées ci-dessous et dans l'ordre suivant :

| | |
|---|---|
| Composé lyophilisé (citrate substitué A2) | 3650 mg |
| Insuline aspart à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B51. Préparation d'une solution d'insuline lispro à 100 UI/mL en présence du citrate substitué A3 et de citrate

Pour un volume final de 100 mL de composition, avec un ratio massique [citrate substitué A3]/[insuline lispro] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A3) | 730 mg |
| - Solution commerciale d'Humalog® | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B52. Préparation d'une solution d'insuline humaine à 100 UI/mL en présence du citrate substitué A3 et de citrate

Pour un volume final de 100 mL de composition, avec un ratio massique [citrate substitué A3]/[insuline humaine] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A3) | 730 mg |
| - Solution commerciale d'Humulin® R | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B53. Préparation d'une solution d'insuline aspart à 100 UI/mL en présence du citrate substitué A3 et de citrate

Pour un volume final de 100 mL de composition, avec un ratio massique [citrate substitué A3]/[insuline aspart] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A3) | 730 mg |
| - Solution commerciale de Novolog® | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B54. Préparation d'une solution d'insuline lispro à 200 UI/mL en présence du citrate substitué A3 et de citrate.

Pour un volume final de 100 mL de formulation avec un ratio massique [citrate substitué A3]/[insuline lispro] de 2,0 et une concentration de 18,6 mM de citrate, les différents réactifs sont additionnés dans les quantités spécifiées ci-dessous et dans l'ordre suivant :

| | |
|---|---|
| - Composé lyophilisé (citrate substitué A3) | 1460 mg |
| - Insuline lispro à 200 UI/mL | 100 mL |
| - Solution de citrate de sodium à 1,188 M | 1566 µL |

Le pH final est ajusté à 7,4 ± 0,4. La solution limpide est filtrée sur une membrane 0,22 µm et stockée à 4°C.

### B55. Préparation d'une solution d'insuline humaine à 200 UI/mL en présence du citrate substitué A3 et de citrate.

Pour un volume final de 100 mL de formulation avec un ratio massique [citrate substitué A3]/[insuline humaine] de 2,0 et une concentration de 18,6 mM de citrate, les différents réactifs sont additionnés en quantités précisées ci-dessous et dans l'ordre suivant :

| | |
|---|---|
| - Composé lyophilisé (citrate substitué A3) | 1460 mg |
| - Insuline humaine à 200 UI/mL | 100 mL |
| - Solution de citrate de sodium à 1.188 M | 1566 µL |

Le pH final est ajusté à 7,4 ± 0,4. La solution limpide est filtrée sur une membrane 0,22 µm et stockée à 4°C.

### B56. Préparation d'une solution d'insuline aspart à 200 UI/mL en présence du citrate substitué A3 et de citrate.

Pour un volume final de 100 mL de formulation avec un ratio massique [citrate substitué A3]/[insuline aspart] de 2,0 et une concentration de 18,6 mM de citrate, les différents réactifs sont additionnés en quantités précisées ci-dessous et dans l'ordre suivant :

| | |
|---|---|
| - Composé lyophilisé (citrate substitué A3) | 1460 mg |
| - Insuline aspart à 200 UI/mL | 100 mL |
| - Solution de citrate de sodium à 1.188 M | 1566 µL |

Le pH final est ajusté à 7,4 ± 0,4. La solution limpide est filtrée sur une membrane 0,22 µm et stockée à 4°C.

### B57. Préparation d'une solution d'insuline lisoro à 300 UI/mL en présence du citrate substitué A3 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline lispro à 300 UI/mL | 100 mL |
| Citrate de sodium | 720 mg |

Le pH final est ajusté à 7,4 ± 0,4. La solution limpide est filtrée sur une membrane 0,22 µm et stockée à 4°C.

### B58. Préparation d'une solution d'insuline lisoro à 400 UI/mL en présence du citrate substitué A3 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline lispro à 400 UI/mL | 100 mL |
| Citrate de sodium | 960 mg |

Le pH final est ajusté à 7,4 ± 0,4. La solution limpide est filtrée sur une membrane 0,22 µm et stockée à 4°C.

### B59. Préparation d'une solution d'insuline lispro à 500 UI/mL en présence du citrate substitué A3 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline lispro à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B60. Préparation d'une solution d'insuline humaine à 500 UI/mL en présence du citrate substitué A3 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline humaine à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B61. Préparation d'une solution d'insuline aspart à 500 UI/mL en présence du citrate substitué A3 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline aspart à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B62. Préparation d'une solution d'insuline lispro à 100 UI/mL en présence du citrate substitué A3.

Pour un volume final de 100 mL de composition, avec un ratio massique [citrate substitué A3]/[insuline lispro] de 2,0, les différents réactifs sont additionnés dans les quantités spécifiées ci-dessous et dans l'ordre suivant :

| | |
|---|---|
| - Composé lyophilisé (citrate substitué A3) | 730 mg |
| - Solution commerciale d'Humalog® | 100 mL |

Le pH final est ajusté à 7,4 ± 0,4. La solution limpide est filtrée sur une membrane 0,22 µm et stockée à 4°C.

### B63. Préparation d'une solution d'insuline humaine à 100 UI/mL en présence du citrate substitué A3.

Pour un volume final de 100 mL de composition, avec un ratio massique [citrate substitué A3]/[insuline humaine] de 2,0, les différents réactifs sont additionnés dans les quantités spécifiées ci-dessous et dans l'ordre suivant :

| | |
|---|---|
| - Composé lyophilisé (citrate substitué A3) | 730 mg |
| - Solution commerciale d'Humulin® R | 100 mL |

Le pH final est ajusté à 7,4 ± 0,4. La solution limpide est filtrée sur une membrane 0,22 µm et stockée à 4°C.

### B64. Préparation d'une solution d'insuline lispro à 200 UI/mL en présence du citrate substitué A3.

Pour un volume final de 100 mL de formulation avec un ratio massique [citrate substitué A3]/[insuline lispro] de 2,0, les différents réactifs sont additionnés dans les quantités spécifiées ci-dessous et dans l'ordre suivant :

| | |
|---|---|
| - Composé lyophilisé (citrate substitué A3) | 1460 mg |
| - Insuline lispro à 200 UI/mL | 100 mL |

Le pH final est ajusté à 7,4 ± 0,4. La solution limpide est filtrée sur une membrane 0,22 µm et stockée à 4°C.

### B65. Préparation d'une solution d'insuline humaine à 200 UI/mL en présence du citrate substitué A3.

Pour un volume final de 100 mL de formulation avec un ratio massique [citrate substitué A3]/[insuline humaine] de 2,0, les différents réactifs sont additionnés en quantités précisées ci-dessous et dans l'ordre suivant :

| | |
|---|---|
| - Composé lyophilisé (citrate substitué A3) | 1460 mg |
| - Insuline humaine à 200 UI/mL | 100 mL |

Le pH final est ajusté à 7,4 ± 0,4. La solution limpide est filtrée sur une membrane 0,22 µm et stockée à 4°C.

### B66. Préparation d'une solution d'insuline lispro à 300 UI/mL en présence du citrate substitué A3.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline lispro à 300 UI/mL | 100 mL |

Le pH final est ajusté à 7,4 ± 0,4. La solution limpide est filtrée sur une membrane 0,22 µm et stockée à 4°C.

### B67. Préparation d'une solution d'insuline humaine à 300 UI/mL en présence du citrate substitué A3.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline humaine à 300 UI/mL | 100 mL |

Le pH final est ajusté à 7,4 ± 0,4. La solution limpide est filtrée sur une membrane 0,22 µm et stockée à 4°C.

### B68. Préparation d'une solution d'insuline lispro à 400 UI/mL en présence du citrate substitué A3.

Pour un volume final de 100 mL de formulation, les différents réactifs sont additionnés en quantités précisées ci-dessous et dans l'ordre suivant :

| | |
|---|---|
| - Composé lyophilisé (citrate substitué A3) | 1460 mg |
| Insuline lispro à 400 UI/mL | 100 mL |

Le pH final est ajusté à 7,4 ± 0,4. La solution limpide est filtrée sur une membrane 0,22 µm et stockée à 4°C.

### B69. Préparation d'une solution d'insuline humaine à 400 UI/mL en présence du citrate substitué A3.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline humaine à 400 UI/mL | 100 mL |

Le pH final est ajusté à 7,4 ± 0,4. La solution limpide est filtrée sur une membrane 0,22 µm et stockée à 4°C.

### B70. Préparation d'une solution d'insuline lispro à 500 UI/mL en présence du citrate substitué A3.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline lispro à 500 UI/mL | 100 mL |

Le pH final est ajusté à 7,4 ± 0,4. La solution limpide est filtrée sur une membrane 0,22 µm et stockée à 4°C.

### B71. Préparation d'une solution d'insuline humaine à 500 UI/mL en présence du citrate substitué A3.

Pour un volume final de 100 mL de formulation , les différents réactifs sont additionnés en quantités précisées ci-dessous et dans l'ordre suivant :

| | |
|---|---|
| Composé lyophilisé (citrate substitué A3) | 1460 mg |
| Insuline humaine à 500 UI/mL | 100 mL |

Le pH final est ajusté à 7,4 ± 0,4. La solution limpide est filtrée sur une membrane 0,22 µm et stockée à 4°C.

### B72. Préparation d'une solution d'insuline lispro à 100 UI/mL en présence du citrate substitué A3.

Pour un volume final de 100 mL de composition, avec un ratio massique [citrate substitué A3]/[insuline lispro] de 4,0, les différents réactifs sont additionnés dans les quantités spécifiées ci-dessous et dans l'ordre suivant :

| | |
|---|---|
| - Composé lyophilisé (citrate substitué A3) | 1460 mg |
| - Solution commerciale d'Humalog® | 100 mL |

Le pH final est ajusté à 7,4 ± 0,4.

La solution limpide est filtrée sur une membrane 0,22 µm et stockée à 4°C.

### B73. Préparation d'une solution d'insuline humaine à 100 UI/mL en présence du citrate substitué A3.

Pour un volume final de 100 mL de composition, avec un ratio massique [citrate substitué A3]/[insuline humaine] de 4,0, les différents réactifs sont additionnés dans les quantités spécifiées ci-dessous et dans l'ordre suivant :

| | |
|---|---|
| - Composé lyophilisé (citrate substitué A3) | 1460 mg |
| - Solution commerciale d'Humulin® R | 100 mL |

Le pH final est ajusté à 7,4 ± 0,4.

La solution limpide est filtrée sur une membrane 0,22 µm et stockée à 4°C.

### B74. Préparation d'une solution d'insuline lisoro à 200 UI/mL en présence du citrate substitué A3.

Pour un volume final de 100 mL de formulation, les différents réactifs sont additionnés dans les quantités spécifiées ci-dessous et dans l'ordre suivant :

| | |
|---|---|
| - Composé lyophilisé (citrate substitué A3) | 1460 mg |
| - Insuline lispro à 200 UI/mL | 100 mL |

Le pH final est ajusté à 7,4 ± 0,4. La solution limpide est filtrée sur une membrane 0,22 µm et stockée à 4°C.

### B75. Préparation d'une solution d'insuline humaine à 200 UI/mL en présence du citrate substitué A3.

Pour un volume final de 100 mL de formulation, les différents réactifs sont additionnés en quantités précisées ci-dessous et dans l'ordre suivant :

| | |
|---|---|
| - Composé lyophilisé (citrate substitué A3) | 1460 mg |
| - Insuline humaine à 200 UI/mL | 100 mL |

Le pH final est ajusté à 7,4 ± 0,4. La solution limpide est filtrée sur une membrane 0,22 µm et stockée à 4°C.

### B76. Préparation d'une solution d'Insuline lispro à 300 UI/mL en présence du citrate substitué A3.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline lispro à 300 UI/mL | 100 mL |

Le pH final est ajusté à 7,4 ± 0,4. La solution limpide est filtrée sur une membrane 0,22 µm et stockée à 4°C.

### B77. Préparation d'une solution d'insuline humaine à 300 UI/mL en présence du citrate substitué A3.

Pour un volume final de 100 mL de formulation, les différents réactifs sont additionnés en quantités précisées ci-dessous et dans l'ordre suivant :

| | |
|---|---|
| - Composé lyophilisé (citrate substitué A3) | 1460 mg |
| - Insuline humaine à 300 UI/mL | 100 mL |

Le pH final est ajusté à 7,4 ± 0,4. La solution limpide est filtrée sur une membrane 0,22 µm et stockée à 4°C.

### B78. Préparation d'une solution d'insuline lispro à 400 UI/mL en présence du citrate substitué A3.

Pour un volume final de 100 mL de formulation, les différents réactifs sont additionnés en quantités précisées ci-dessous et dans l'ordre suivant :

| | |
|---|---|
| - Composé lyophilisé (citrate substitué A3) | 1460 mg |
| - Insuline lispro à 400 UI/mL | 100 mL |

Le pH final est ajusté à 7,4 ± 0,4. La solution limpide est filtrée sur une membrane 0,22 µm et stockée à 4°C.

### B79. Préparation d'une solution d'insuline humaine à 400 UI/mL en présence du citrate substitué A3.

Pour un volume final de 100 mL de formulation, les différents réactifs sont additionnés en quantités précisées ci-dessous et dans l'ordre suivant :

| | |
|---|---|
| - Composé lyophilisé (citrate substitué A3) | 1460 mg |
| - Insuline humaine à 400 UI/mL | 100 mL |

Le pH final est ajusté à 7,4 ± 0,4. La solution limpide est filtrée sur une membrane 0,22 µm et stockée à 4°C.

### B80. Préparation d'une solution d'insuline lispro à 500 UI/mL en présence du citrate substitué A3.

Pour un volume final de 100 mL de formulation, les différents réactifs sont additionnés en quantités précisées ci-dessous et dans l'ordre suivant :

| | |
|---|---|
| - Composé lyophilisé (citrate substitué A3) | 1460 mg |
| - Insuline lispro à 500 UI/mL | 100 mL |

Le pH final est ajusté à 7,4 ± 0,4. La solution limpide est filtrée sur une membrane 0,22 µm et stockée à 4°C.

### B81. Préparation d'une solution d'insuline humaine à 500 UI/mL en présence du citrate substitué A3.

Pour un volume final de 100 mL de formulation, les différents réactifs sont additionnés en quantités précisées ci-dessous et dans l'ordre suivant :

| | |
|---|---|
| Composé lyophilisé (citrate substitué A3) | 1460 mg |
| Insuline humaine à 500 UI/mL | 100 mL |

Le pH final est ajusté à 7,4 ± 0,4. La solution limpide est filtrée sur une membrane 0,22 µm et stockée à 4°C.

### B82. Préparation d'une solution d'insuline aspart à 100 UI/mL en présence du citrate substitué A3

Pour un volume final de 100 mL de composition, avec un ratio massique [citrate substitué A3]/[insuline aspart] de 2,0, les différents réactifs sont additionnés dans les quantités spécifiées ci-dessous et dans l'ordre suivant :

| | |
|---|---|
| - Composé lyophilisé (citrate substitué A3) | 730 mg |
| - Solution commerciale de Novolog® | 100 mL |

Le pH final est ajusté à 7,4 ± 0,4.

La solution limpide est filtrée sur une membrane 0,22 µm et stockée à 4°C.

### B83. Préparation d'une solution d'insuline aspart à 100 UI/mL en présence du citrate substitué A3

Pour un volume final de 100 mL de composition, avec un ratio massique [citrate substitué A3]/[insuline aspart] de 4,0, les différents réactifs sont additionnés dans les quantités spécifiées ci-dessous et dans l'ordre suivant :

| | |
|---|---|
| - Composé lyophilisé (citrate substitué A3) | 1460 mg |
| - Solution commerciale de Novolog® | 100 mL |

Le pH final est ajusté à 7,4 ± 0,4.

La solution limpide est filtrée sur une membrane 0,22 µm et stockée à 4°C.

### B84. Préparation d'une solution d'insuline glulisine à 100 UI/mL en présence du citrate substitué A3 et de citrate

Pour un volume final de 100 mL de composition, avec un ratio massique [citrate substitué A3]/[insuline glulisine] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A3) | 730 mg |
| - Solution commerciale d'Apidra® | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B85. Préparation d'une solution d'insuline glulisine à 100 UI/mL en présence du citrate substitué A3

Pour un volume final de 100 mL de composition, avec un ratio massique [citrate substitué A3]/[insuline glulisine] de 2,0, les différents réactifs sont additionnés dans les quantités spécifiées ci-dessous et dans l'ordre suivant :

| | |
|---|---|
| - Composé lyophilisé (citrate substitué A3) | 730 mg |
| - Solution commerciale d'Apidra® | 100 mL |

Le pH final est ajusté à 7,4 ± 0,4.

La solution limpide est filtrée sur une membrane 0,22 µm et stockée à 4°C.

### B86. Préparation d'une solution d'insuline glulisine à 100 UI/mL en présence du citrate substitué A3

Pour un volume final de 100 mL de composition, avec un ratio massique [citrate substitué A3]/[insuline glulisine] de 4,0, les différents réactifs sont additionnés dans les quantités spécifiées ci-dessous et dans l'ordre suivant :

| | |
|---|---|
| - Composé lyophilisé (citrate substitué A3) | 1460 mg |
| - Solution commerciale d'Apidra® | 100 mL |

Le pH final est ajusté à 7,4 ± 0,4.

La solution limpide est filtrée sur une membrane 0,22 µm et stockée à 4°C.

### B87. Préparation d'une solution d'insuline lispro à 100 UI/mL en présence du citrate substitué A4 et de citrate

Pour un volume final de 100 mL de composition, avec un ratio massique [citrate substitué A4]/[insuline lispro] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A4) | 730 mg |
| - Solution commerciale d'Humalog® | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B88. Préparation d'une solution d'insuline humaine à 100 UI/mL en présence du citrate substitué A4 et de citrate

Pour un volume final de 100 mL de composition, avec un ratio massique [citrate substitué A4]/[insuline humaine] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A4) | 730 mg |
| - Solution commerciale d'Humulin® R | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B89. Préparation d'une solution d'insuline aspart à 100 UI/mL en présence du citrate substitué A4 et de citrate

Pour un volume final de 100 mL de composition, avec un ratio massique [citrate substitué A4]/[insuline aspart] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A4) | 730 mg |
| - Solution commerciale de Novolog® | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B90. Préparation d'une solution d'insuline lispro à 200 UI/mL en présence du citrate substitué A4 et de citrate.

Pour un volume final de 100 mL de formulation avec un ratio massique [citrate substitué A4]/[insuline lispro] de 2,0 et une concentration de 18,6 mM de citrate, les différents réactifs sont additionnés dans les quantités spécifiées ci-dessous et dans l'ordre suivant :

| | |
|---|---|
| - Composé lyophilisé (citrate substitué A4) | 1460 mg |
| - Insuline lispro à 200 UI/mL | 100 mL |
| - Solution de citrate de sodium à 1,188 M | 1566 µL |

Le pH final est ajusté à 7,4 ± 0,4. La solution limpide est filtrée sur une membrane 0,22 µm et stockée à 4°C.

### B91. Préparation d'une solution d'insuline humaine à 200 UI/mL en présence du citrate substitué A4 et de citrate.

Pour un volume final de 100 mL de formulation avec un ratio massique [citrate substitué A4]/[insuline humaine] de 2,0 et une concentration de 18,6 mM de citrate, les différents réactifs sont additionnés en quantités précisées ci-dessous et dans l'ordre suivant :

| | |
|---|---|
| - Composé lyophilisé (citrate substitué A4) | 1460 mg |
| - Insuline humaine à 200 UI/mL | 100 mL |
| - Solution de citrate de sodium à 1.188 M | 1566 µL |

Le pH final est ajusté à 7,4 ± 0,4. La solution limpide est filtrée sur une membrane 0,22 µm et stockée à 4°C.

### B92. Préparation d'une solution d'insuline aspart à 200 UI/mL en présence du citrate substitué A4 et de citrate.

Pour un volume final de 100 mL de formulation avec un ratio massique [citrate substitué A4]/[insuline aspart] de 2,0 et une concentration de 18,6 mM de citrate, les différents réactifs sont additionnés en quantités précisées ci-dessous et dans l'ordre suivant :

| | |
|---|---|
| - Composé lyophilisé (citrate substitué A4) | 1460 mg |
| - Insuline aspart à 200 UI/mL | 100 mL |
| - Solution de citrate de sodium à 1.188 M | 1566 µL |

Le pH final est ajusté à 7,4 ± 0,4. La solution limpide est filtrée sur une membrane 0,22 µm et stockée à 4°C.

### B93. Préparation d'une solution d'insuline lispro à 300 UI/mL en présence du citrate substitué A4 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline lispro à 300 UI/mL | 100 mL |
| Citrate de sodium | 720 mg |

Le pH final est ajusté à 7,4 ± 0,4. La solution limpide est filtrée sur une membrane 0,22 µm et stockée à 4°C.

### B94. Préparation d'une solution d'insuline lispro à 400 UI/mL en présence du citrate substitué A4 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| - Insuline lispro à 400 UI/mL | 100 mL |
| - Citrate de sodium | 960 mg |

Le pH final est ajusté à 7,4 ± 0,4. La solution limpide est filtrée sur une membrane 0,22 µm et stockée à 4°C.

### B95. Préparation d'une solution d'insuline lispro à 500 UI/mL en présence du citrate substitué A4 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline lispro à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B96. Préparation d'une solution d'insuline humaine à 500 UI/mL en présence du citrate substitué A4 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline humaine à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B97. Préparation d'une solution d'insuline aspart à 500 UI/mL en présence du citrate substitué A4 et de citrate.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline aspart à 500 UI/mL | 100 mL |
| Citrate de sodium | 1200 mg |

Le pH final est ajusté à 7,4 ± 0,4. La solution limpide est filtrée sur une membrane 0,22 µm et stockée à 4°C.

### B98. Préparation d'une solution d'insuline lispro à 100 UI/mL en présence du citrate substitué A4.

Pour un volume final de 100 mL de composition, avec un ratio massique [citrate substitué A4]/[insuline lispro] de 2,0, les différents réactifs sont additionnés dans les quantités spécifiées ci-dessous et dans l'ordre suivant :

| | |
|---|---|
| - Composé lyophilisé (citrate substitué A4) | 730 mg |
| - Solution commerciale d'Humalog® | 100 mL |

Le pH final est ajusté à 7,4 ± 0,4. La solution limpide est filtrée sur une membrane 0,22 µm et stockée à 4°C.

### B99. Préparation d'une solution d'insuline humaine à 100 UI/mL en présence du citrate substitué A4.

Pour un volume final de 100 mL de composition, avec un ratio massique [citrate substitué A4]/[insuline humaine] de 2,0, les différents réactifs sont additionnés dans les quantités spécifiées ci-dessous et dans l'ordre suivant :

| | |
|---|---|
| - Composé lyophilisé (citrate substitué A4) | 730 mg |
| - Solution commerciale d'Humulin® R | 100 mL |

Le pH final est ajusté à 7,4 ± 0,4. La solution limpide est filtrée sur une membrane 0,22 µm et stockée à 4°C.

### B100. Préparation d'une solution d'insuline lispro à 200 UI/mL en présence du citrate substitué A4.

Pour un volume final de 100 mL de formulation avec un ratio massique [citrate substitué A4]/[insuline lispro] de 2,0, les différents réactifs sont additionnés dans les quantités spécifiées ci-dessous et dans l'ordre suivant :

| | |
|---|---|
| - Composé lyophilisé (citrate substitué A4) | 1460 mg |
| - Insuline lispro à 200 UI/mL | 100 mL |

Le pH final est ajusté à 7,4 ± 0,4. La solution limpide est filtrée sur une membrane 0,22 µm et stockée à 4°C.

### B101. Préparation d'une solution d'insuline humaine à 200 UI/mL en présence du citrate substitué A4.

Pour un volume final de 100 mL de formulation avec un ratio massique [citrate substitué A4]/[insuline humaine] de 2,0, les différents réactifs sont additionnés en quantités précisées ci-dessous et dans l'ordre suivant :

| | |
|---|---|
| - Composé lyophilisé (citrate substitué A4) | 1460 mg |
| - Insuline humaine à 200 UI/mL | 100 mL |

Le pH final est ajusté à 7,4 ± 0,4. La solution limpide est filtrée sur une membrane 0,22 µm et stockée à 4°C.

### B102. Préparation d'une solution d'insuline lispro à 300 UI/mL en présence du citrate substitué A4.

Pour un volume final de 100 mL de formulation, les différents réactifs sont additionnés en quantités précisées ci-dessous et dans l'ordre suivant :

| | |
|---|---|
| - Composé lyophilisé (citrate substitué A4) | 1460 mg |
| - Insuline lispro à 300 UI/mL | 100 mL |

Le pH final est ajusté à 7,4 ± 0,4. La solution limpide est filtrée sur une membrane 0,22 µm et stockée à 4°C.

### B103. Préparation d'une solution d'insuline humaine à 300 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline humaine à 300 UI/mL | 100 mL |

Le pH final est ajusté à 7,4 ± 0,4. La solution limpide est filtrée sur une membrane 0,22 µm et stockée à 4°C.

### B104. Préparation d'une solution d'insuline lispro à 400 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline lispro à 400 UI/mL | 100 mL |

Le pH final est ajusté à 7,4 ± 0,4. La solution limpide est filtrée sur une membrane 0,22 µm et stockée à 4°C.

### B1O5. Préparation d'une solution d'insuline humaine à 400 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline humaine à 400 UI/mL | 100 mL |

Le pH final est ajusté à 7,4 ± 0,4. La solution limpide est filtrée sur une membrane 0,22 µm et stockée à 4°C.

### B106. Préparation d'une solution d'insuline lispro à 500 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460mg |
| Insuline lispro à 500 UI/mL | 100 mL |

Le pH final est ajusté à 7,4 ± 0,4. La solution limpide est filtrée sur une membrane 0,22 µm et stockée à 4°C.

### B107. Préparation d'une solution d'insuline humaine à 500 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline humaine à 500 UI/mL | 100 mL |

Le pH final est ajusté à 7,4 ± 0,4. La solution limpide est filtrée sur une membrane 0,22 µm et stockée à 4°C.

### B108. Préparation d'une solution d'insuline lispro à 100 UI/mL en présence du citrate substitué A4.

Pour un volume final de 100 mL de composition, avec un ratio massique [citrate substitué A4]/[insuline lispro] de 4,0, les différents réactifs sont additionnés dans les quantités spécifiées ci-dessous et dans l'ordre suivant :

| | |
|---|---|
| - Composé lyophilisé (citrate substitué A4) | 1460 mg |
| - Solution commerciale d'Humalog® | 100 mL |

Le pH final est ajusté à 7,4 ± 0,4.

La solution limpide est filtrée sur une membrane 0,22 µm et stockée à 4°C.

### B109. Préparation d'une solution d'insuline humaine à 100 UI/mL en présence du citrate substitué A4.

Pour un volume final de 100 mL de composition, avec un ratio massique [citrate substitué A4]/[insuline humaine] de 4,0, les différents réactifs sont additionnés dans les quantités spécifiées ci-dessous et dans l'ordre suivant :

| | |
|---|---|
| - Composé lyophilisé (citrate substitué A4) | 1460 mg |
| - Solution commerciale d'Humulin® R | 100 mL |

Le pH final est ajusté à 7,4 ± 0,4.

La solution limpide est filtrée sur une membrane 0,22 µm et stockée à 4°C.

### B110. Préparation d'une solution d'insuline lispro à 200 UI/mL en présence du citrate substitué A4.

Pour un volume final de 100 mL de formulation, les différents réactifs sont additionnés dans les quantités spécifiées ci-dessous et dans l'ordre suivant :

| | |
|---|---|
| - Composé lyophilisé (citrate substitué A4) | 1460 mg |
| - Insuline lispro à 200 UI/mL | 100 mL |

Le pH final est ajusté à 7,4 ± 0,4. La solution limpide est filtrée sur une membrane 0,22 µm et stockée à 4°C.

### B111. Préparation d'une solution d'insuline humaine à 200 UI/mL en présence du citrate substitué A4.

Pour un volume final de 100 mL de formulation, les différents réactifs sont additionnés en quantités précisées ci-dessous et dans l'ordre suivant :

| | |
|---|---|
| - Composé lyophilisé (citrate substitué A4) | 1460 mg |
| - Insuline humaine à 200 UI/mL | 100 mL |

Le pH final est ajusté à 7,4 ± 0,4. La solution limpide est filtrée sur une membrane 0,22 µm et stockée à 4°C.

### B112. Préparation d'une solution d'insuline lispro à 300 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline lispro à 300 UI/mL | 100 mL |

Le pH final est ajusté à 7,4 ± 0,4. La solution limpide est filtrée sur une membrane 0,22 µm et stockée à 4°C.

### B113. Préparation d'une solution d'insuline humaine à 300 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline humaine à 300 UI/mL | 100 mL |

Le pH final est ajusté à 7,4 ± 0,4. La solution limpide est filtrée sur une membrane 0,22 µm et stockée à 4°C.

### B114. Préparation d'une solution d'insuline lispro à 400 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460mg |
| Insuline lispro à 400 UI/mL | 100 mL |

Le pH final est ajusté à 7,4 ± 0,4. La solution limpide est filtrée sur une membrane 0,22 µm et stockée à 4°C.

### B115. Préparation d'une solution d'insuline humaine à 400 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline humaine à 400 UI/mL | 100 mL |

Le pH final est ajusté à 7,4 ± 0,4. La solution limpide est filtrée sur une membrane 0,22 µm et stockée à 4°C.

### B116. Préparation d'une solution d'insuline lispro à 500 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline lispro à 500 UI/mL | 100 mL |

Le pH final est ajusté à 7,4 ± 0,4. La solution limpide est filtrée sur une membrane 0,22 µm et stockée à 4°C.

### B117. Préparation d'une solution d'insuline humaine à 500 UI/mL en présence du citrate substitué A4.

Pour un volume final de 100 mL de formulation, les différents réactifs sont additionnés en quantités précisées ci-dessous et dans l'ordre suivant :

| | |
|---|---|
| Composé lyophilisé (citrate substitué A4) | 1460 mg |
| Insuline humaine à 500 UI/mL | 100 mL |

Le pH final est ajusté à 7,4 ± 0,4. La solution limpide est filtrée sur une membrane 0,22 µm et stockée à 4°C.

### B118. Préparation d'une solution d'insuline aspart à 100 UI/mL en présence du citrate substitué A4

Pour un volume final de 100 mL de composition, avec un ratio massique [citrate substitué A4]/[insuline aspart] de 2,0, les différents réactifs sont additionnés dans les quantités spécifiées ci-dessous et dans l'ordre suivant :

| | |
|---|---|
| - Composé lyophilisé (citrate substitué A4) | 730 mg |
| - Solution commerciale de Novolog® | 100 mL |

Le pH final est ajusté à 7,4 ± 0,4.

La solution limpide est filtrée sur une membrane 0,22 µm et stockée à 4°C.

### B119. Préparation d'une solution d'insuline aspart à 100 UI/mL en présence du citrate substitué A4

Pour un volume final de 100 mL de composition, avec un ratio massique [citrate substitué A4]/[insuline aspart] de 4,0, les différents réactifs sont additionnés dans les quantités spécifiées ci-dessous et dans l'ordre suivant :

| | |
|---|---|
| - Composé lyophilisé (citrate substitué A4) | 1460 mg |
| - Solution commerciale de Novolog® | 100 mL |

Le pH final est ajusté à 7,4 ± 0,4.

La solution limpide est filtrée sur une membrane 0,22 µm et stockée à 4°C.

### B120. Préparation d'une solution d'insuline glulisine à 100 UI/mL en présence du citrate substitué A4 et de citrate

Pour un volume final de 100 mL de composition, avec un ratio massique [citrate substitué A4]/[insuline glulisine] de 2,0 et une concentration de 9,3 mM de citrate, les différents réactifs sont additionnés dans les quantités spécifiées ci-dessous et dans l'ordre suivant :

| | |
|---|---|
| - Composé lyophilisé (citrate substitué A4) | 730 mg |
| - Solution commerciale d'Apidra® | 100 mL |
| - Solution de citrate de sodium à 1,188 M | 783 µL |

Le pH final est ajusté à 7,4 ± 0,4.

La solution limpide est filtrée sur une membrane 0,22 µm et stockée à 4°C.

### B121, Préparation d'une solution d'insuline glulisine à 100 UI/mL en présence du citrate substitué A4

Pour un volume final de 100 mL de composition, avec un ratio massique [citrate substitué A4]/[insuline glulisine] de 2,0, les différents réactifs sont additionnés dans les quantités spécifiées ci-dessous et dans l'ordre suivant :

| | |
|---|---|
| - Composé lyophilisé (citrate substitué A4) | 730 mg |
| - Solution commerciale d'Apidra® | **100** mL |

Le pH final est ajusté à 7,4 ± 0,4.

La solution limpide est filtrée sur une membrane 0,22 µm et stockée à 4°C.

### B122. Préparation d'une solution d'insuline glulisine à 100 UI/mL en présence du citrate substitué A4

Pour un volume final de 100 mL de composition, avec un ratio massique [citrate substitué A4]/[insuline glulisine] de 4,0, les différents réactifs sont additionnés dans les quantités spécifiées ci-dessous et dans l'ordre suivant :

| | |
|---|---|
| - Composé lyophilisé (citrate substitué A4) | 1460 mg |
| - Solution commerciale d'Apidra® | 100 mL |

Le pH final est ajusté à 7,4 ± 0,4.

La solution limpide est filtrée sur une membrane 0,22 µm et stockée à 4°C.

### C Pharmacodynamie et pharmacocinétique

### C1 : Protocole de mesure de la Pharmacodynamie et de la pharmacocinétique des solutions d'insuline

Des porcs domestiques d'environ 50 kg, préalablement cathétérisés au niveau de la jugulaire, sont mis à jeun 2,5 heures avant le début de l'expérience. Dans l'heure précédant l'injection d'insuline, 3 prélèvements sanguins sont réalisés afin de déterminer le niveau basal de glucose et d'insuline.

L'injection d'insuline à la dose de 0,125 UI/kg pour l'insuline lispro est réalisée en sous-cutané au niveau du flanc ou du cou de l'animal à l'aide d'un stylo à insuline (Novo, Sanofi ou Lilly) équipé d'une aiguille 31 G.

Des prélèvements sanguins sont ensuite réalisés toutes les 4 minutes pendant 20 minutes puis toutes les 10 minutes jusqu'à 3 heures. Après chaque prélèvement, le cathéter est rincé avec une solution diluée d'héparine.

Une goutte de sang est prélevée pour déterminer la glycémie au moyen d'un glucomètre.

Les courbes de pharmacodynamie du glucose exprimées en pourcent du niveau basal sont ensuite tracées. Le temps nécessaire pour atteindre le taux minimum de glucose dans le sang et 50% du taux minimum de glucose dans le sang pour chaque porc sont déterminés et reportés comme Tmin glucose et T50%Rmin glucose, respectivement. Les moyennes des Tmin glucose et des T50%Rmin glucose sont ensuite calculées.

Le sang restant est collecté dans un tube sec et est centrifugé pour isoler le sérum. Les taux d'insuline dans les échantillons de sérum sont mesurés par la méthode immuno-enzymatique ELISA en sandwich pour chaque porc.

Les courbes de pharmacocinétique exprimées en delta du niveau basal sont ensuite tracées. Le temps nécessaire pour atteindre la concentration maximale et le temps nécessaire pour atteindre 50% de la concentration maximale d'insuline dans le sérum pour chaque porc sont déterminés et reportés comme Tmax insuline et T50%Cmax insuline, respectivement. Les moyennes des Tmax insuline et des T50%Cmax insuline sont ensuite calculées.

### C2 : Résultats de Pharmacodynamie et de Pharmacocinétique des solutions d'insulines des exemples B2 et B11 (injection dans le flanc)

| **Exemple** | **Insuline** | **Citrate substitué** | **Excipient** | **Nombre de porcs** |
|---|---|---|---|---|
| B2 | Lispro | - | - | 9 |
| B11 | Lispro | A1 | Citrate 9,3 mM | 10 |

Les résultats de pharmacodynamie obtenus avec les compositions décrites dans les exemples B2 et B11 sont présentés sur la figure 1. L'analyse de ces courbes montre que la composition de l'exemple B11 comprenant le citrate substitué A1 et le citrate comme excipient (courbe tracée avec les carrés correspondant à l'exemple B11, Tmin glucose = 44 ± 18 min et T50%Rmin glucose = 15 ± 3 min) permet d'obtenir une action plus rapide que celle de la composition commerciale Humalog® de l'exemple B2 (courbe tracée avec les triangles correspondant à l'exemple B2, Tmin glucose = 61 ± 23 min et T50%Rmin glucose = 26 ± 9 min).

Les résultats de pharmacocinétique obtenus avec les compositions décrites dans les exemples B2 et B11 sont présentés sur la figure 2. L'analyse de ces courbes montre que la composition de l'exemple B11 comprenant le citrate substitué A1 et le citrate comme excipient (courbe tracée avec les carrés correspondant à l'exemple B11, Tmax insuline = 20 ± 23 min et T50%Cmax insuline = 6 ± 5 min) induit une absorption plus rapide de l'insuline lispro que la composition commerciale Humalog® de l'exemple B2 (courbe tracée avec les triangles correspondant à l'exemple B2, Tmax insuline = 39 ± 18 min et T50%Cmax insuline = 24 ± 14 min).

### C3 : Résultats de Pharmacodynamie et de Pharmacocinétique des solutions d'insulines des exemples B2 et B26 (injection dans le flanc)

| **Exemple** | **Insuline** | **Citrate substitué** | **Excipient** | **Nombre de porcs** |
|---|---|---|---|---|
| B2 | lispro | - | - | 9 |
| B26 | lispro | A1 à 14,6 mq/ml | - | 12 |

Les résultats de pharmacodynamie obtenus avec les compositions décrites dans les exemples B2 et B26 sont présentés sur la figure 3. L'analyse de ces courbes montre que la composition de l'exemple B26 comprenant le citrate substitué A1 à 14,6 mg/ml (courbe tracée avec les carrés correspondant à l'exemple B26, Tmin glucose = 37 ± 18 min et T50%Rmin glucose = 14 ± 3 min) permet d'obtenir une action plus rapide que celle de la composition commerciale Humalog® de l'exemple B2 (courbe tracée avec les triangles correspondant à l'exemple B2, Tmin glucose = 61 ± 23 min et T50%Rmin glucose = 26 ± 9 min).

Les résultats de pharmacocinétique obtenus avec les compositions décrites dans les exemples B2 et B26 sont présentés sur la figure 4. L'analyse de ces courbes montre que la composition de l'exemple B26 comprenant le citrate substitué A1 à 14,6 mg/ml (courbe tracée avec les carrés correspondant à l'exemple B26, Tmax insuline = 22 ± 17 min et T50%Cmax insuline = 5 ± 3 min) induit une absorption plus rapide de l'insuline lispro que la composition commerciale Humalog® de l'exemple B2 (courbe tracée avec les triangles correspondant à l'exemple B2, Tmax insuline = 39 ± 18 min et T50%Cmax insuline = 24 ± 14 min).

### C4 : Résultats de Pharmacodynamie et de Pharmacocinétique des solutions d'insulines des exemples B2 et B39 (injection dans le flanc)

| **Exemple** | **Insuline** | **Citrate substitué** | **Excipient** | **Nombre de porcs** |
|---|---|---|---|---|
| B2 | lispro | - | - | 14 |
| B39 | lispro | A2 | Citrate 9,3 mM | 12 |

Les résultats de pharmacodynamie obtenus avec les compositions décrites dans les exemples B2 et B39 sont présentés sur la figure 5. L'analyse de ces courbes montre que la composition de l'exemple B39 comprenant le citrate substitué A2 et le citrate comme excipient (courbe tracée avec les carrés correspondant à l'exemple B39, Tmin glucose = 37 ± 12 min et T50%Rmin glucose = 14 ± 3 min) permet d'obtenir une action plus rapide que celle de la composition commerciale Humalog® de l'exemple B2 (courbe tracée avec les triangles correspondant à l'exemple B2, Tmin glucose = 76 ± 40 min et T50%Rmin glucose = 29 ± 13 min).

Les résultats de pharmacocinétique obtenus avec les compositions décrites dans les exemples B2 et B39 sont présentés sur la figure 6. L'analyse de ces courbes montre que la composition de l'exemple B39 comprenant le citrate substitué A2 et le citrate comme excipient (courbe tracée avec les carrés correspondant à l'exemple B39, Tmax insuline = 34 ± 26 min et T50%Cmax insuline = 9 ± 6 min) induit une absorption plus rapide de l'insuline lispro que la composition commerciale Humalog® de l'exemple B2 (courbe tracée avec les triangles correspondant à l'exemple B2, Tmax insuline = 43 ± 24 min et T50%Cmax insuline = 20 ± 19 min).

### C5 : Résultats de Pharmacodynamie et de Pharmacocinétique des solutions d'insuline des exemples B2 et B51 (injection dans le flanc)

| **Exemple** | **Insuline** | **Citrate substitué** | **Excipient** | **Nombre de porcs** |
|---|---|---|---|---|
| B2 | Lispro | - | - | 14 |
| B51 | Lispro | A3 | Citrate 9,3 mM | 12 |

Les résultats de pharmacodynamie obtenus avec les compositions décrites dans les exemples B2 et B51 sont présentés sur la figure 7. L'analyse de ces courbes montre que la composition de l'exemple B51 comprenant le citrate substitué A3 et le citrate comme excipient (courbe tracée avec les carrés correspondant à l'exemple B51, Tmin glucose = 46 ± 14 min et T50%Rmin glucose = 15 ± 4 min) permet d'obtenir une action plus rapide que celle de la composition commerciale Humalog® de l'exemple B2 (courbe tracée avec les triangles correspondant à l'exemple B2, Tmin glucose = 76 ± 40 min et T50%Rmin glucose = 29 ± 13 min).

Les résultats de pharmacocinétique obtenus avec les compositions décrites dans les exemples B2 et B51 sont présentés sur la figure 8. L'analyse de ces courbes montre que la composition de l'exemple B51 comprenant le citrate substitué A3 et le citrate comme excipient (courbe tracée avec les carrés correspondant à l'exemple B51, Tmax insuline = 17 ± 9 min et T50%Cmax insuline = 6 ± 3 min) induit une absorption plus rapide de l'insuline lispro que la composition commerciale Humalog® de l'exemple B2 (courbe tracée avec les triangles correspondant à l'exemple B2, Tmax insuline = 43 ± 24 min et T50%Cmax insuline = 20 ± 19 min).

### C6 : Résultats de Pharmacodynamie et de Pharmacocinétique des solutions d'insuline des exemples B2 et B62 (injection dans le cou)

| **Exemple** | **Insuline** | **Citrate substitué** | **Excipient** | **Nombre de porcs** |
|---|---|---|---|---|
| B2 | Lispro | - | - | 11 |
| B62 | Lispro | A3 | - | 11 |

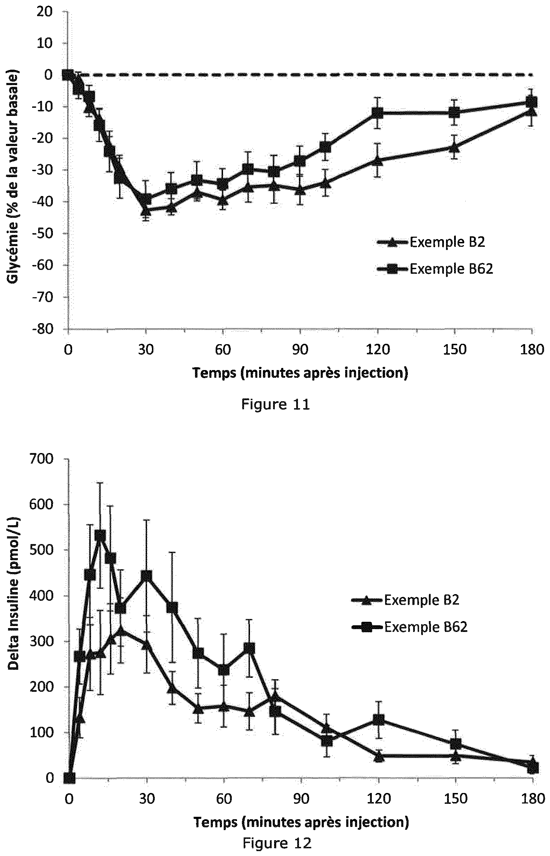

Les résultats de pharmacodynamie obtenus avec les compositions décrites dans les exemples B2 et B62 sont présentés sur la figure 11. L'analyse de ces courbes montre que la composition de l'exemple B62 comprenant le citrate substitué A3 (courbe tracée avec les carrés correspondant à l'exemple B62, Tmin glucose = 46 ± 24 min et T50%Rmin glucose = 16 ± 6 min) permet d'obtenir une action plus rapide que celle de la composition commerciale Humalog® de l'exemple B2 (courbe tracée avec les triangles correspondant à l'exemple B2, Tmin glucose = 63 ± 35 min et T50%Rmin glucose = 19 ± 6 min).

Les résultats de pharmacocinétique obtenus avec les compositions décrites dans les exemples B2 et B62 sont présentés sur la figure 12. L'analyse de ces courbes montre que la composition de l'exemple B62 comprenant le citrate substitué A3 (courbe tracée avec les carrés correspondant à l'exemple B62, Tmax insuline = 24 ± 20 min et T50%Cmax insuline = 7 ± 4 min) induit une absorption plus rapide de l'insuline lispro que la composition commerciale Humalog® de l'exemple B2 (courbe tracée avec les triangles correspondant à l'exemple B2, Tmax insuline = 26 ± 19 min et T50%Cmax insuline = 9 ± 7 min).

### D Dichroïsme circulaire

### D1 Etat d'association de l'insuline lispro évalué par dichroïsme circulaire en présence de différents citrates substitués et de citrate

Le dichroïsme circulaire permet d'étudier la structure secondaire et quaternaire de l'insuline. Les monomères d'insuline s'organisent en dimères et en hexamères. L'hexamère est la forme de l'insuline la plus stable physiquement et chimiquement. Il existe deux formes hexamèriques, la forme R6 et la forme T6. L'insuline lispro présente un signal fort à 240 nm caractéristique de la forme hexamèrique R6 (forme la plus stable). La perte du signal à 240 nm est reliée à une déstabilisation de l'hexamère et au passage de R6 à T6.

### Préparation d'une solution de citrate de sodium à 1,010 M

Une solution de citrate de sodium est obtenue en solubilisant 14,90 g de citrate de sodium (50,69 mmol) dans 50 mL d'eau dans une fiole jaugée. Le pH est ajusté à 7,4 par ajout de 0,21 mL d'HCl 1M.

### Préparation des solutions d'insuline lispro à 100 UI/mL en présence de citrate substitué et de citrate

Pour un volume final de 100 mL de formulation, avec une concentration en citrate substitué à 7,3 mg/mL et une concentration de 9,3 mM de citrate, les différents réactifs sont additionnés dans les quantités spécifiées ci-dessous et dans l'ordre suivant :

| | |
|---|---|
| - Citrate substitué lyophilisé | 730 mg |
| - Solution commerciale Humalog® 100 UI /mL | 100 mL |
| - Solution de citrate de sodium à 1,010 M | 921 µL |

Pour le citrate, on peut utiliser la forme acide ou la forme basique de sel de sodium, de potassium ou d'un autre sel compatible avec une formulation injectable.

Le pH final est ajusté à 7,4 ± 0,4

La solution limpide est filtrée sur une membrane 0,22 µm et stockée à 4°C.

### Préparation de la solution d'insuline lispro à 100 UI/mL en présence de citrate substitué à 14,6 Mg/ml

Pour un volume final de 100 mL de formulation, avec une concentration en citrate substitué à 7,3 mg/mL, les différents réactifs sont additionnés dans les quantités spécifiées ci-dessous et dans l'ordre suivant :

| | |
|---|---|
| - Citrate substitué lyophilisé | 1460 mg |
| - Solution commerciale Humalog® 100 UI /mL | 100 mL |

Le pH final est ajusté à 7,4 ± 0,4. La solution limpide est filtrée sur une membrane 0,22µm et stockée à 4°C.

### Préparation de la solution d'insuline lispro à 100 UI/mL en présence d'EDTA

Pour un volume final de 100 mL de formulation, avec une concentration de 300 µM d'EDTA, les différents réactifs sont additionnés dans les quantités spécifiées ci-dessous et dans l'ordre suivant :

| | |
|---|---|
| - Solution commerciale Humalog® 100 UI /mL | 100 mL |
| - Solution commerciale d'EDTA à 0,5 M | 60 µL |

Le pH final est ajusté à 7,4 ± 0,4. La solution limpide est filtrée sur une membrane 0,22 µm et stockée à 4°C.

Deux séries de mesures ont été réalisées. Les résultats obtenus sont présentés en figure 9 et en figure 10.

La figure 9 décrit en ordonnée le signal CD à 240 nm (deg.cm².dmol⁻¹) et en abscisse :
- A : insuline lispro 100 UI/ml
- B : insuline lispro 100 UI/ml + 7,3 mg de citrate substitué A1
- B' : insuline lispro 100 UI/ml + 7,3 mg de citrate substitué A1 et citrate à 9,3 mM
- C : insuline lispro 100 UI/ml + 7,3 mg de citrate substitué A2
- C' : insuline lispro 100 UI/ml + 7,3 mg de citrate substitué A2 et citrate à 9,3 mM
- D : insuline lispro 100 UI/ml + 7,3 mg de citrate substitué A3
- D' : insuline lispro 100 UI/ml + 7,3 mg de citrate substitué A3 et citrate à 9,3 mM
- E : insuline lispro + EDTA à 300 µM

La figure 10 décrit en ordonnée le signal CD à 240 nm (deg.cm².dmol⁻¹) et en abscisse :
- A : insuline lispro 100 UI/ml
- F : insuline lispro 100 UI/ml + 14.6 mg de citrate substitué A1
- E : Insuline lispro + EDTA à 300 µM

L'EDTA déstructure complétement la forme R6 de l'insuline Lispro. L'EDTA a donc un effet marqué sur l'hexamère.

Au contraire, les mélanges composés anioniques substitués/citrate n'ont que très peu d'impact sur le signal CD à 240 nm. Ces composés ont donc pas ou peu d'impact sur la structure R6 de l'hexamère, et à fortiori sur la structure hexamèrique de l'insuline lispro. Dans les compositions selon l'invention l'insuline est donc considérée comme étant sous forme héxamérique.

### D2 Etat d'association de l'insuline lispro évalué par dichroïsme circulaire en présence du citrate substitué A3

Le dichroïsme circulaire permet d'étudier la structure secondaire et quaternaire de l'insuline. Les monomères d'insuline s'organisent en dimères et en hexamères. L'hexamère est la forme de l'insuline la plus stable physiquement et chimiquement. Il existe deux formes hexamèriques, la forme R6 et la forme T6. L'insuline Lispro présente un signal fort à 240 nm caractéristique de la forme hexamèrique R6 (forme la plus stable). La perte du signal à 240 nm est reliée à une déstabilisation de l'hexamère et au passage de R6 à T6.

### Préparation d'une solution de citrate de sodium à 1.010 M

Une solution de citrate de sodium est obtenue en solubilisant 14,90 g de citrate de sodium (50,69 mmol) dans 50 mL d'eau dans une fiole jaugée. Le pH est ajusté à 7,4 par ajout de 0,21 mL d'HCl 1M.

### Préparation des solutions d'insuline lispro à 100 UI/mL en présence de citrate substitué et de citrate

Pour un volume final de 100 mL de formulation, avec une concentration en citrate substitué à 7,3 mg/mL et une concentration de 9,3 mM de citrate, les différents réactifs sont additionnés dans les quantités spécifiées ci-dessous et dans l'ordre suivant :

| | |
|---|---|
| - Citrate substitué lyophilisé | 730 mg |
| - Solution commerciale Humalog® 100 UI /mL | 100 mL |
| - Solution de citrate de sodium à 1,010 M | 921 µL |

Pour le citrate, on peut utiliser la forme acide ou la forme basique de sel de sodium, de potassium ou d'un autre sel compatible avec une formulation injectable.

Le pH final est ajusté à 7,4 ± 0,4

La solution limpide est filtrée sur une membrane 0,22 µm et stockée à 4°C.

### Préparation de la solution d'insuline lispro à 100 UI/mL en présence de citrate substitué à 7,3 mg/ml ou à 14.6 mg/ml

Pour un volume final de 100 mL de formulation, avec une concentration en citrate substitué à 7,3 mg/ml ou 14,6 mg/mL, les différents réactifs sont additionnés dans les quantités spécifiées ci-dessous et dans l'ordre suivant :

| | |
|---|---|
| - Citrate substitué lyophilisé | 730 mg ou 1460 mg |
| - Solution commerciale Humalog® 100 UI /mL | 100 mL |

Le pH final est ajusté à 7,4 ± 0,4. La solution limpide est filtrée sur une membrane 0,22µm et stockée à 4°C.

### Préparation de la solution d'insuline lispro à 100 UI/mL en présence d'EDTA

Pour un volume final de 100 mL de formulation, avec une concentration de 300 µM d'EDTA, les différents réactifs sont additionnés dans les quantités spécifiées ci-dessous et dans l'ordre suivant :

| | |
|---|---|
| - Solution commerciale Humalog® 100 UI /mL | 100 mL |
| - Solution commerciale d'EDTA à 0,5 M | 60 µL |

Le pH final est ajusté à 7,4 ± 0,4. La solution limpide est filtrée sur une membrane 0,22 µm et stockée à 4°C.

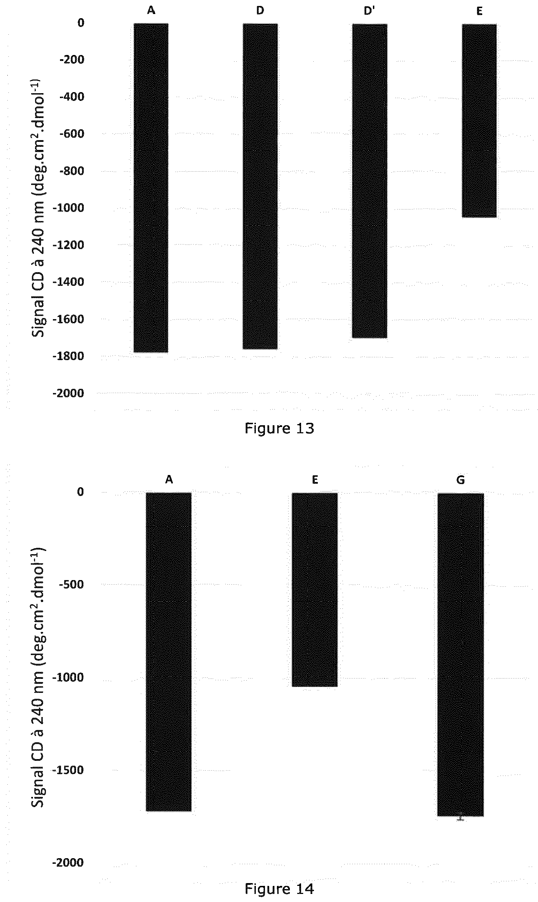

Deux séries de mesures ont été réalisées. Les résultats obtenus sont présentés en figure 13 et en figure 14.

La figure 13 décrit en ordonnée le signal CD à 240 nm (deg.cm².dmol⁻¹) et en abscisse:
- A : insuline lispro 100 UI/ml
- D : insuline lispro 100 UI/ml + 7,3 mg/ml de citrate substitué A3
- D' : insuline lispro 100 UI/ml + 7,3 mg/ml de citrate substitué A3 et citrate à 9,3 mM
- E : insuline lispro + EDTA à 300 µM

La figure 14 décrit en ordonnée le signal CD à 240 nm (deg.cm².dmol⁻¹) et en abscisse:
- A : insuline lispro 100 UI/ml
- E : Insuline lispro + EDTA à 300 µM
- G : insuline lispro 100 UI/ml + 14,6 mg/ml de citrate substitué A3

L'EDTA déstructure complétement la forme R6 de l'insuline lispro. L'EDTA a donc un effet marqué sur l'hexamère.

Au contraire, le citrate substitué A3 seul et le citrate substitué A3 en présence de citrate, n'ont que très peu d'impact sur le signal CD à 240 nm. Ces composés ont donc pas ou peu d'impact sur la structure R6 de l'hexamère, et à fortiori sur la structure hexamèrique de l'insuline lispro. Dans les compositions selon l'invention l'insuline est donc considérée comme étant sous forme hexamérique.

### D3 Etat d'association de l'insuline humaine évalué par dichroïsme circulaire en présence de citrate substitué A3

Le dichroïsme circulaire permet d'étudier la structure secondaire et quaternaire de l'insuline. Les monomères d'insuline s'organisent en dimères et en hexamères. L'hexamère est la forme de l'insuline la plus stable physiquement et chimiquement. Il existe deux formes hexamériques, la forme R6 et la forme T6. L'insuline humaine présente un signal fort à 240 nm caractéristique de la forme hexamérique R6 (forme la plus stable). La perte du signal à 240 nm est reliée à une déstabilisation de l'hexamère et au passage de R6 à T6.

### Préparation des solutions d'insuline humaine à 100 UI/mL en présence de citrate substitué et de citrate

Pour un volume final de 100 mL de formulation, avec une concentration en citrate substitué à 7,3 mg/mL et une concentration de 9,3 mM de citrate, les différents réactifs sont additionnés dans les quantités spécifiées ci-dessous et dans l'ordre suivant :

| | |
|---|---|
| - Citrate substitué lyophilisé | 730 mg |
| - Solution commerciale Humulin® R 100 UI /mL | 100 mL |
| - Solution de citrate de sodium à 1,010 M | 921 µL |

Pour le citrate, on peut utiliser la forme acide ou la forme basique de sel de sodium, de potassium ou d'un autre sel compatible avec une formulation injectable.

Le pH final est ajusté à 7,4 ± 0,4

La solution limpide est filtrée sur une membrane 0,22 µm et stockée à 4°C.

### Préparation de la solution d'insuline humaine à 100 UI/mL en présence de citrate substitué à 7.3 mg/ml ou 14.6 mg/ml

Pour un volume final de 100 mL de formulation, avec une concentration en citrate substitué à 7,3 mg/ml ou à 14,6 mg/mL, les différents réactifs sont additionnés dans les quantités spécifiées ci-dessous et dans l'ordre suivant :

| | |
|---|---|
| - Citrate substitué lyophilisé | 730 mg ou 1460 mg |
| - Solution commerciale Humulin® R 100 UI /mL | 100 mL |

Le pH final est ajusté à 7,4 ± 0,4. La solution limpide est filtrée sur une membrane 0,22µm et stockée à 4°C.

### Préparation de la solution d'insuline humaine à 100 UI/mL en présence d'EDTA

Pour un volume final de 100 mL de formulation, avec une concentration de 300 µM ou 6 mM d'EDTA, les différents réactifs sont additionnés dans les quantités spécifiées ci-dessous et dans l'ordre suivant :

| | |
|---|---|
| - Solution commerciale Humulin® R 100 UI /mL | 100 mL |
| - Sodium EDTA (poudre) | 11 mg (300 µM) ou221 mg (6 mM) |

Le pH final est ajusté à 7,4 ± 0,4. La solution limpide est filtrée sur une membrane 0,22 µm et stockée à 4°C.

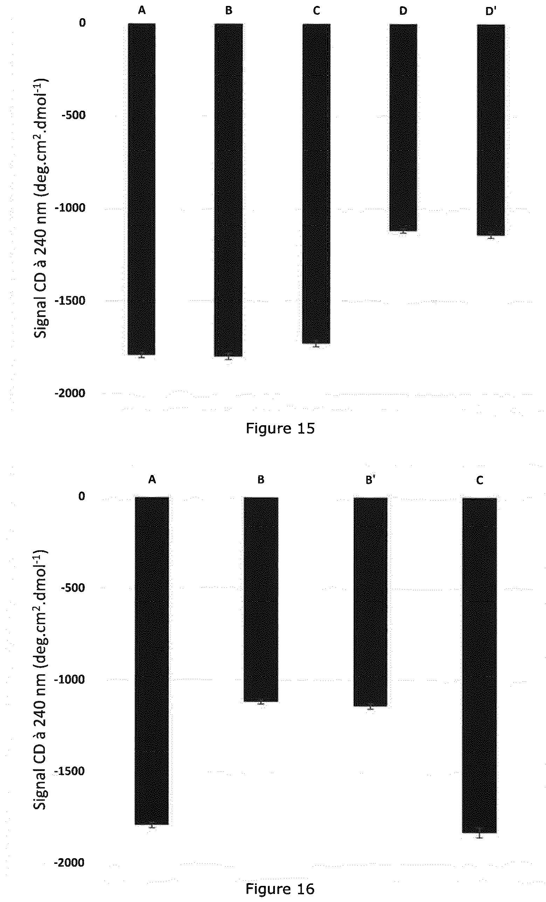

Deux séries de mesures ont été réalisées. Les résultats obtenus sont présentés en figure 15 et en figure 16.

La figure 15 décrit en ordonnée le signal CD à 240 nm (deg.cm².dmol⁻¹) et en abscisse :
- A : insuline humaine 100 UI/ml
- B : insuline humaine 100 UI/ml + 7,3 mg/ml de citrate substitué A3
- C : insuline humaine 100 UI/ml + 7,3 mg/ml de citrate substitué A3 et citrate à 9,3 mM
- D : insuline humaine 100 UI/ml + EDTA à 300 µM
- D' : insuline humaine 100 UI/ml + EDTA à 6 mM

La figure 16 décrit en ordonnée le signal CD à 240 nm (deg.cm².dmol⁻¹) et en abscisse :
- A : insuline humaine 100 UI/ml (Humulin® R)
- B : insuline humaine 100 UI/ml + EDTA à 300 µM
- B': insuline humaine 100 UI/ml + EDTA à 6 mM
- C : insuline humaine 100 UI/ml + 14,6 mg/ml de citrate substitué A3.

L'EDTA déstructure complétement la forme R6 de l'insuline humaine. L'EDTA a donc un effet marqué sur l'hexamère.

Au contraire, le citrate substitué A3 seul et le citrate substitué A3 avec du citrate, n'ont que très peu d'impact sur le signal CD à 240 nm. Ces composés n'ont donc pas, ou ont peu d'impact sur la structure R6 de l'hexamère, et à fortiori sur la structure hexamérique de l'insuline humaine. Dans les compositions selon l'invention l'insuline est donc considérée comme étant sous forme hexamérique.

### E Solubilité des composés

### El Solubilité des citrates substitués (A1, A2, A3 et A4)

La solubilité des citrates substitués (A1, A2, A3 et A4) a été mesurée dans une solution tampon.

Dans une solution tampon à 50 mM de Tris/HCl pH 7,4 (1 ml), est introduit le citrate substitué sous forme de poudre (20 mg). Les tubes ont été agités (rolling shaker) pendant 30 minutes à température ambiante puis inspectés visuellement. Le pH est ajusté à 7,4 ± 0,4.

Aucune trace d'insoluble n'est observée. Les citrates substitués A1, A2, A3 et A4 sont donc solubles à la concentration de 20 mg/ml dans une solution tampon à 50 mM Tris/HCl à pH 7,4.

La limite de solubilité de chacun des citrates substitués A1, A2, A3 et A4 est donc supérieure à 20 mg/ml dans une solution tampon à 50 mM Tris/HCl à pH 7,4.

La solubilité des citrates substitués (A1, A2, A3 et A4) a été mesurée en présence d'insuline lispro 100 U/ml. Dans une solution commerciale d'insuline lispro (Humalog®) (1 mL), est introduit le citrate substitué sous forme de poudre (20 mg). Le tube a été agité (rolling shaker) pendant 30 minutes à température ambiante puis inspecté visuellement. Le pH est ajusté à 7,4 ± 0,4.

Aucune trace d'insoluble n'est observée. Les citrates substitués A1, A2, A3 et A4 sont donc solubles à la concentration de 20 mg/ml en présence d'insuline lispro 100 UI/ml.

La limite de solubilité de chacun des citrates substitués A1, A2, A3 et A4 est donc supérieure à 20 mg/ml en présence d'insuline lispro 100 UI/ml.

### E2 Solubilité des composés de l'art antérieur (CE1 et CE2)

La solubilité des composés de l'art antérieur (CEI et CE2) a été mesurée.

4 mL d'une solution tampon à 50 mM de Tris/HCl à pH 7,4 ont été introduits dans un tube en verre, à température ambiante. Les composés sous forme de poudre ont été ajoutés successivement à raison de 8 mg par ajout. Après chaque ajout de composé, les tubes ont été agités (rolling shaker) pendant 30 minutes à température ambiante puis inspectés visuellement. Le pH est ajusté à 7,4 ± 0,4.

Les concentrations pour lesquelles des insolubles ont été observés sont définies comme étant supérieures à la limite de solubilité du composé.

**Tableau 3 : solubilité des contre-exemples dans une solution tampon Tris/HCl**

| **Composé** | **Solubilité (mg/ml)** |
|---|---|
| CE1 | < 2 |
| CE2 | < 2 |

La solubilité des composés de l'art antérieur a également été mesurée en présence d'insuline lispro 100 U/mL. 1 mL d'une solution commerciale d'Humalog a été introduit dans un tube en verre, à température ambiante. Les composés sous forme de poudre ont été ajoutés successivement à raison de 2 mg par ajout. Après chaque ajout de composé, les tubes ont été agités (rolling shaker) pendant 30 minutes à température ambiante puis inspectés visuellement. Le pH est ajusté à 7,4 ± 0,4.

Les concentrations pour lesquelles des insolubles ont été observés sont définies comme étant supérieures à la limite de solubilité du composé en présence d'insuline lispro 100 UI/ml.

**Tableau 4 : solubilité des contre-exemples en présence d'insuline lispro 100 UI/ml**

| | |
|---|---|
| **Composé** | **Solubilité (mg/ml)** |
| CE1 | <2 |
| CE2 | <2 |

Les composés de l'art antérieur ne peuvent pas être utilisés dans des solutions aqueuses selon l'invention. On observe une limite de solubilité inférieure à 2 mg/ml pour les composés CE1 et CE2 qui est au moins 10 fois inférieure à celle des citrates substitués de l'invention.

## Revendications

1. Composition, sous forme de solution aqueuse, comprenant une insuline sous forme hexamèrique, et au moins un citrate substitué de Formule I: 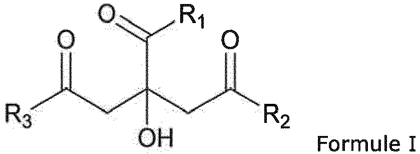 dans laquelle :
- R₁, R₂, R₃ identiques ou différents, représentent OH ou AA,
- au moins un des R₁, R₂, R₃ est un radical AA,
- AA est un radical issu d'un acide aminé aromatique naturel ou synthétique comprenant au moins un groupement phényle ou un groupement indole, substitué ou non substitué, ledit radical AA présentant au moins une fonction acide carboxylique libre,
- les fonctions acide carboxylique sont sous forme de sel d'un métal alcalin choisi parmi Na⁺ et K⁺.

2. Composition selon la revendication 1, **caractérisée en ce qu'**elle comprend en outre au moins un composé polyanionique différent dudit citrate substitué.

3. Composition selon la revendication 1 ou 2, **caractérisée en ce que** le citrate substitué est choisi parmi les composés de formule I dans laquelle le radical AA est issu d'un acide aminé aromatique naturel.

4. Composition selon l'une quelconque des revendications précédentes, **caractérisée en ce que** le citrate substitué est choisi parmi les composés de formule I dans laquelle au moins R₁ est un radical AA .

5. Composition selon l'une quelconque des revendications précédentes, **caractérisée en ce que** le citrate substitué est choisi parmi les composés de formule I dans laquelle au moins R₂ est un radical AA.

6. Composition selon l'une quelconque des revendications précédentes, **caractérisée en ce que** le citrate substitué est choisi parmi les composés de formule I dans laquelle au moins R₁ et R₂ sont un radical AA.

7. Composition selon l'une quelconque des revendications précédentes, **caractérisée en ce que** le citrate substitué est choisi parmi les composés de formule I dans laquelle au moins R₂ et R₃ sont un radical AA.

8. Composition selon l'une quelconque des revendications précédentes, **caractérisée en ce que** le citrate substitué répond à la Formule I dans laquelle R₁, R₂ et R₃ sont un radical AA.

9. Composition selon l'une quelconque des revendications précédentes, **caractérisée en ce que** le citrate substitué répond à la Formule I dans laquelle R₁ est un radical AA, R₂ et R₃ sont OH et le radical AA est issu de la phénylalanine.

10. Composition selon l'une quelconque des revendications précédentes, **caractérisée en ce que** le citrate substitué répond à la Formule I dans laquelle R₂ est un radical AA, R₁ et R₃ sont OH et le radical AA est issu de la phénylalanine.

11. Composition selon l'une quelconque des revendications précédentes, **caractérisée en ce que** le citrate substitué répond à la Formule I dans laquelle R₁ est OH, R₂ et R₃ sont un radical AA, et le radical AA est issu de la phénylalanine.

12. Composition selon l'une quelconque des revendications 1 à 6, **caractérisée en ce que** le citrate substitué répond à la Formule I dans laquelle R₁, R₂ et R₃ sont un radical AA, et le radical AA est issu de la phénylalanine.

13. Composition selon l'une quelconque des revendications précédentes, **caractérisée en ce que** les ratios molaires citrate substitué/insuline sont compris entre 3 et 400.

14. Composition selon l'une quelconque des revendications précédentes, **caractérisée en ce que** les ratios massiques citrate substitué/insuline sont compris entre 0,5 et 30.

15. Composition selon l'une quelconque des revendications précédentes, **caractérisée en ce que** la concentration en citrate substitué est comprise entre 1,8 et 100 mg/mL.

16. Composition selon l'une quelconque des revendications 1 à 15, **caractérisée en ce que** l'insuline est l'insuline humaine choisie parmi les insulines humaines recombinante.

17. Composition selon l'une quelconque des revendications 1 à 15, **caractérisée en ce que** l'insuline est une insuline analogue choisie dans le groupe constitué par l'insuline lispro (Humalog®), l'insuline aspart (Novolog®, Novorapid®) et l'insuline glulisine (Apidra®).

18. Composition phamaceutique comprenant une composition selon l'une quelconque des revendications précédentes, **caractérisée en ce que** la concentration en insuline est comprise entre 240 et 3000 µM (40 à 500 UI/mL).

19. Composition selon l'une quelconque des revendications 2 à 18, **caractérisée en ce que** le composé polyanionique est choisi dans le groupe constitué des polyacides carboxyliques et leurs sels de Na⁺, K⁺, Ca²⁺ ou Mg²⁺.

20. Composition selon l'une quelconque des revendications 2 à 19, **caractérisée en ce que** la concentration en composé polyanionique est comprise entre 2 et 150 mM.

21. Formulation pharmaceutique comprenant une composition selon l'une quelconque des revendications précédentes.

## Patentansprüche

1. Zusammensetzung in der Form einer wässrigen Lösung umfassend ein Insulin in hexamerer Form und wenigstens ein substituiertes Citrat gemäß Formel I: 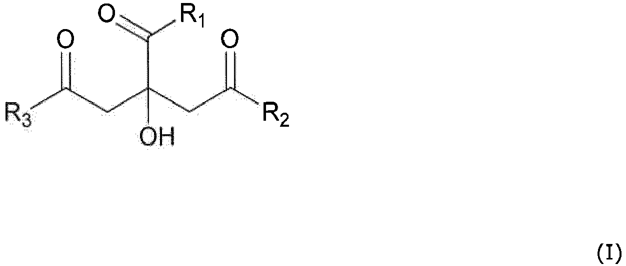 wobei
- R₁, R₂, R₃ identisch oder verschieden sind und OH oder AA darstellen,
- wenigstens einer von R₁, R₂, R₃ ein Radikal AA ist,
- AA ein Radikal ist resultierend aus einer natürlichen oder synthetischen aromatischen Aminosäure umfassend wenigstens eine Phenyl-Gruppe oder eine Indol-Gruppe, substituiert oder unsubstituiert, wobei das Radikal AA wenigstens eine freie Carbonsäurefunktion aufweist,
- Carbonsäurefunktionen in der Form eines Alkalimetallsalzes vorliegen ausgewählt aus Na⁺ und K⁺.

2. Zusammensetzung nach Anspruch 1, **dadurch gekennzeichnet, dass** sie zusätzlich wenigstens eine polyanionische Verbindung umfasst, die verschieden ist von dem substituierten Citrat.

3. Zusammensetzung nach Anspruch 1 oder 2, **dadurch gekennzeichnet, dass** das substituierte Citrat ausgewählt ist aus den Verbindungen gemäß Formel I, wobei das Radikal AA aus einer natürlichen aromatischen Aminosäure resultiert.

4. Zusammensetzung nach einem der vorangehenden Ansprüche, **dadurch gekennzeichnet, dass** das substituierte Citrat ausgewählt ist aus den Verbindungen gemäß Formel I, wobei wenigstens R₁ ein Radikal AA ist.

5. Zusammensetzung nach einem der vorangehenden Ansprüche, **dadurch gekennzeichnet, dass** das substituierte Citrat ausgewählt ist aus den Verbindungen gemäß Formel I, wobei wenigstens R₂ ein Radikal AA ist.

6. Zusammensetzung nach einem der vorangehenden Ansprüche, **dadurch gekennzeichnet, dass** das substituierte Citrat ausgewählt ist aus den Verbindungen gemäß Formel I, wobei wenigstens R₁ und R₂ ein Radikal AA sind.

7. Zusammensetzung nach einem der vorangehenden Ansprüche, **dadurch gekennzeichnet, dass** das substituierte Citrat ausgewählt ist aus den Verbindungen gemäß Formel I, wobei wenigstens R₂ und R₃ ein Radikal AA sind.

8. Zusammensetzung nach einem der vorangehenden Ansprüche, **dadurch gekennzeichnet, dass** das substituierte Citrat Formel I entspricht, wobei R₁, R₂ und R₃ ein Radikal AA sind.

9. Zusammensetzung nach einem der vorangehenden Ansprüche, **dadurch gekennzeichnet, dass** das substituierte Citrat Formel I entspricht, wobei R₁ ein Radikal AA ist, R₂ und R₃ OH sind und das Radikal AA aus Phenylalanin resultiert.

10. Zusammensetzung nach einem der vorangehenden Ansprüche, **dadurch gekennzeichnet, dass** das substituierte Citrat Formel I entspricht, wobei R₂ ein Radikal AA ist, R₁ und R₃ OH sind und das Radikal aus Phenylalanin resultiert.

11. Zusammensetzung nach einem der vorangehenden Ansprüche, **dadurch gekennzeichnet, dass** das substituierte Citrat Formel I entspricht, wobei R₁ OH ist, R₂ und R₃ ein Radikal AA sind und das Radikal AA aus Phenylalanin resultiert.

12. Zusammensetzung nach einem der Ansprüche 1 bis 6, **dadurch gekennzeichnet, dass** das substituierte Citrat Formel I entspricht, wobei R₁, R₂ und R₃ ein Radikal AA sind und das Radikal AA aus Phenylalanin resultiert.

13. Zusammensetzung nach einem der vorangehenden Ansprüche, **dadurch gekennzeichnet, dass** die Molverhältnisse substituiertes Citrat/Insulin zwischen 3 und 400 betragen.

14. Zusammensetzung nach einem der vorangehenden Ansprüche, **dadurch gekennzeichnet, dass** die Massenverhältnisse substituiertes Citrat/Insulin zwischen 0,5 und 30 betragen.

15. Zusammensetzung nach einem der vorangehenden Ansprüche, **dadurch gekennzeichnet, dass** die Konzentration an substituiertem Citrat zwischen 1,8 und 100 mg/ml beträgt.

16. Zusammensetzung nach einem der Ansprüche 1 bis 15, **dadurch gekennzeichnet, dass** das Insulin menschliches Insulin ist, das ausgewählt ist aus rekombinanten menschlichen Insulinen.

17. Zusammensetzung nach einem der Ansprüche 1 bis 15, **dadurch gekennzeichnet, dass** das Insulin ein Insulinanalog ist, das ausgewählt ist aus der Gruppe bestehen aus Insulin lispro (Humalog®), Insulin aspart (Novolog®, Novorapid®) und Insulin glulisin (Apidra®).

18. Pharmazeutische Zusammensetzung umfassend eine Zusammensetzung nach einem der vorangehenden Ansprüche, **dadurch gekennzeichnet, dass** die Konzentration an Insulin zwischen 240 und 3000 µM (40 bis 500 IU/ml) beträgt.

19. Zusammensetzung nach einem der Ansprüche 2 bis 18, **dadurch gekennzeichnet, dass** die polyanionische Verbindung ausgewählt ist aus der Gruppe bestehend aus Polycarbonsäuren und ihren Na⁺-, K⁺-, Ca²⁺- oder Mg²⁺-Salzen.

20. Zusammensetzung nach einem der Ansprüche 2 bis 19, **dadurch gekennzeichnet, dass** die Konzentration an polyanionischer Verbindung zwischen 2 und 150 mM beträgt.

21. Pharmazeutische Formulierung umfassend eine Zusammensetzung nach einem der vorangehenden Ansprüche.

## Claims

1. Composition, in the form of an aqueous solution, comprising an insulin in hexameric form, and at least one substituted citrate of Formula I: 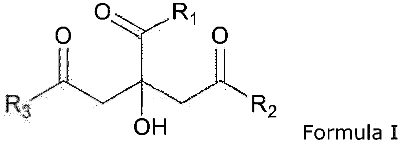 in which:
- R₁, R₂, R₃, identical or different, represent OH or AA,
- at least one of the R₁, R₂, R₃ is an AA radical,
- AA is a radical resulting from a natural or synthetic aromatic amino acid comprising at least one phenyl group or indole group, substituted or not substituted, said AA radical having at least one free carboxylic acid function,
- the carboxylic acid functions are in the form of a salt of an alkali metal selected from Na⁺ and K⁺.

2. Composition according to claim 1, **characterized in that** it comprises, in addition, at least one polyanionic compound different from said substituted citrate.

3. Composition according to claim 1 or 2, **characterized in that** the substituted citrate is selected from the compounds of formula I in which the AA radical results from a natural aromatic amino acid.

4. Composition according to any one of the preceding claims, **characterized in that** the substituted citrate is selected from the compounds of formula I in which at least R₁ is an AA radical.

5. Composition according to any one of the preceding claims, **characterized in that** the substituted citrate is selected from the compounds of formula I in which at least R₂ is an AA radical.

6. Composition according to any of the preceding claims, **characterized in that** the substituted citrate is selected from the compounds of formula I in which at least R₁ and R₂ are an AA radical.

7. Composition according to any one of the preceding claims, **characterized in that** the substituted citrate is selected from the compounds of formula I in which at least R₂ and R₃ are an AA radical.

8. Composition according to any one of the preceding claims, **characterized in that** the substituted citrate has the formula I in which R₁, R₂ and R₃ are an AA radical.

9. Composition according to any one of the preceding claims, **characterized in that** the substituted citrate has the formula I in which R₁ is an AA radical, R₂ and R₃ are OH, and the AA radical results from phenylalanine.

10. Composition according to any one of the preceding claims, **characterized in that** the substituted citrate has the formula I in which R₂ is an AA radical, R₁ and R₃ are OH, and the AA radical results from phenylalanine.

11. Composition according to any one of the preceding claims, **characterized in that** the substituted citrate has the formula I in which R₁ is OH, R₂ and R₃ are an AA radical, and the AA radical results from phenylalanine.

12. Composition according to any one of Claims 1 to 6, **characterized in that** the substituted citrate has the formula I in which R₁, R₂ and R₃ are an AA radical, and the AA radical results from phenylalanine.

13. Composition according to any one of the preceding claims, **characterized in that** the substituted citrate/insulin molar ratios are between 3 and 400.

14. Composition according to any one of the preceding claims, **characterized in that** the substituted citrate/insulin mass ratios are between 0.5 and 30.

15. Composition according to any one of the preceding claims, **characterized in that** the concentration of substituted citrate is between 1.8 and 100 mg/mL.

16. Composition according to any one of claims 1 to 15, **characterized in that** the insulin is human insulin selected from recombinant human insulins.

17. Composition according to any one of claims 1 to 15, **characterized in that** the insulin is an insulin analog selected from the group consisting of the insulin lispro (Humalog®), the insulin aspart (Novolog®, Novorapid®), and the insulin glulisine (Apidra®).

18. Pharmaceutical composition comprising a composition according to any one of the preceding claims, **characterized in that** the concentration of insulin is between 240 and 3000 µM (40 to 500 IU/mL).

19. Composition according to any one of claims 2 to 18, **characterized in that** the polyanionic compound is selected from the group consisting of carboxylic polyacids and their salts of Na⁺, K⁺, Ca²⁺ or Mg²⁺.

20. Composition according to any one of claims 2 to 19, **characterized in that** the concentration of polyanionic compound is between 2 and 150 mM.

21. Pharmaceutical formulation comprising a composition according to any one of the preceding claims.
